(19) Europäisches Patentamt
European Patent Office
Office européen des brevets

(11) EP 4 644 389 A1

# (12) EUROPEAN PATENT APPLICATION
published in accordance with Art. 153(4) EPC

(43) Date of publication:
**05.11.2025 Bulletin 2025/45**

(21) Application number: **23910839.2**

(22) Date of filing: **28.12.2023**

(51) International Patent Classification (IPC):
***C07D 413/14*** *(2006.01)* ***C07D 401/12*** *(2006.01)*
***C07D 401/14*** *(2006.01)* ***C07D 403/12*** *(2006.01)*
***C07D 405/14*** *(2006.01)* ***C07D 417/14*** *(2006.01)*
***A61K 31/435*** *(2006.01)* ***A61K 31/4353*** *(2006.01)*
***A61P 35/00*** *(2006.01)*

(52) Cooperative Patent Classification (CPC):
**A61K 31/435; A61K 31/4353; A61K 31/4709; A61K 31/501; A61K 31/5377; A61P 35/00; A61P 35/02; C07D 401/12; C07D 401/14; C07D 403/12; C07D 405/12; C07D 405/14; C07D 413/06; C07D 413/14; C07D 417/14;** (Cont.)

(86) International application number:
**PCT/CN2023/142809**

(87) International publication number:
**WO 2024/140923 (04.07.2024 Gazette 2024/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **30.12.2022 CN 202211706668**
**17.03.2023 CN 202310259932**
**11.04.2023 CN 202310378374**

(71) Applicant: **Suzhou Puhe Biopharma Co., Ltd.**
**Suzhou, Jiangsu 215124 (CN)**

(72) Inventors:
- **LIU, Bin**
  **Suzhou, Jiangsu 215124 (CN)**
- **GAO, Feng**
  **Suzhou, Jiangsu 215124 (CN)**
- **GUO, Yongqi**
  **Suzhou, Jiangsu 215124 (CN)**
- **JING, Liandong**
  **Suzhou, Jiangsu 215124 (CN)**
- **WU, Yongyong**
  **Suzhou, Jiangsu 215124 (CN)**
- **LI, Zhizhong**
  **Suzhou, Jiangsu 215124 (CN)**
- **WU, Zhuo**
  **Suzhou, Jiangsu 215124 (CN)**

(74) Representative: **BIP Patents**
**c/o Bayer Intellectual Property GmbH**
**Alfred-Nobel-Straße 50**
**40789 Monheim am Rhein (DE)**

## (54) PRMT5-MTA INHIBITOR

(57) The present invention provides a compound as a PRMT5-MTA inhibitor, which is a compound of formula (I) or a pharmaceutically acceptable salt, isotopic variant, tautomer, or stereoisomer thereof. The present invention also provides a pharmaceutical composition containing said compound and use thereof in the treatment of cancer.

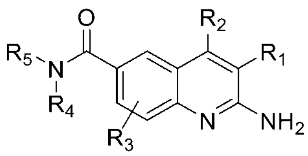

(I)

EP 4 644 389 A1

(52) Cooperative Patent Classification (CPC): (Cont.)
**C07D 491/052**

## Description

### Technical Field

The present invention belongs to the field of medicine and specifically relates to a PRMT5-MTA inhibitor.

### Background Technology

Protein arginine methyltransferases (Protein arginine methyltransferase, PRMT) are capable of methylating both histone and non-histone proteins, thereby participating in the regulation of biological processes such as gene transcription, signal transduction, protein stability, cell proliferation, differentiation, apoptosis, and tumor formation (Nat Rev Mol Cell Biol. 2019 Oct;20(10):642-657) (Nat Rev Drug Discov. 2021 Jul;20(7):509-530). Currently, 11 PRMT family members have been identified, and based on differences in the catalytic arginine methylation mechanism, they can be classified into Type I, Type 11, and Type 111, wherein PRMT5 belongs to Type II, and its catalytic form is symmetric dimethylation.

As an epigenetic enzyme, PRMT5 is involved in various biological processes, including transcriptional regulation, RNA metabolism, ribosome biogenesis, and cell cycle regulation. PRMT5 protein is overexpressed in various types of cancer, including B-cell and T-cell lymphomas, metastatic melanoma, neuroblastoma, glioblastoma, ovarian cancer, and breast cancer, etc., and increasing evidence suggests that it plays a crucial role in tumor initiation and progression (Cell Stress. 2020 Aug; 4(8): 199-215) (Cancer Gene Ther. 2022 Mar;29(3-4):264-276.). On such a basis, PRMT5 inhibitors have become an anti-cancer therapeutic R&D hot spot.

Early PRMT5 inhibitors can be classified into two types, and one type is substrate-competitive inhibitors, represented by GSK3326595; the other type is SAM-competitive inhibitors, represented by JNJ64619178. These two types of drugs both exhibit strong inhibitory activity against PRMT5 and demonstrate potent anti-tumor activity. However, due to their strong inhibitory effect on PRMT5 in both normal and tumor cells, severe hematological toxicity has been observed, limiting their clinical application and affecting their therapeutic efficacy (Bioorg Med Chem Lett. 2019 Jun 1;29(11):1264-1269) (Expert Opin Ther Pat. 2019 Feb;29(2):97-114.) (Annals of Oncology (2020) 31 (suppl_4): S462-S504. 10.1016/annonc/annonc271) (Annals of Oncology (2019) 30 (suppl_5): v159-v193. 10.1093/annonc/mdz244).

In 2016, a study published in Science revealed that MTAP loss exhibits synthetic lethality with PRMT5 (Science. 2016 Mar 11;351(6278):1214-8.). MTAP has a high rate of loss in various solid tumors, including pancreatic cancer and gliomas. MTAP is an intracellular MTA degradation enzyme. The loss of MTAP can lead to intracellular accumulation of MTA, which competes with SAM, the functional methyl donor substrate of PRMT5, for binding to PRMT5, thereby inhibiting the function of PRMT5. Since MTA specifically accumulates in MTAP-deficient tumor cells, enhancing MTA and PRMT5 binding inhibition can achieve selective inhibition of PRMT5 activity in tumor cells while having a weaker inhibitory effect on PRMT5 activity in normal cells, thus providing a safe therapeutic window that ensures anti-tumor efficacy while reducing toxicity (Nat Rev Drug Discov. 2020 Jan;19(1):23-38) (Cell Rep. 2016 Apr 19;15(3):574-587). Currently, MTA-cooperative PRMT5 inhibitors have obtained preclinical validation data (J Med Chem. 2022 Feb 10;65(3):1749-1766). The development of MTA-cooperative PRMT5 inhibitors has great potential in the treatment of MTAP-deficient tumors.

Although progress has been made in PRMT5 research, there is still a lack of effective and selective PRMT5-MTA inhibitors, and no PRMT5-MTA inhibitors have yet entered Phase 2 clinical trials. Therefore, developing highly selective PRMT5-MTA inhibitors will overcome the shortcomings of the first two generations of non-selective PRMT5 inhibitors, to meet clinical needs.

### Summary of the Invention

The present invention provides a novel and highly selective PRMT5-MTA inhibitor. The present invention is realized through the following aspects or embodiments.

In one aspect, the present invention provides a compound of formula (I), or a pharmaceutically acceptable salt, isotopic variant, tautomer, or stereoisomer thereof:

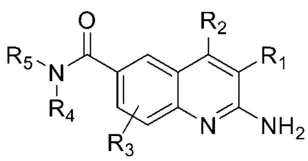

(I)

wherein:

$R_1$ is selected from $C_{1-6}$ alkyl, $C_{3-6}$ cycloalkyl, or $-CH_2OR_a$;
$R_a$ is selected from H or methyl;
$R_2$ is selected from H, $C_{1-6}$ alkyl, $C_{1-6}$ alkoxy, or halogen;
$R_3$ is selected from H, halogen, $OR_b$, CN, $C_{1-6}$ alkyl, or $C_{1-6}$ haloalkyl;
$R_4$ and $R_5$ are independently selected from H, $C_{1-6}$ alkyl, $C_{1-6}$ haloalkyl, $C_{3-6}$ cycloalkyl, 4-12 membered heterocyclyl, or 5-10 membered heteroaryl; the 4-12 membered heterocyclyl or 5-10 membered heteroaryl is optionally substituted with one or two $R_6$, $R_6$ being selected from H, CN, halogen, $C_{1-6}$ alkyl, $C_{1-6}$ haloalkyl, $SF_5$, $OR_b$, $SCF_3$, or 5-6 membered heteroaryl;
alternatively, $R_4$, $R_5$, and the nitrogen atom to which they are attached together form a 4-10 membered heterocyclyl, the 4-10 membered heterocyclyl being optionally substituted with one, two, three, or four $R_x$;
$R_x$ is selected from halogen, $C_{1-6}$ alkyl, $C_{1-6}$ haloalkyl, $C_{1-6}$ haloalkoxy, phenyl or 5-10 membered heteroaryl, the phenyl or 5-10 membered heteroaryl being optionally substituted with one, two, or three $R_y$;
$R_y$ is selected from halogen, $C_{1-6}$ alkyl, $C_{1-6}$ haloalkyl, SFs, CN, $OR_b$, $C_{1-6}$ alkoxy, $C_{3-6}$ cycloalkyl, 4-12 membered heterocyclyl, 5-10 membered heteroaryl or phenyl, the 5-10 membered heteroaryl or phenyl being optionally substituted with one, two, or three $R_z$;
$R_z$ is selected from $C_{1-6}$ alkyl, halogen, CN, $C_{1-6}$ haloalkyl, or $C_{1-6}$ alkoxy;
$R_b$ is selected from H, $C_{1-6}$ alkyl, or $C_{1-6}$ haloalkyl.

In another aspect, the present invention provides a compound of formula (II), or a pharmaceutically acceptable salt, isotopic variant, tautomer, or stereoisomer thereof:

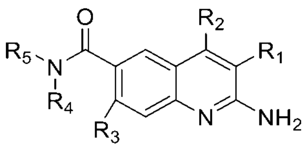
(II)

wherein:

$R_1$ is selected from $C_{1-6}$ alkyl, cyclopropyl, or $-CH_2OH$;
$R_2$ is selected from H, $C_{1-6}$ alkyl, $C_{1-6}$ alkoxy, or halogen;
$R_3$ is selected from H, halogen, CN, or methyl;
$R_4$ and $R_5$ are independently selected from H, $C_{1-6}$ alkyl, $C_{1-6}$ haloalkyl, $C_{3-6}$ cycloalkyl, 4-12 membered heterocyclyl, or 5-10 membered heteroaryl; the 4-12 membered heterocyclyl or 5-10 membered heteroaryl is optionally substituted with one or two $R_6$, $R_6$ being selected from H, CN, halogen, $C_{1-6}$ alkyl, $C_{1-6}$ haloalkyl, $SF_5$, $OR_b$, $SCF_3$, or 5-6 membered heteroaryl;
alternatively, $R_4$, $R_5$, and the nitrogen atom to which they are attached together form a 4-10 membered heterocyclyl, the 4-10 membered heterocyclyl being optionally substituted with one, two, three, or four $R_x$;
$R_x$ is selected from halogen, $C_{1-6}$ alkyl, $C_{1-6}$ haloalkyl, $C_{1-6}$ haloalkoxy, phenyl or 5-10 membered heteroaryl, the phenyl or 5-10 membered heteroaryl being optionally substituted with one, two, or three $R_y$;
$R_y$ is selected from halogen, $C_{1-6}$ alkyl, $C_{1-6}$ haloalkyl, $SF_5$, CN, $OR_b$, $C_{1-6}$ alkoxy, $C_{3-6}$ cycloalkyl, 4-12 membered heterocyclyl, 5-10 membered heteroaryl or phenyl, the 5-10 membered heteroaryl or phenyl being optionally substituted with one, two, or three $R_z$;
$R_z$ is selected from $C_{1-6}$ alkyl, halogen, CN, $C_{1-6}$ haloalkyl, or $C_{1-6}$ alkoxy;
$R_b$ is selected from H, $C_{1-6}$ alkyl, or $C_{1-6}$ haloalkyl.

In another aspect, the present invention provides a pharmaceutical composition comprising a compound of the present invention and optionally a pharmaceutically acceptable excipient.
In another aspect, the present invention provides a pharmaceutical composition containing a compound of the present invention and a pharmaceutically acceptable excipient, further comprising other therapeutic agents.
In another aspect, the present invention provides use of the compound of the present invention in the preparation of a drug for treating and/or preventing PRMT5 methyltransferase-mediated diseases.
In another aspect, the present invention provides a method for treating and/or preventing PRMT5 methyltransferase-mediated diseases in a subject, comprising administering to the subject the compound of the present invention or the composition of the present invention.
In another aspect, the present invention provides the compound of the present invention or the composition of the present invention for use in treating and/or preventing PRMT5 methyltransferase-mediated diseases.
In specific embodiments, the diseases treated by the present invention include cancers selected from the

following: Schwannoma, adenocarcinoma, adrenal cancer, anal cancer, angiosarcoma (e.g., lymphangiosarcoma, lymphangioendothelial sarcoma, hemangioma), appendiceal cancer, benign monoclonal gammopathy, cholangiocarcinoma, bladder cancer, brain cancer (e.g., meningioma, glioma, such as astrocytoma, oligodendroglioma, medulloblastoma), bronchial cancer, carcinoid tumor, cervical cancer (e.g., cervical adenocarcinoma), choriocarcinoma, chordoma, craniopharyngioma, colorectal cancer (e.g., colon cancer, rectal cancer, colorectal adenocarcinoma), epithelial cancer, ependymoma, endothelial sarcoma (e.g., Kaposi's sarcoma, multiple idiopathic hemorrhagic sarcoma), endometrial cancer (e.g., uterine cancer, uterine sarcoma), esophageal cancer (e.g., esophageal adenocarcinoma, Barrett's adenocarcinoma), Ewing's sarcoma, eye cancer (e.g., intraocular melanoma, retinoblastoma), eosinophilia, gallbladder cancer, gastric cancer (e.g., gastric adenocarcinoma), gastrointestinal stromal tumor (GIST), head and neck cancer (e.g., head and neck squamous cell carcinoma, oral cancer (e.g., oral squamous cell carcinoma), laryngeal cancer (e.g., laryngeal cancer, pharyngeal cancer, nasopharyngeal cancer, oropharyngeal cancer)), hematologic cancers (e.g., leukemia, including acute lymphoblastic leukemia (ALL) (e.g., B-cell ALL, T-cell ALL), acute myeloid leukemia (AML) (e.g., B-cell AML, T-cell AML), chronic myeloid leukemia (CML) (e.g., B-cell CML, T-cell CML), chronic lymphocytic leukemia (CLL) (e.g., B-cell CLL, T-cell CLL), follicular lymphoma, chronic lymphocytic leukemia/small lymphocytic lymphoma (CLL/SLL), marginal zone B-cell lymphoma (e.g., mucosa-associated lymphoid tissue (MALT) lymphoma, nodal marginal zone B-cell lymphoma, splenic marginal zone B-cell lymphoma), primary mediastinal B-cell lymphoma, Burkitt's lymphoma, lymphoplasmacytic lymphoma, hairy cell leukemia (HCL), immunoblastic large cell lymphoma, precursor B-lymphoblastic lymphoma, and primary central nervous system (CNS) lymphoma; as well as T-cell non-Hodgkin's lymphoma, such as precursor T-lymphoblastic lymphoma/leukemia, peripheral T-cell lymphoma (e.g., cutaneous T-cell lymphoma (e.g., mycosis fungoides, Sezary syndrome), angioimmunoblastic T-cell lymphoma, extranodal natural killer/T-cell lymphoma, enteropathy-associated T-cell lymphoma, subcutaneous panniculitis-like T-cell lymphoma, anaplastic large cell lymphoma); mixtures of one or more of the aforementioned leukemias/lymphomas; multiple myeloma (MM)), heavy chain diseases (e.g., $\alpha$-chain disease, $\gamma$-chain disease, $\mu$-chain disease), hemangioblastoma, inflammatory myofibroblastic tumor, immunocytic amyloidosis, kidney cancer (e.g., nephroblastoma, renal cell carcinoma), liver cancer (e.g., hepatocellular carcinoma, malignant hepatocellular carcinoma), lung cancer (e.g., bronchial cancer, small cell lung cancer (SCLC), non-small cell lung cancer (NSCLC), lung adenocarcinoma, leiomyosarcoma (LMS)), mastocytosis (e.g., systemic mastocytosis), myelodysplastic syndrome (MDS), mesothelioma, myeloproliferative diseases (MPD) (e.g., polycythemia vera (PV), primary thrombocythemia (ET), idiopathic myelofibrosis (AMM), chronic idiopathic myelofibrosis, chronic granulocytic leukemia (CML), chronic neutrophilic leukemia (CNL), hypereosinophilic syndrome (HES)), neuroblastoma, neurofibroma (e.g., neurofibromatosis type 1 or type 2, schwannomatosis), neuroendocrine carcinoma (e.g., gastroenteropancreatic neuroendocrine tumors (GEP-NET), carcinoid tumor), osteosarcoma, ovarian cancer (e.g., cystadenocarcinoma, ovarian embryonal carcinoma, ovarian adenocarcinoma), papillary adenocarcinoma, penile cancer.

From the following specific embodiments, examples, and claims, other objectives and advantages of the present invention will be apparent to those skilled in the art.

Definitions

Chemical Definitions

The definitions of specific functional groups and chemical terms are described in more detail below.

When a numerical range is listed, it is intended to include each value and subranges within the specified range. For example, "$C_{1-6}$ alkyl" includes $C_1$, $C_2$, $C_3$, $C_4$, $C_5$, $C_6$, $C_{1-6}$, $C_{1-5}$, $C_{1-4}$, $C_{1-3}$, $C_{1-2}$, $C_{2-6}$, $C_{2-5}$, $C_{2-4}$, $C_{2-3}$, $C_{3-6}$, $C_{3-5}$, $C_{3-4}$, $C_{4-6}$, $C_{4-5}$, and $C_{5-6}$ alkyl.

"$C_{1-6}$ alkyl" refers to a straight-chain or branched saturated hydrocarbon group containing 1 to 6 carbon atoms. In some embodiments, $C_{1-4}$ alkyl and $C_{1-2}$ alkyl are preferred. Examples of $C_{1-6}$ alkyl include methyl ($C_1$), ethyl ($C_2$), n-propyl ($C_3$), isopropyl ($C_3$), n-butyl ($C_4$), tert-butyl ($C_4$), sec-butyl ($C_4$), isobutyl ($C_4$), n-pentyl ($C_5$), 3-pentyl ($C_5$), pentyl ($C_5$), neopentyl ($C_5$), 3-methyl-2-butyl ($C_5$), tert-pentyl ($C_5$), and n-hexyl ($C_6$). The term "$C_{1-6}$ alkyl" also includes heteroalkyl, in which one or more (e.g., 1, 2, 3, or 4) carbon atoms are replaced by heteroatoms (e.g., oxygen, sulfur, nitrogen, boron, silicon, phosphorus). The alkyl group may be optionally substituted with one or more substituents, for example substituted with 1 to 5 substituents, 1 to 3 substituents, or 1 substituent. Common abbreviations of alkyl include: Me ($-CH_3$), Et ($-CH_2CH_3$), iPr ($-CH(CH_3)_2$), nPr ($-CH_2CH_2CH_3$), n-Bu ($-CH_2CH_2CH_2CH_3$), or i-Bu ($-CH_2CH(CH_3)_2$).

"$C_{2-6}$ alkenyl" refers to a straight-chain or branched hydrocarbon group with 2 to 6 carbon atoms and at least one carbon-carbon double bond. In some embodiments, $C_{2-4}$ alkenyl is preferred. Examples of $C_{2-6}$ alkenyl include: vinyl ($C_2$), 1-propenyl ($C_3$), 2-propenyl ($C_3$), 1-butenyl ($C_4$), 2-butenyl ($C_4$), butadienyl ($C_4$), pentenyl ($C_5$), pentadienyl ($C_5$), hexenyl ($C_6$), etc. The term "$C_{2-6}$ alkenyl" also includes heteroalkenyl, in which one or more (e.g., 1, 2, 3, or 4) carbon atoms are replaced by heteroatoms (e.g., oxygen, sulfur, nitrogen, boron, silicon, phosphorus). The alkenyl group may be optionally substituted with one or more substituents, for example substituted with 1 to 5 substituents, 1 to 3 substituents, or 1

substituent.

"$C_{2-6}$ alkynyl" refers to a straight-chain or branched hydrocarbon group with 2 to 6 carbon atoms, at least one carbon-carbon triple bond, and optionally one or more carbon-carbon double bonds. In some embodiments, $C_{2-4}$ alkynyl is preferred. Examples of $C_{2-6}$ alkynyl include, but are not limited to: ethynyl ($C_2$), 1-propynyl ($C_3$), 2-propynyl ($C_3$), 1-butynyl ($C_4$), 2-butynyl ($C_4$), pentynyl ($C_5$), hexynyl ($C_6$), etc. The term "$C_{2-6}$ alkynyl" also includes heteroalkynyl, in which one or more (e.g., 1, 2, 3, or 4) carbon atoms are replaced by heteroatoms (e.g., oxygen, sulfur, nitrogen, boron, silicon, phosphorus). The alkynyl group may be optionally substituted with one or more substituents, for example substituted with 1 to 5 substituents, 1 to 3 substituents, or 1 substituent.

"Halogenated" or "halogen" refers to fluorine (F), chlorine (Cl), bromine (Br), and iodine (I).

Thus, "$C_{1-6}$ haloalkyl" refers to the aforementioned "$C_{1-6}$ alkyl", in which one or more hydrogen atoms are replaced by a halogen group. In some embodiments, $C_{1-4}$ haloalkyl is particularly preferred, and $C_{1-2}$ haloalkyl is even more preferred. Exemplary haloalkyl includes, but is not limited to: $-CF_3$, $-CH_2F$, $-CHF_2$, $-CHFCH_2F$, $-CH_2CHF_2$, $-CF_2CF_3$, $-CCl_3$, $-CH_2Cl$, $-CHCl_2$, 2,2,2-trifluoro-1,1-dimethyl-ethyl, etc. The haloalkyl group may be substituted at any available attachment point, for example, with 1 to 5 substituents, 1 to 3 substituents, or 1 substituent.

"$C_{1-6}$ alkoxy" refers to an -OR group, in which R is a $C_{1-6}$ alkyl as defined above. $C_{1-4}$ alkoxy is preferred.

"$C_{1-6}$ haloalkoxy" refers to a "$C_{1-6}$ alkoxy", in which one or more hydrogen atoms are replaced by halogen groups. In some embodiments, $C_{1-4}$ haloalkoxy is particularly preferred, and $C_{1-2}$ haloalkoxy is even more preferred.

"$C_{3-10}$ cycloalkyl" refers to a non-aromatic cyclic hydrocarbon group containing 3 to 10 ring carbon atoms and no heteroatoms. In some embodiments, $C_{4-10}$ cycloalkyl, $C_{5-10}$ cycloalkyl, $C_{4-7}$ cycloalkyl, $C_{3-7}$ cycloalkyl, $C_{3-6}$ cycloalkyl, $C_{3-5}$ cycloalkyl, and $C_{3-4}$ cycloalkyl are particularly preferred, and $C_{5-6}$ cycloalkyl is even more preferred. Cycloalkyl also includes fused ring systems in which the cycloalkyl ring is fused with one or more aryl or heteroaryl, wherein the attachment point is on the cycloalkyl ring, and in such cases, the number of carbons still refers to the number of carbons in the cycloalkyl system. Exemplary cycloalkyl includes, but is not limited to: cyclopropyl ($C_3$), cyclopropenyl ($C_3$), cyclobutyl ($C_4$), cyclobutenyl ($C_4$), cyclopentyl ($C_5$), cyclopentenyl ($C_5$), cyclohexyl ($C_6$), cyclohexenyl ($C_6$), cyclohexadienyl ($C_6$), cycloheptyl ($C_7$), cycloheptenyl ($C_7$), cycloheptadienyl ($C_7$), cycloheptatrienyl ($C_7$), etc. The cycloalkyl group may be optionally substituted with one or more substituents, for example substituted with 1 to 5 substituents, 1 to 3 substituents, or 1 substituent.

"3-12 membered heterocyclyl" refers to a 3-12 membered non-aromatic ring group containing ring carbon atoms and 1 to 5 ring heteroatoms, wherein each heteroatom is independently selected from nitrogen, oxygen, sulfur, boron, phosphorus, and silicon. In the heterocyclyl containing one or more nitrogen atoms, the attachment point may be a carbon or nitrogen atom, as long as valency permits. In some embodiments, 3-10 membered heterocyclyl is preferred, which is a 3-10 membered non-aromatic ring system containing ring carbon atoms and 1 to 3 ring heteroatoms; In some embodiments, 4-10 membered heterocyclyl is preferred, which is a 4-10 membered non-aromatic ring system containing ring carbon atoms and 1 to 4 ring heteroatoms; In some embodiments, 5-10 membered heterocyclyl is preferred, which is a 5-10 membered non-aromatic ring system containing ring carbon atoms and 1 to 5 ring heteroatoms; In some embodiments, 5-8 membered heterocyclyl is preferred, which is a 5-8 membered non-aromatic ring system containing ring carbon atoms and 1 to 5 ring heteroatoms; In some embodiments, 3-7 membered heterocyclyl is preferred, which is a 3-7 membered non-aromatic ring system containing ring carbon atoms and 1 to 4 ring heteroatoms; 3-6 membered heterocyclyl is preferred, which is a 3-6 membered non-aromatic ring system containing ring carbon atoms and 1 to 3 ring heteroatoms; 4-7 membered heterocyclyl is preferred, which is a 4-7 membered non-aromatic ring system containing ring carbon atoms and 1 to 3 ring heteroatoms; 4-6 membered heterocyclyl is preferred, which is a 4-6 membered non-aromatic ring system containing ring carbon atoms and 1 to 3 ring heteroatoms; 5-6 membered heterocyclyl is more preferred, which is a 5-6 membered non-aromatic ring system containing ring carbon atoms and 1 to 3 ring heteroatoms; 3-5 membered heterocyclyl is more preferred, which is a 3-5 membered non-aromatic ring system containing ring carbon atoms and 1 to 3 ring heteroatoms. Heterocyclyl also includes fused ring systems in which the heterocyclyl ring is fused with one or more cycloalkyl rings, wherein the attachment point is on the cycloalkyl ring, or in which the heterocyclyl ring is fused with one or more aryl or heteroaryl rings, wherein the attachment point is on the heterocyclic ring; and in such cases, the number of ring members continues to represent the number of members in the heterocyclyl ring system. Exemplary 3-membered heterocyclyl containing one heteroatom includes, but is not limited to: aziridinyl, oxiranyl, thiorenyl. Exemplary 4-membered heterocyclyl containing one heteroatom includes, but is not limited to: azetidinyl, oxetanyl, and thietanyl. Exemplary 5-membered heterocyclyl containing one heteroatom includes, but is not limited to: tetrahydrofuranyl, dihydrofuranyl, tetrahydrothiophenyl, dihydrothiophenyl, pyrrolidinyl, dihydropyrrolyl, and pyrrolyl-2,5-dione. Exemplary 5-membered heterocyclyl containing two heteroatoms includes, but is not limited to: dioxolanyl, oxasulfuranyl, disulfuranyl, and oxazolidin-2-one. Exemplary 5-membered heterocyclyl containing three heteroatoms includes, but is not limited to: triazolinyl, oxadiazolinyl, and thiadiazolinyl. Exemplary 6-membered heterocyclyl containing one heteroatom includes, but is not limited to: piperidinyl, tetrahydropyranyl, dihydropyridinyl, and thianyl. Exemplary 6-membered heterocyclyl containing two heteroatoms includes, but is not limited to: piperazinyl, morpholinyl, dithiacyclohexanyl, and dioxanyl. Exemplary 6-membered heterocyclyl containing three heteroatoms includes, but is not limited to: triazinanyl. Exemplary 7-membered heterocyclyl containing one heteroatom includes, but is not limited to: azepanyl, oxepanyl, and thiepanyl. Exemplary 5-membered heterocyclyl fused with a $C_6$ aryl ring (also referred to herein as 5,6-bicyclic heterocycles) includes, but is not limited to: dihydroindolyl, isodihydroindolyl, dihydrobenzofuranyl, dihydrobenzothiophenyl, benzoxazolinone, etc. Exemplary 6-membered heterocyclyl fused with a $C_6$ aryl ring (also referred to herein as 6,6-bicyclic heterocycles) includes, but is not limited to: tetrahydroquinolinyl, tetrahydroisoquinolinyl, tetrahydrobenzopyranyl, tetrahydropyranopyridinyl, etc. The heterocyclyl group may be optionally substituted with one or more substituents, for example substituted with 1 to 5 substituents, 1 to 3 substituents, or 1 substituent.

"$C_{6-10}$ aryl" refers to a monocyclic or polycyclic (e.g., bicyclic) 4n+2 aromatic ring system with 6-10 ring carbon atoms and zero heteroatoms (e.g., having 6 or 10 $\pi$-electrons shared in a ring arrangement). In some embodiments, the aryl has six ring carbon atoms ("$C_6$ aryl"; e.g., phenyl). In some embodiments, the aryl has ten ring carbon atoms ("$C_{10}$ aryl"; e.g., naphthyl, such as 1-naphthyl and 2-naphthyl). The aryl also includes fused ring systems in which the aryl ring is fused with one or more cycloalkyl or heterocyclyl, and the attachment point is on the aryl ring; in such cases, the number of carbon atoms continues to represent the number of carbon atoms in the aryl ring system. The aryl group may be optionally substituted with one or more substituents, for example substituted with 1 to 5 substituents, 1 to 3 substituents, or 1 substituent.

"5-14 membered heteroaryl" refers to a monocyclic or bicyclic 4n+2 aromatic ring system with 5-14 ring carbon atoms and 1-4 heteroatoms (e.g., having 6, 10, or 14 $\pi$-electrons shared in a ring arrangement), wherein each heteroatom is independently selected from nitrogen, oxygen, and sulfur. In the heteroaryl containing one or more nitrogen atoms, the attachment point may be a carbon or nitrogen atom, as long as valency permits. The heteroaryl bicyclic system may include one or more heteroatoms in one or both rings. The heteroaryl also includes fused ring systems in which the heteroaryl ring is fused with one or more cycloalkyl or heterocyclyl, and the attachment point is on the heteroaryl ring; in such cases, the number of carbon atoms continues to represent the number of carbon atoms in the heteroaryl ring system. In some embodiments, 5-10 membered heteroaryl is preferred, which is a 5-10 membered monocyclic or bicyclic 4n+2 aromatic ring system containing ring carbon atoms and 1-4 ring heteroatoms. In some embodiments, 5-10 membered heteroaryl is preferred, which is a 6-10 membered monocyclic or bicyclic 4n+2 aromatic ring system containing ring carbon atoms and 1-4 ring heteroatoms. In some embodiments, 5-9 membered heteroaryl is preferred, which is a 5-9 membered monocyclic or bicyclic 4n+2 aromatic ring system containing ring carbon atoms and 1-4 ring heteroatoms. In other embodiments, 5-6 membered heteroaryl is particularly preferred, which is a 5-6 membered monocyclic or bicyclic 4n+2 aromatic ring system containing ring carbon atoms and 1-4 ring heteroatoms. Exemplary 5-membered heteroaryl containing one heteroatom includes, but is not limited to: pyrrolyl, furanyl, and thiophenyl. Exemplary 5-membered heteroaryl containing two heteroatoms includes, but is not limited to: imidazolyl, pyrazolyl, oxazolyl, isoxazolyl, thiazolyl, and isothiazolyl. Exemplary 5-membered heteroaryl containing three heteroatoms includes, but is not limited to: triazolyl, oxadiazolyl (e.g., 1,2,4-oxadiazolyl), and thiadiazolyl. Exemplary 5-membered heteroaryl containing four heteroatoms includes, but is not limited to: tetrazolyl. Exemplary 6-membered heteroaryl containing one heteroatom includes, but is not limited to: pyridyl. Exemplary 6-membered heteroaryl containing two heteroatoms includes, but is not limited to: pyrazinyl, pyrimidinyl, and pyridazinyl. Exemplary 6-membered heteroaryl containing three or four heteroatoms includes, but is not limited to: triazinyl and tetrazinyl. Exemplary 7-membered heteroaryl containing one heteroatom includes, but is not limited to: azepinyl, oxepinyl, and thiepinyl. Exemplary 5,6-bicyclic heteroaryl includes, but is not limited to: indolyl, isoindolyl, indazolyl, benzotriazolyl, benzothiophenyl, isobenzothiophenyl, benzofuranyl, isobenzofuranyl, benzimidazolyl, benzoxazolyl, isobenzoxazolyl, benzooxadiazolyl, benzothiazolyl, isobenzothiazolyl, benzothiadiazolyl, cinnolinyl, and purinyl. Exemplary 6,6-bicyclic heteroaryl includes, but is not limited to: quinolinyl, isoquinolinyl, cinnolinyl, quinoxalinyl, phthalazinyl, and quinazolinyl. The heteroaryl group may be optionally substituted with one or more substituents, for example substituted with 1 to 5 substituents, 1 to 3 substituents, or 1 substituent.

"Alkylene," "heterocyclene," "arylene," or "heteroarylene" refers to a divalent group formed by removing another hydrogen from the above-defined "cycloalkyl," "heterocyclyl," "aryl," or "heteroaryl", which may be substituted or unsubstituted. For example, "$C_{5-7}$ alkylene" refers to a divalent group formed by removing another hydrogen from $C_{5-7}$ cycloalkyl, "5-8 membered heterocyclene" refers to a divalent group formed by removing another hydrogen from 5-8 membered heterocyclyl, "$C_{6-10}$ arylene" refers to a divalent group formed by removing another hydrogen from a $C_{6-10}$ aryl, and "5-6 membered heteroarylene" refers to a divalent group formed by removing another hydrogen from 5-6 membered heteroaryl.

The alkyl, alkenyl, alkynyl, cycloalkyl, heterocyclyl, aryl, and heteroaryl defined herein are optionally substituted groups.

Exemplary substituents on carbon atoms include, but are not limited to: halogen, -CN, $-NO_2$, $-N_3$, $-SO_2H$, $-SO_3H$, -OH, $-OR^{aa}$, $-ON(R^{bb})_2$, $-N(R^{bb})_2$, $-N(R^{bb})_3{}^+X^-$, $-N(OR^{cc})R^{bb}$, -SH, $-SR^{aa}$, $-SSR^{cc}$, $-C(=O)R^{aa}$, $-CO_2H$, -CHO, $-C(OR^{cc})_2$, $-CO_2R^{aa}$, $-OC(=O)R^{aa}$, $-OCO_2R^{aa}$, $-C(=O)N(R^{bb})_2$, $-OC(=O)N(R^{bb})_2$, $-NR^{bb}C(=O)R^{aa}$, $-NR^{bb}CO_2R^{aa}$, $-NR^{bb}C(=O)N(R^{bb})_2$, $-C(=NR^{bb})R^{aa}$, $-C(=NR^{bb})OR^{aa}$, $-OC(=NR^{bb})R^{aa}$, $-OC(=NR^{bb})OR^{aa}$, $-C(=NR^{bb})N(R^{bb})_2$, $-OC(=NR^{bb})N(R^{bb})_2$, $-NR^{bb}C(=NR^{bb})N(R^{bb})_2$, $-C(=O)NR^{bb}SO_2R^{aa}$, $-NR^{bb}SO_2R^{aa}$, $-SO_2N(R^{bb})_2$, $-SO_2R^{aa}$, $-SO_2OR^{aa}$, $-OSO_2R^{aa}$, $-S(=O)R^{aa}$, $-OS(=O)R^{aa}$, $-Si(R^{aa})_3$, $-OSi(R^{aa})_3$, $-C(=S)N(R^{bb})_2$, $-C(=O)SR^{aa}$, $-C(=S)SR^{aa}$, $-SC(=S)SR^{aa}$, $-SC(=O)SR^{aa}$,

$-OC(=O)SR^{aa}$, $-SC(=O)OR^{aa}$, $-SC(=O)R^{aa}$, $-P(=O)_2R^{aa}$, $-OP(=O)_2R^{aa}$, $-P(=O)(R^{aa})_2$, $-OP(=O)(R^{aa})_2$, $-OP(=O)(OR^{cc})_2$, $-P(=O)_2N(R^{bb})_2$, $-OP(=O)_2N(R^{bb})_2$, $-P(=O)(NR^{bb})_2$, $-OP(=O)(NR^{bb})_2$, $-NR^{bb}P(=O)(OR^{cc})_2$, $-NR^{bb}P(=O)(NR^{bb})_2$, $-P(R^{cc})_2$, $-P(R^{cc})_3$, $-OP(R^{cc})_2$, $-OP(R^{cc})_3$, $-B(R^{aa})_2$, $-B(OR^{cc})_2$, $-BR^{aa}(OR^{cc})$, alkyl, haloalkyl, alkenyl, alkynyl, cycloalkyl, heterocyclyl, aryl, and heteroaryl, wherein each alkyl, alkenyl, alkynyl, cycloalkyl, heterocyclyl, aryl, and heteroaryl is independently substituted with 0, 1, 2, 3, 4, or 5 $R^{dd}$ groups;

Alternatively, the two geminal hydrogens on a carbon atom are replaced by a group =O, =S, $=NN(R^{bb})_2$, $=NNR^{bb}C(=O)R^{aa}$, $=NNR^{bb}C(=O)OR^{aa}$, $=NNR^{bb}S(=O)_2R^{aa}$, $=NR^{bb}$, or $=NOR^{cc}$;

Each $R^{aa}$ is independently selected from alkyl, haloalkyl, alkenyl, alkynyl, cycloalkyl, heterocyclyl, aryl, and heteroaryl, or two $R^{aa}$ groups combine to form a heterocyclic or heteroaryl ring, wherein each alkyl, alkenyl, alkynyl, cycloalkyl, heterocyclyl, aryl, and heteroaryl is independently substituted with 0, 1, 2, 3, 4, or 5 $R^{dd}$ groups;

Each $R^{bb}$ is independently selected from hydrogen, -OH, $-OR^{aa}$, $-N(R^{cc})_2$, -CN, $-C(=O)R^{aa}$, $-C(=O)N(R^{cc})_2$, $-CO_2R^{aa}$, $-SO_2R^{aa}$, $-C(=NR^{cc})OR^{aa}$, $-C(=NR^{cc})N(R^{cc})_2$, $-SO_2N(R^{cc})_2$, $-SO_2R^{cc}$, $-SO_2OR^{cc}$, $-SOR^{aa}$, $-C(=S)N(R^{cc})_2$, $-C(=O)SR^{cc}$, $-C(=S)SR^{cc}$, $-P(=O)_2R^{aa}$, $-P(=O)(R^{aa})_2$, $-P(=O)_2N(R^{cc})_2$, $-P(=O)(NR^{cc})_2$, alkyl, haloalkyl, alkenyl, alkynyl, cycloalkyl, heterocyclyl, aryl, and heteroaryl, or two $R^{bb}$ groups combine to form a heterocyclyl or heteroaryl ring, wherein each alkyl, alkenyl, alkynyl, cycloalkyl, heterocyclyl, aryl, and heteroaryl is independently substituted with 0, 1, 2, 3, 4, or 5 $R^{dd}$ groups;

Each $R^{cc}$ is independently selected from hydrogen, alkyl, haloalkyl, alkenyl, alkynyl, cycloalkyl, heterocyclyl, aryl, and heteroaryl, or two $R^{cc}$ groups combine to form a heterocyclyl or heteroaryl ring, wherein each alkyl, alkenyl, alkynyl, cycloalkyl, heterocyclyl, aryl, and heteroaryl is independently substituted with 0, 1, 2, 3, 4, or 5 $R^{dd}$ groups;

Each $R^{dd}$ is independently selected from halogen, -CN, $-NO_2$, $-N_3$, $-SO_2H$, $-SO_3H$, -OH, $-OR^{ee}$, $-ON(R^{ff})_2$, $-N(R^{ff})_2$, $-N(R^{ff})_3^+X^-$, $-N(OR^{ee})R^{ff}$, -SH, $-SR^{ee}$, $-SSR^{ee}$, $-C(=O)R^{ee}$, $-CO_2H$, $-CO_2R^{ee}$, $-OC(=O)R^{ee}$, $-OCO_2R^{ee}$, $-C(=O)N(R^{ff})_2$, $-OC(=O)N(R^{ff})_2$, $-NR^{ff}C(=O)R^{ee}$, $-NR^{ff}CO_2R^{ee}$, $-NR^{ff}C(=O)N(R^{ff})_2$, $-C(=NR^{ff})OR^{ee}$, $-OC(=NR^{ff})R^{ee}$, $-OC=NR^{ff})OR^{ee}$, $-C=NR^{ff})N(R^{ff})_2$, $-OC=NR^{ff})N(R^{ff})_2$, $-NR^{ff}Q=NR^{ff})N(R^{ff})_2$, $-NR^{ff}SO_2R^{ee}$, $-SO_2N(R^{ff})_2$, $-SO_2R^{ee}$, $-SO_2OR^{ee}$, $-OSO_2R^{ee}$, $-S(=O)R^{ee}$, $-Si(R^{ee})_3$, $-OSi(R^{ee})_3$, $-C(=S)N(R^{ff})_2$, $-C(=O)SR^{ee}$, $-C(=S)SR^{ee}$, $-SC(=S)SR^{ee}$, $-P(=O)_2R^{ee}$, $-P(=O)(R^{ee})_2$, $-OP(=O)(R^{ee})_2$, $-OP(=O)(OR^{ee})_2$, alkyl, haloalkyl, alkenyl, alkynyl, cycloalkyl, heterocyclyl, aryl, heteroaryl, wherein each alkyl, alkenyl, alkynyl, cycloalkyl, heterocyclyl, aryl, and heteroaryl is independently substituted with 0, 1, 2, 3, 4, or 5 $R^{gg}$ groups, or two geminal $R^{dd}$ substituents may combine to form =O or =S;

Each $R^{ee}$ is independently selected from alkyl, haloalkyl, alkenyl, alkynyl, cycloalkyl, aryl, heterocyclyl, and heteroaryl, wherein each alkyl, alkenyl, alkynyl, cycloalkyl, heterocyclyl, aryl, and heteroaryl is independently substituted with 0, 1, 2, 3, 4, or 5 $R^{gg}$ groups;

Each $R^{ff}$ is independently selected from hydrogen, alkyl, haloalkyl, alkenyl, alkynyl, cycloalkyl, heterocyclyl, aryl, and heteroaryl, or two $R^{ff}$ groups combine to form a heterocyclyl or heteroaryl ring, wherein each alkyl, alkenyl, alkynyl, cycloalkyl, heterocyclyl, aryl, and heteroaryl is independently substituted with 0, 1, 2, 3, 4, or 5 $R^{gg}$ groups;

Each $R^{gg}$ is independently selected from: halogen, -CN, $-NO_2$, $-N_3$, $-SO_2H$, $-SO_3H$, -OH, $-OC_{1-6}$ alkyl, $-ON(C_{1-6}$ alkyl$)_2$, $-N(C_{1-6}$ alkyl$)_2$, $-N(C_{1-6}$ alkyl$)_3^+X^-$, $-NH(C_{1-6}$ alkyl$)_2^+X^-$, $-NH_2(C_{1-6}$ alkyl$)^+X^-$, $-NH_3^+X^-$, $-N(OC_{1-6}$ alkyl$)(C_{1-6}$ alkyl), $-N(OH)(C_{1-6}$ alkyl), -NH(OH), -SH, $-SC_{1-6}$ alkyl, $-SS(C_{1-6}$ alkyl), $-C(=O)(C_{1-6}$ alkyl), $-CO_2H$, $-CO_2(C_{1-6}$ alkyl), $-OC(=O)(C_{1-6}$ alkyl), $-OCO_2(C_{1-6}$ alkyl), $-C(=O)NH_2$, $-C(=O)N(C_{1-6}$ alkyl$)_2$, $-OC(=O)NH(C_{1-6}$ alkyl), $-NHC(=O)(C_{1-6}$ alkyl), $-N(C_{1-6}$ alkyl$)C(=O)(C_{1-6}$ alkyl), $-NHCO_2(C_{1-6}$ alkyl), $-NHC(=O)N(C_{1-6}$ alkyl$)_2$, $-NHC(=O)NH(C_{1-6}$ alkyl), $-NHC(=O)NH_2$, $-C(=NH)O(C_{1-6}$ alkyl), $-OC(=NH)(C_{1-6}$ alkyl), $-OC(=NH)OC_{1-6}$ alkyl, $-C(=NH)N(C_{1-6}$ alkyl$)_2$, $-C(=NH)NH(C_{1-6}$ alkyl), $-C(=NH)NH_2$, $-OC(=NH)N(C_{1-6}$ alkyl$)_2$, $-OC(NH)NH(C_{1-6}$ alkyl), $-OC(NH)NH_2$, $-NHC(NH)N(C_{1-6}$ alkyl$)_2$, $-NHC(=NH)NH_2$, $-NHSO_2(C_{1-6}$ alkyl), $-SO_2N(C_{1-6}$ alkyl$)_2$, $-SO_2NH(C_{1-6}$ alkyl), $-SO_2NH_2$, $-SO_2C_{1-6}$ alkyl, $-SO_2OC_{1-6}$ alkyl, $-OSO_2C_{1-6}$ alkyl, $-SOC_{1-6}$ alkyl, $-Si(C_{1-6}$ alkyl$)_3$, $-OSi(C_{1-6}$ alkyl$)_3$, $-C(=S)N(C_{1-6}$ alkyl$)_2$, $C(=S)NH(C_{1-6}$ alkyl), $C(=S)NH_2$, $-C(=O)S(C_{1-6}$ alkyl), $-C(=S)SC_{1-6}$ alkyl, $-SC(=S)SC_{1-6}$ alkyl, $-P(=O)_2(C_{1-6}$ alkyl), $-P(=O)(C_{1-6}$ alkyl$)_2$, $-OP(=O)(C_{1-6}$ alkyl$)_2$, $-OP(=O)(OC_{1-6}$ alkyl$)_2$, $C_{1-6}$ alkyl, $C_{1-6}$ haloalkyl, $C_2$-$C_6$ alkenyl, $C_2$-$C_6$ alkynyl, $C_3$-$C_7$ cycloalkyl, $C_6$-$C_{10}$ aryl, $C_3$-$C_7$ heterocyclyl, $C_5$-$C_{10}$ heteroaryl; or two geminal $R^{gg}$ substituents may combine to form =O or =S; wherein $X^-$ is a counterion.

Exemplary substituents on the nitrogen atom include, but are not limited to: hydrogen, -OH, $-OR^{aa}$, $-N(R^{cc})_2$, -CN, $-C(=O)R^{aa}$, $-C(=O)N(R^{cc})_2$, $-CO_2R^{aa}$, $-SO_2R^{aa}$, $-C(=NR^{bb})R^{aa}$, $-C(=NR^{cc})OR^{aa}$, $-C(=NR^{cc})N(R^{cc})_2$, $-SO_2N(R^{cc})_2$, $-SO_2R^{cc}$, $-SO_2OR^{cc}$, $-SOR^{aa}$, $-C(=S)N(R^{cc})_2$, $-C(=O)SR^{cc}$, $-C(=S)SR^{cc}$, $-P(=O)_2R^{aa}$, $-P(=O)(R^{aa})_2$, $-P(=O)_2N(R^{cc})_2$, $-P(=O)(NR^{cc})_2$, alkyl, haloalkyl, alkenyl, alkynyl, cycloalkyl, heterocyclyl, aryl, and heteroaryl, or two $R^{cc}$ groups attached to the nitrogen atom combine to form a heterocyclyl or heteroaryl ring, wherein each alkyl, alkenyl, alkynyl, cycloalkyl, heterocyclyl, aryl, and heteroaryl is independently substituted with 0, 1, 2, 3, 4, or 5 $R^{dd}$ groups, and wherein $R^{aa}$, $R^{bb}$, $R^{cc}$, and $R^{dd}$ are as defined above.

The term "deuterium (D or 2H)" refers to the stable isotope of hydrogen, which is naturally present at an abundance of 0.015 mol%. The term "deuterated" refers to the replacement of one or more hydrogen atoms (H) in a group or compound with deuterium (D).

A "deuterated compound" refers to a compound in which one or more hydrogen atoms bonded to carbon are replaced with one or more deuterium atoms. Similarly, "deuterated" refers to a chemical structure or organic group in which one or more hydrogen atoms bonded to carbon are replaced with one or more deuterium atoms, such as "deuterated alkyl,"

"deuterated cycloalkyl," "deuterated heterocycloalkyl," and "deuterated aryl," etc. For example, "deuterated alkyl" refers to an alkyl group as defined in this application, in which at least one hydrogen atom bonded to carbon is replaced with deuterium. In deuterated alkyl, at least one carbon atom is bonded to at least one deuterium; a carbon atom may be bonded to multiple deuterium atoms; multiple carbon atoms in the alkyl may also be bonded to deuterium. For example, deuterated methyl includes methyl-$d_3$, in which three hydrogen atoms are replaced with deuterium; it also includes monodeuterated methyl and dideuterated methyl. In some embodiments, the compounds of the present invention include deuterated compounds.

**Other Definitions**

The term "pharmaceutically acceptable salt" as used herein refers to those carboxylate salts and amino acid addition salts of the compounds of the present invention, which, within the scope of sound medical judgment, are suitable for contact with the tissues of a patient without causing undue toxicity, irritation, allergic reactions, or other adverse effects, are commensurate with a reasonable benefit/risk ratio, and are effective for their intended application, including (if applicable) the zwitterionic form of the compounds of the present invention.

The "subject" for drug administration includes, but is not limited to, humans (i.e., males or females of any age group, such as pediatric subjects (e.g., infants, children, adolescents) or adult subjects (e.g., young adults, middle-aged adults, or elderly adults)) and/or non-human animals, such as mammals, including primates (e.g., cynomolgus monkeys, rhesus monkeys), cattle, pigs, horses, sheep, goats, rodents, cats, and/or dogs. In some embodiments, the subject is a human. In some embodiments, the subject is a non-human animal. The terms "human," "patient," and "subject" are used interchangeably herein.

The terms "disease," "disorder," and "condition" are used interchangeably herein.

Generally, an "effective amount" of a compound refers to an amount sufficient to elicit a desired biological response. As understood by those skilled in the art, the effective amount of the compound of the present invention may vary depending on factors such as: the biological target, the pharmacokinetics of the compound, the disease being treated, the mode of administration, and the subject’s age, health status, and symptoms. An effective amount includes both a therapeutically effective amount and a prophylactically effective amount.

The term "in combination" and related terms refer to simultaneous or sequential administration of the compound of the present invention and another therapeutic agent. For example, the compound of the present invention may be administered simultaneously or sequentially with another therapeutic agent in separate unit dosage forms, or may be administered together in a single unit dosage form.

**Specific Embodiments**

As used herein, a "compound of the present invention" refers to the compound of formula (I) (including sub-formulas such as formula (II), (III-1), (III-1a), (III-1b), (III-2), (III-2a), (III-2b), (III-3), (III-3a), (III-3b), (IV-1), (IV-1a), (IV-1b), (IV-2), (IV-2a), (IV-2b), (IV-3), (IV-3a), or (IV-3b), etc.), its pharmaceutically acceptable salts, enantiomers, diastereomers, or isotopic variants, and mixtures thereof.

In one embodiment, the present invention relates to a compound of formula (I), or a pharmaceutically acceptable salt, isotopic variant, tautomer, or stereoisomer thereof:

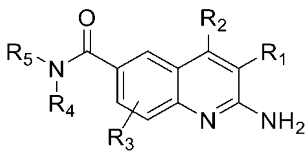
(I)

wherein:

$R_1$ is selected from $C_{1-6}$ alkyl, $C_{3-6}$ cycloalkyl, or $-CH_2OR_a$;
$R_a$ is selected from H or methyl;
$R_2$ is selected from H, $C_{1-6}$ alkyl, $C_{1-6}$ alkoxy, or halogen;
$R_3$ is selected from H, halogen, $OR_b$, CN, $C_{1-6}$ alkyl, or $C_{1-6}$ haloalkyl;
$R_4$ and $R_5$ are independently selected from H, $C_{1-6}$ alkyl, $C_{1-6}$ haloalkyl, $C_{3-6}$ cycloalkyl, 4-12 membered heterocyclyl, or 5-10 membered heteroaryl; the 4-12 membered heterocyclyl or 5-10 membered heteroaryl is optionally substituted with one or two $R_6$, $R_6$ being selected from H, CN, halogen, $C_{1-6}$ alkyl, $C_{1-6}$ haloalkyl, $SF_5$, $OR_b$, $SCF_3$, or 5-6 membered heteroaryl;
alternatively, $R_4$, $R_5$, and the nitrogen atom to which they are attached together form a 4-10 membered heterocyclyl,

the 4-10 membered heterocyclyl being optionally substituted with one, two, three, or four $R_x$;
$R_x$ is selected from halogen, $C_{1-6}$ alkyl, $C_{1-6}$ haloalkyl, $C_{1-6}$ haloalkoxy, phenyl or 5-10 membered heteroaryl, the phenyl or 5-10 membered heteroaryl being optionally substituted with one, two, or three $R_y$;
$R_y$ is selected from halogen, $C_{1-6}$ alkyl, $C_{1-6}$ haloalkyl, SFs, CN, $OR_b$, $C_{1-6}$ alkoxy, $C_{3-6}$ cycloalkyl, 4-12 membered heterocyclyl, 5-10 membered heteroaryl or phenyl, the 5-10 membered heteroaryl or phenyl being optionally substituted with one, two, or three $R_z$;
$R_z$ is selected from $C_{1-6}$ alkyl, halogen, CN, $C_{1-6}$ haloalkyl, or $C_{1-6}$ alkoxy;
$R_b$ is selected from H, $C_{1-6}$ alkyl, or $C_{1-6}$ haloalkyl.

In another embodiment, the present invention relates to a compound of formula (II), or a pharmaceutically acceptable salt, isotopic variant, tautomer, or stereoisomer thereof:

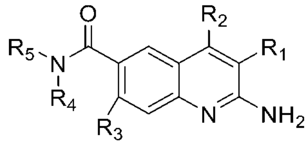 (II)

wherein,

$R_1$ is selected from $C_{1-6}$ alkyl, cyclopropyl, or $-CH_2OH$;
$R_2$ is selected from H, $C_{1-6}$ alkyl, $C_{1-6}$ alkoxy, or halogen;
$R_3$ is selected from H, halogen, CN, or methyl;
$R_4$ and $R_5$ are independently selected from H, $C_{1-6}$ alkyl, $C_{1-6}$ haloalkyl, $C_{3-6}$ cycloalkyl, 4-12 membered heterocyclyl or 5-10 membered heteroaryl; the 4-12 membered heterocyclyl or 5-10 membered heteroaryl is optionally substituted with one or two $R_6$, $R_6$ being selected from H, CN, halogen, $C_{1-6}$ alkyl, $C_{1-6}$ haloalkyl, $SF_5$, $OR_b$, $SCF_3$, or 5-6 membered heteroaryl;
alternatively, $R_4$, $R_5$, and the nitrogen atom to which they are attached together form a 4-10 membered heterocyclyl, the 4-10 membered heterocyclyl being optionally substituted with one, two, three, or four $R_x$;
$R_x$ is selected from halogen, $C_{1-6}$ alkyl, $C_{1-6}$ haloalkyl, $C_{1-6}$ haloalkoxy, phenyl or 5-10 membered heteroaryl, the phenyl or 5-10 membered heteroaryl being optionally substituted with one, two, or three $R_y$;
$R_y$ is selected from halogen, $C_{1-6}$ alkyl, $C_{1-6}$ haloalkyl, SFs, CN, $OR_b$, $C_{1-6}$ alkoxy, $C_{3-6}$ cycloalkyl, 4-12 membered heterocyclyl, 5-10 membered heteroaryl or phenyl, the 5-10 membered heteroaryl or phenyl being optionally substituted with one, two, or three $R_z$;
$R_z$ is selected from $C_{1-6}$ alkyl, halogen, CN, $C_{1-6}$ haloalkyl, or $C_{1-6}$ alkoxy;
$R_b$ is selected from H, $C_{1-6}$ alkyl, or $C_{1-6}$ haloalkyl.

In some embodiments of the compounds of formula (I) or (II), $R_1$ is cyclopropyl.
In some embodiments of the compounds of formula (I) or (II), $R_1$ is hydroxymethyl.
In some embodiments of the compounds of formula (I) or (II), $R_1$ is hydroxymethyl; $R_4$ is H or $C_{1-6}$ alkyl; $R_5$ is 4-12 membered heterocyclyl; the 4-12 membered heterocyclyl is optionally substituted with one or two $R_6$.
In some embodiments of the compounds of formula (I) or (II), $R_1$ is hydroxymethyl; $R_4$ is H or $C_{1-6}$ alkyl; $R_5$ is 4-12 membered heterocyclyl; the 4-12 membered heterocyclyl is optionally substituted with one or two $R_6$.
In some embodiments of the compounds of formula (I) or (II), $R_1$ is hydroxymethyl; $R_4$, $R_5$, and the nitrogen atom to which they are attached together form 4-10 membered heterocyclyl, which is optionally substituted with one, two, three, or four $R_x$ substituents.
In another embodiment, the present invention provides a compound of formula (III-1), (III-1a), or (III-1b), or a pharmaceutically acceptable salt, isotopic variant, tautomer, or stereoisomer thereof:

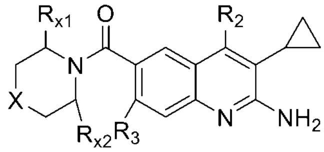 (III-1)

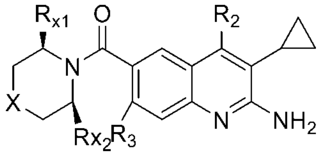 (III-1a) or 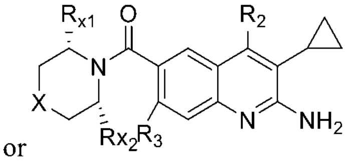 (III-1b)

wherein,

- $R_2$ is selected from H, methyl, ethyl, methoxy, or F;
- $R_3$ is selected from H, F, Cl, CN, or methyl;
- $R_{x1}$ is selected from phenyl or 5-10 membered heteroaryl, the phenyl or 5-10 membered heteroaryl being optionally substituted with one, two, or three $R_y$;
- $R_y$ is selected from halogen, $C_{1-6}$ alkyl, $C_{1-6}$ haloalkyl, SFs, CN, $OR_b$, $C_{1-6}$ alkoxy, $C_{3-6}$ cycloalkyl, 4-12 membered heterocyclyl, 5-10 membered heteroaryl or phenyl, the 5-10 membered heteroaryl or phenyl being optionally substituted with one, two, or three $R_z$;
- $R_z$ is selected from $C_{1-6}$ alkyl, halogen, CN, $C_{1-6}$ haloalkyl, or $C_{1-6}$ alkoxy;
- $R_b$ is selected from H, $C_{1-6}$ alkyl, or $C_{1-6}$ haloalkyl;
- $R_{x2}$ is selected from $C_{1-6}$ alkyl, $C_{1-6}$ haloalkyl, or $C_{1-6}$ haloalkoxy;
- X is selected from $-CH_2-$, O, S, or $-NR_c-$;
- $R_c$ is selected from H or $C_{1-6}$ alkyl.

In another more specific embodiment, the present invention provides the above-mentioned compounds of formula (III-1), (III-1a), or (III-1b), or a pharmaceutically acceptable salt, isotopic variant, tautomer, or stereoisomer thereof, wherein,

- $R_2$ is selected from H, methyl, ethyl, methoxy, or F;
- $R_3$ is selected from H, F, Cl, CN, or methyl;
- $R_{x1}$ is selected from phenyl or a 6-membered heteroaryl, the phenyl or 6-membered heteroaryl being optionally substituted with one, two, or three $R_y$;
- $R_y$ is selected from halogen, $C_{1-6}$ alkyl, $C_{1-6}$ haloalkyl, SFs, CN, $OR_b$, $C_{1-6}$ alkoxy, $C_{3-6}$ cycloalkyl, 4-12 membered heterocyclyl, 5-10 membered heteroaryl or phenyl, the 5-10 membered heteroaryl or phenyl being optionally substituted with one, two, or three $R_z$;
- $R_z$ is selected from $C_{1-6}$ alkyl, halogen, CN, $C_{1-6}$ haloalkyl, or $C_{1-6}$ alkoxy;
- $R_b$ is selected from H, $C_{1-6}$ alkyl, or $C_{1-6}$ haloalkyl;
- $R_{x2}$ is selected from methyl, ethyl, trifluoromethyl, or trifluoromethoxy;
- X is selected from $-CH_2-$, O, or S.

In some embodiments, $R_2$ is selected from H, methyl, or ethyl. In some embodiments, $R_2$ is methyl. In some embodiments, $R_2$ is ethyl.

In some embodiments, $R_3$ is selected from H, F, or Cl. In some embodiments, $R_3$ is F or Cl. In some embodiments, $R_3$ is F.

In some embodiments, $R_{x1}$ is selected from phenyl or a 6-membered heteroaryl, the phenyl or 6-membered heteroaryl being optionally substituted with one $R_y$; $R_y$ is selected from halogen, $C_{1-6}$ alkyl, $C_{1-6}$ alkoxy, $C_{1-6}$ haloalkyl, $C_{1-6}$ haloalkoxy, 5-10 membered heteroaryl or phenyl, the 5-10 membered heteroaryl or phenyl being optionally substituted with one $R_z$; $R_z$ is selected from $C_{1-6}$ alkyl, halogen, CN, $C_{1-6}$ haloalkyl, or $C_{1-6}$ alkoxy. In some embodiments, $R_z$ is selected from methyl, ethyl, F, Cl, trifluoromethyl, difluoromethyl, trifluoromethoxy, difluoromethoxy, methoxy, ethoxy, or isopropoxy.

In some embodiments, $R_{x2}$ is $C_{1-6}$ alkyl. In some embodiments, $R_{x2}$ is methyl or ethyl. In some embodiments, $R_{x2}$ is methyl.

In some embodiments, X is O. In some embodiments, X is $-CH_2-$. In some embodiments, X is S. In some embodiments, X is -NH-.

In some embodiments, $R_3$ is F, and $R_{x2}$ is $C_{1-6}$ alkyl. In some embodiments, $R_3$ is F, and $R_{x2}$ is methyl or ethyl.

In another embodiment, the present invention provides a compound of formula (III-2), (III-2a), or (III-2b), or a pharmaceutically acceptable salt, isotopic variant, tautomer, or stereoisomer thereof:

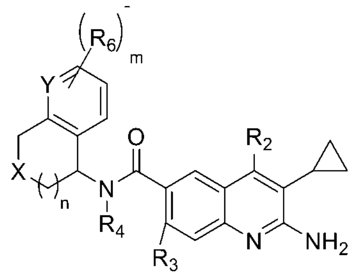
(III-2)

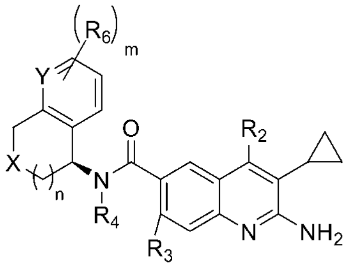
(III-2a) or
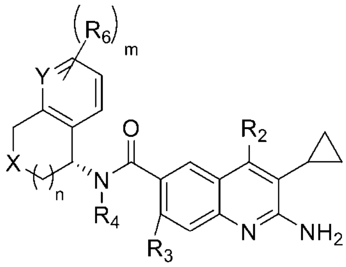
(III-2b)

wherein,

$R_2$ is selected from H, methyl, ethyl, methoxy, or F;
$R_3$ is selected from H, F, Cl, CN, or methyl;
$R_4$ is selected from H, $C_{1-6}$ alkyl, $C_{1-6}$ haloalkyl, $C_{3-6}$ cycloalkyl, or 4-12 membered heterocyclyl;
$R_6$ is selected from H, CN, halogen, $C_{1-6}$ alkyl, $C_{1-6}$ haloalkyl, $SF_5$, $OR_b$, or a 5-6 membered heteroaryl;
$R_b$ is selected from H, $C_{1-6}$ alkyl, or $C_{1-6}$ haloalkyl;
X is selected from $-CH_2-$, O, S, or $-NR_c-$;
Y is selected from CH or N;
$R_c$ is selected from H or $C_{1-6}$ alkyl;
m is 0, 1, or 2;
n is 0, 1, 2, or 3.

In another more specific embodiment, the present invention provides a compound of formula (III-3), (III-3a), or (III-3b), or a pharmaceutically acceptable salt, isotopic variant, tautomer, or stereoisomer thereof:

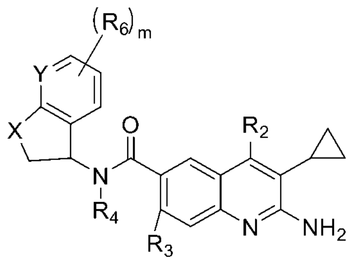
(III-3)

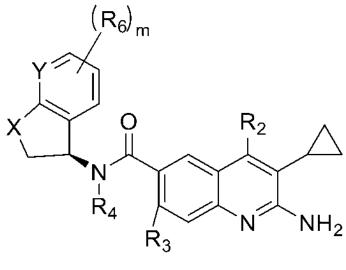
(III-3a) or
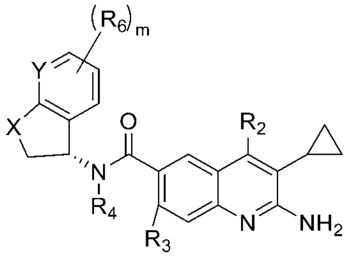
(III-3b)

wherein,

$R_2$ is selected from H, methyl, ethyl, methoxy, or F;
$R_3$ is selected from H, F, Cl, CN, or methyl;
$R_4$ is selected from H, $C_{1-6}$ alkyl, $C_{1-6}$ haloalkyl, $C_{3-6}$ cycloalkyl, or 4-12 membered heterocyclyl;
$R_6$ is selected from H, CN, halogen, $C_{1-6}$ alkyl, $C_{1-6}$ haloalkyl, $SF_5$, $OR_b$, $SCF_3$, or 5-6 membered heteroaryl;
$R_b$ is selected from H, $C_{1-6}$ alkyl, or $C_{1-6}$ haloalkyl;
X is selected from $-CH_2-$, O, S, or $-NR_c-$;
Y is selected from CH or N;
$R_c$ is selected from H or $C_{1-6}$ alkyl;
m is 0, 1, or 2.

In another embodiment, the present invention provides a compound of formula (IV-1), (IV-1a), or (IV-1b), or a pharmaceutically acceptable salt, isotopic variant, tautomer, or stereoisomer thereof:

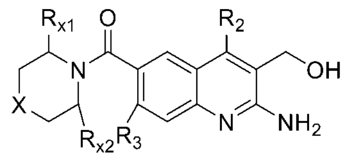
(IV-1)

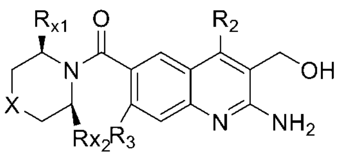
(IV-1a) or
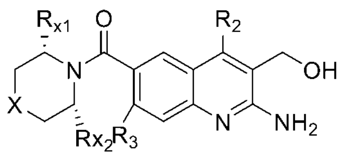
(IV-1b)

wherein,

$R_2$ is selected from H, methyl, ethyl, methoxy, or F;
$R_3$ is selected from H, F, Cl, CN, or methyl;
$R_{x1}$ is selected from phenyl or 5-10 membered heteroaryl, the phenyl or 5-10 membered heteroaryl being optionally substituted with one, two, or three $R_y$;
$R_y$ is selected from halogen, $C_{1-6}$ alkyl, $C_{1-6}$ haloalkyl, SFs, CN, $OR_b$, $C_{1-6}$ alkoxy, $C_{3-6}$ cycloalkyl, 4-12 membered heterocyclyl, 5-10 membered heteroaryl or phenyl, the 5-10 membered heteroaryl or phenyl being optionally substituted with one, two, or three $R_z$;
$R_z$ is selected from $C_{1-6}$ alkyl, halogen, CN, $C_{1-6}$ haloalkyl, or $C_{1-6}$ alkoxy;
$R_b$ is selected from H, $C_{1-6}$ alkyl, or $C_{1-6}$ haloalkyl;
$R_{x2}$ is selected from $C_{1-6}$ alkyl, $C_{1-6}$ haloalkyl, or $C_{1-6}$ haloalkoxy;
X is selected from $-CH_2-$, O, S, or $-NR_c-$;
$R_c$ is selected from H or $C_{1-6}$ alkyl.

In another more specific embodiment, the present invention provides a compound of the above-mentioned formula (IV-1), (IV-1a), or (IV-1b), or a pharmaceutically acceptable salt, isotopic variant, tautomer, or stereoisomer thereof, wherein,

$R_2$ is selected from H, methyl, ethyl, methoxy, or F;
$R_3$ is selected from H, F, Cl, CN, or methyl;
$R_{x1}$ is selected from phenyl or 6-membered heteroaryl, the phenyl or 6-membered heteroaryl being optionally substituted with one, two, or three $R_y$;
$R_y$ is selected from halogen, $C_{1-6}$ alkyl, $C_{1-6}$ haloalkyl, SFs, CN, $OR_b$, $C_{1-6}$ alkoxy, $C_{3-6}$ cycloalkyl, 4-12 membered heterocyclyl, 5-10 membered heteroaryl or phenyl, the 5-10 membered heteroaryl or phenyl being optionally substituted with one, two, or three $R_z$;
$R_z$ is selected from $C_{1-6}$ alkyl, halogen, CN, $C_{1-6}$ haloalkyl, or $C_{1-6}$ alkoxy;
$R_b$ is selected from H, $C_{1-6}$ alkyl, or $C_{1-6}$ haloalkyl;
$R_{x2}$ is selected from methyl, ethyl, trifluoromethyl, or trifluoromethoxy;
X is selected from $-CH_2-$, O, or S.

In some embodiments, $R_2$ is selected from H, methyl, or ethyl. In some embodiments, $R_2$ is methyl. In some

embodiments, $R_2$ is ethyl.

In some embodiments, $R_3$ is selected from H, F, or Cl. In some embodiments, $R_3$ is F or Cl. In some embodiments, $R_3$ is F. In some embodiments, $R_3$ is Cl.

In some embodiments, $R_{x1}$ is selected from phenyl or 6-membered heteroaryl, the phenyl or 6-membered heteroaryl being optionally substituted with one $R_y$. In some embodiments, the 6-membered heteroaryl is pyridyl or pyrimidyl. In some embodiments, the 6-membered heteroaryl is pyridyl.

In some embodiments, $R_y$ is selected from halogen, $C_{1-6}$ alkyl, $C_{1-6}$ haloalkyl, $C_{1-6}$ haloalkoxy, 5-10 membered heteroaryl or phenyl, the 5-10 membered heteroaryl or phenyl being optionally substituted with one, two, or three $R_z$; $R_z$ is selected from $C_{1-6}$ alkyl, halogen, CN, $C_{1-6}$ haloalkyl, or $C_{1-6}$ alkoxy. In some embodiments, $R_y$ is selected from halogen, $C_{1-6}$ alkyl, $C_{1-6}$ haloalkyl, $C_{1-6}$ alkoxy, $C_{1-6}$ haloalkoxy, pyridyl, or phenyl, the pyridyl or phenyl being optionally substituted with one, two, or three $R_z$; $R_z$ is selected from $C_{1-6}$ alkyl, halogen, CN, $C_{1-6}$ haloalkyl, or $C_{1-6}$ alkoxy. In some embodiments, $R_z$ is selected from methyl, ethyl, F, Cl, trifluoromethyl, difluoromethyl, methoxy, ethoxy, or isopropoxy.

In some embodiments, $R_{x2}$ is $C_{1-6}$ alkyl. In some embodiments, $R_{x2}$ is methyl or ethyl. In some embodiments, $R_{x2}$ is methyl.

In some embodiments, X is O. In some embodiments, X is $-CH_2-$. In some embodiments, X is S. In some embodiments, X is -NH-.

In another embodiment, the present invention provides the compounds of formula (IV-2), (IV-2a), or (IV-2b), or pharmaceutically acceptable salts, isotope variants, tautomers, or stereoisomers thereof:

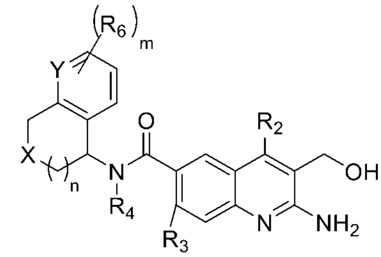
(IV-2)

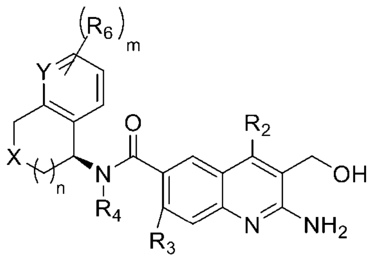
(IV-2a) or

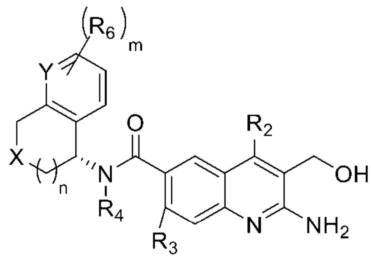
(IV-2b)

wherein,

$R_2$ is selected from H, methyl, ethyl, methoxy, or F;
$R_3$ is selected from H, F, Cl, CN, or methyl;
$R_4$ is selected from H, $C_{1-6}$ alkyl, $C_{1-6}$ haloalkyl, $C_{3-6}$ cycloalkyl, or 4-12 membered heterocyclyl;
$R_6$ is selected from H, CN, halogen, $C_{1-6}$ alkyl, $C_{1-6}$ haloalkyl, $SF_5$, $OR_b$, $SCF_3$, or 5-6 membered heteroaryl;
$R_b$ is selected from H, $C_{1-6}$ alkyl, or $C_{1-6}$ haloalkyl;
X is selected from $-CH_2-$, O, S, or $-NR_c-$;
Y is selected from CH or N;
$R_c$ is selected from H or $C_{1-6}$ alkyl;
m is 0, 1, or 2;
n is 0, 1, 2, or 3.

In another more specific embodiment, the present invention provides the above-described compounds, or pharmaceutically acceptable salts, isotope variants, tautomers, or stereoisomers thereof, wherein:

$R_2$ is selected from H, methyl, ethyl, methoxy, or F;
$R_3$ is selected from H, F, Cl, CN, or methyl;
$R_4$ is selected from H, methyl, ethyl, trifluoromethyl, trifluoroethyl, cyclopropyl, cyclobutyl, azetidinyl, oxetanyl, or

tetrahydrofuran;
$R_6$ is selected from H, CN, F, Cl, methyl, ethyl, trifluoromethyl, trifluoroethyl, SFs, methoxy, trifluoromethoxy, difluoromethoxy, pyridyl,

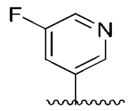
,

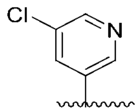

or

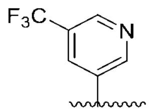
;

X is selected from $-CH_2-$, O, S, or -NH-;
Y is selected from CH or N;
m is 1;
n is 0, 1, 2, or 3.

In some embodiments, $R_2$ is selected from H, methyl, or ethyl. In some embodiments, $R_2$ is methyl. In some embodiments, $R_2$ is ethyl.

In some embodiments, $R_3$ is selected from H, F, or Cl. In some embodiments, $R_3$ is F or Cl. In some embodiments, $R_3$ is F. In some embodiments, $R_3$ is Cl.

In some embodiments, $R_4$ is selected from H or $C_{1-6}$ alkyl. In some embodiments, $R_4$ is selected from $C_{1-6}$ alkyl. In some embodiments, $R_4$ is selected from methyl or ethyl.

In some embodiments, $R_6$ is selected from H, halogen, $C_{1-6}$ alkyl, $C_{1-6}$ haloalkyl, $C_{1-6}$ haloalkoxy, or 5-6 membered heteroaryl. In some embodiments, $R_6$ is selected from halogen, $C_{1-6}$ haloalkyl, $C_{1-6}$ haloalkoxy, or 5-6 membered heteroaryl. In some embodiments, $R_6$ is selected from H, F, Cl, trifluoromethyl, trifluoroethyl, difluoromethoxy, trifluoromethoxy, pyridyl,

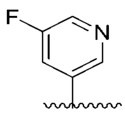
,

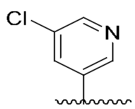

or

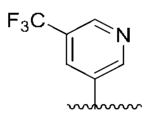
.

In some embodiments, X is selected from O or S; and Y is selected from CH or N. In some embodiments, X is O; and Y is CH or N. In some embodiments, X is O; and Y is N.

In some embodiments, m is 1.

In some embodiments, n is 0. In some embodiments, n is 1.

In another embodiment, the present invention provides a compound of formula (IV-3), (IV-3a), or (IV-3b), or a

pharmaceutically acceptable salt, isotope variant, tautomer, or stereoisomer thereof:

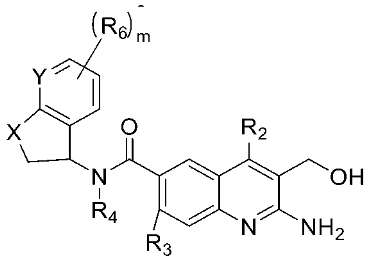

(IV-3)

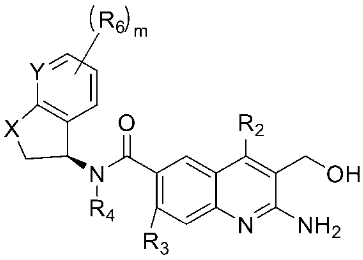

(IV-3a) or

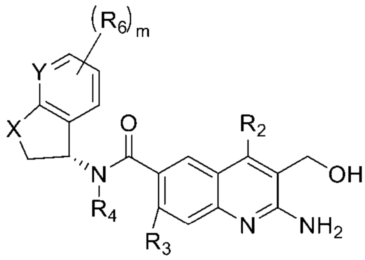

(IV-3b)

wherein,

$R_2$ is selected from H, methyl, ethyl, methoxy, or F;
$R_3$ is selected from H, F, Cl, CN, or methyl;
$R_4$ is selected from H, $C_{1-6}$ alkyl, deuterated $C_{1-6}$ alkyl, $C_{1-6}$ haloalkyl, $C_{3-6}$ cycloalkyl, or a 4-12 membered heterocyclyl;
$R_6$ is selected from H, CN, halogen, $C_{1-6}$ alkyl, $C_{1-6}$ haloalkyl, $SF_5$, $OR_b$, $SCF_3$, or 5-6 membered heteroaryl;
$R_b$ is selected from H, $C_{1-6}$ alkyl, or $C_{1-6}$ haloalkyl;
X is selected from $-CH_2-$, O, S, or $-NR_c-$;
Y is selected from CH or N;
$R_c$ is selected from H or $C_{1-6}$ alkyl;
m is 0, 1, or 2.

In another more specific embodiment, the present invention provides the above-described compounds, or pharmaceutically acceptable salts, isotope variants, tautomers, or stereoisomers thereof, wherein:

$R_2$ is selected from H, methyl, ethyl, methoxy, or F;
$R_3$ is selected from H, F, Cl, CN, or methyl;
$R_4$ is selected from H, methyl, methyl-$d_3$, ethyl, trifluoromethyl, trifluoroethyl, cyclopropyl, cyclobutyl, azetidine, oxetane, or tetrahydrofuran;
$R_6$ is selected from H, CN, F, Cl, methyl, ethyl, trifluoromethyl, trifluoroethyl, SFs, methoxy, trifluoromethoxy, difluoromethoxy, pyridyl,

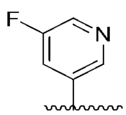

,

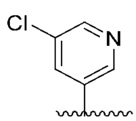

or

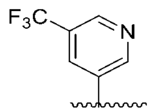;

X is selected from $-CH_2-$, O, S, or -NH-;
Y is selected from CH or N;
m is 1.

In some embodiments, $R_2$ is selected from H, methyl, or ethyl. In some embodiments, $R_2$ is methyl. In some embodiments, $R_2$ is ethyl.

In some embodiments, $R_3$ is selected from H, F, or Cl. In some embodiments, $R_3$ is F or Cl. In some embodiments, $R_3$ is F. In some embodiments, $R_3$ is Cl.

In some embodiments, $R_4$ is selected from H or $C_{1-6}$ alkyl. In some embodiments, $R_4$ is selected from $C_{1-6}$ alkyl. In some embodiments, $R_4$ is selected from methyl, methyl-$d_3$, or ethyl.

In some embodiments, $R_6$ is selected from H, halogen, $C_{1-6}$ alkyl, $C_{1-6}$ haloalkyl, or $C_{1-6}$ haloalkoxy. In some embodiments, $R_6$ is selected from halogen, $C_{1-6}$ haloalkyl, or $C_{1-6}$ haloalkoxy. In some embodiments, $R_6$ is selected from H, F, Cl, trifluoromethyl, trifluoroethyl, difluoromethoxy, or trifluoromethoxy.

In some embodiments, X is selected from O or S; and Y is selected from CH or N. In some embodiments, X is O; and Y is CH or N. In some embodiments, X is O; and Y is CH.

In some embodiments, m is 1.

In another more specific embodiment, the present invention provides a compound, or a pharmaceutically acceptable salt, isotope variant, tautomer, or stereoisomer thereof, wherein the compound is selected from:

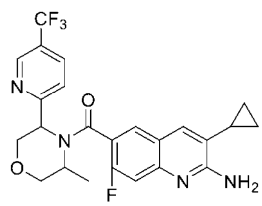,
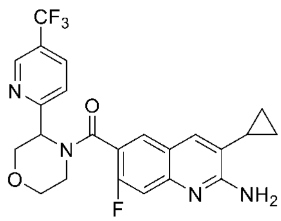,
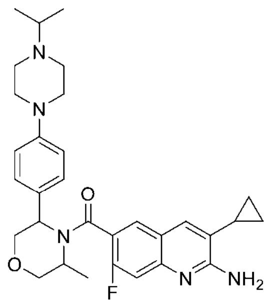,
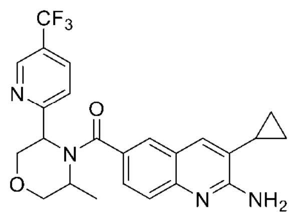,

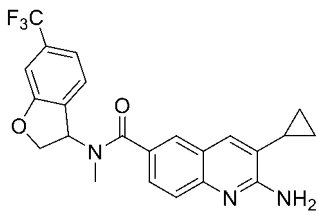,
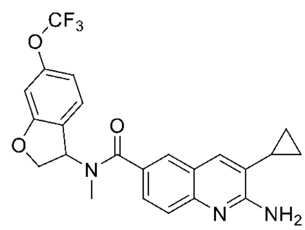,
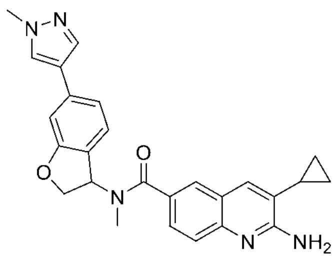,

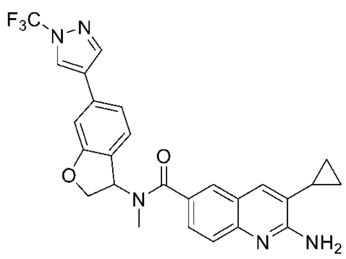,
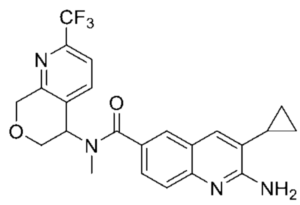,
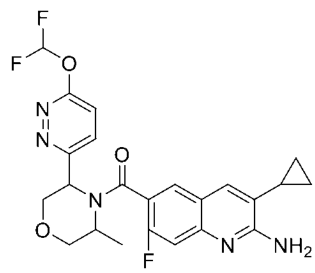,

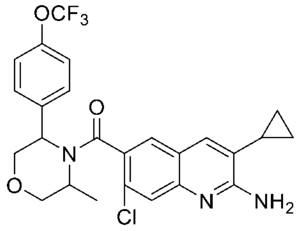, 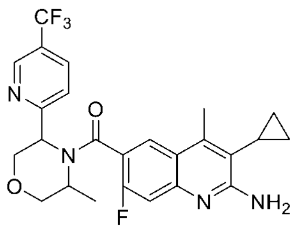, 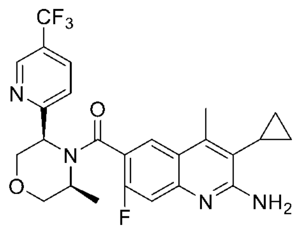,

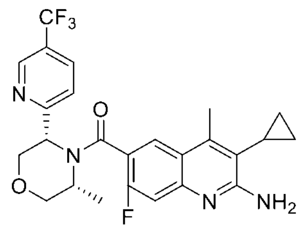, 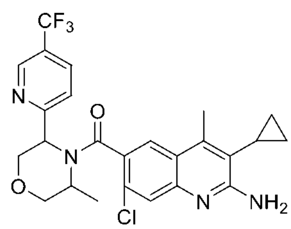, 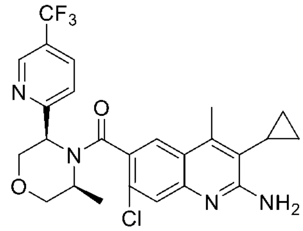, 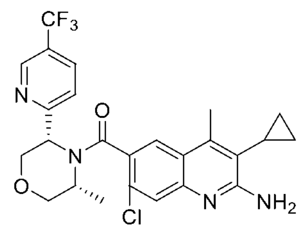,

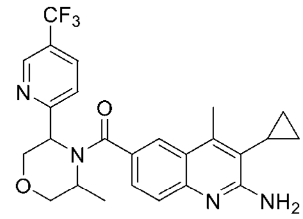, 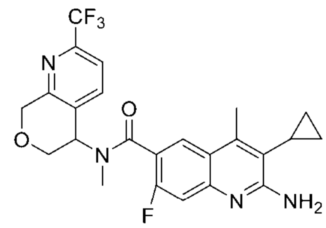, 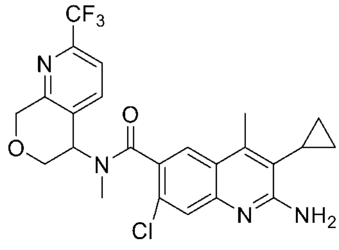,

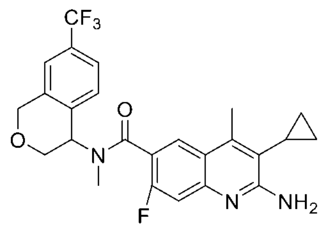, 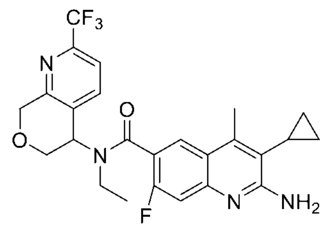, 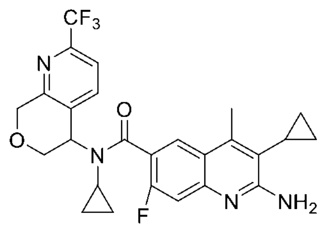,

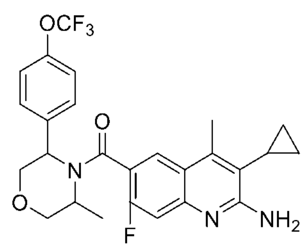, 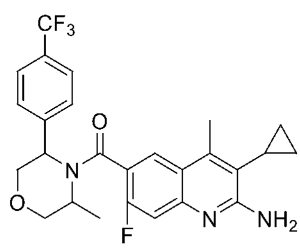, 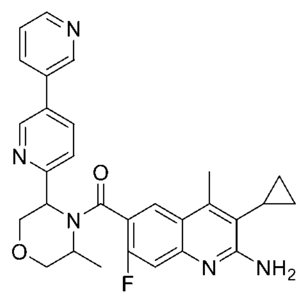,

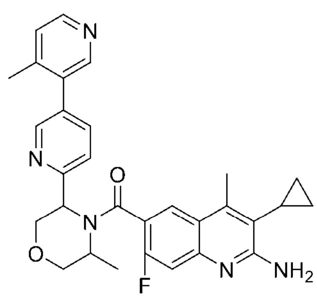, 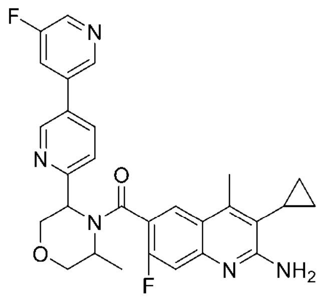, 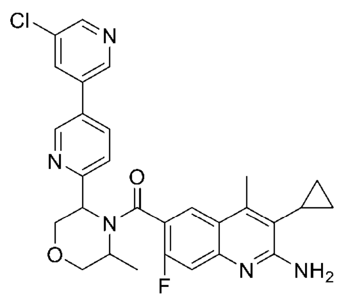,

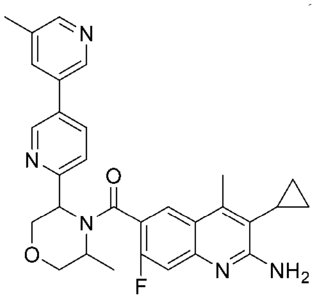, 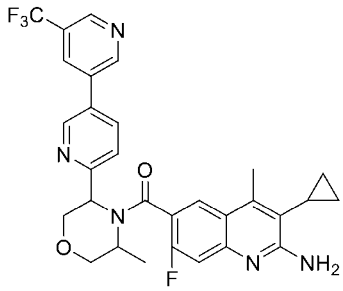, 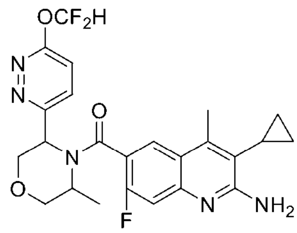,

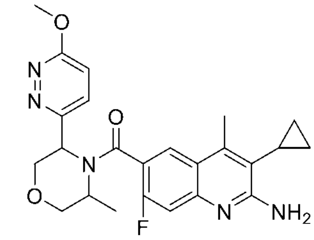, 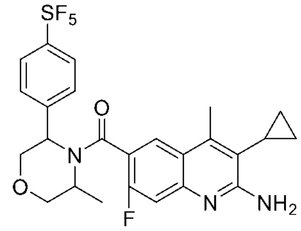, 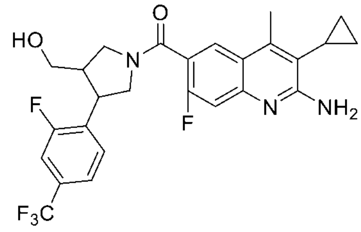,

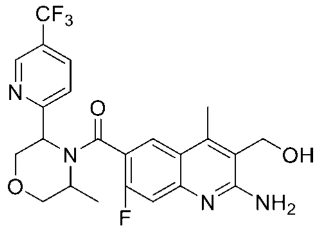, 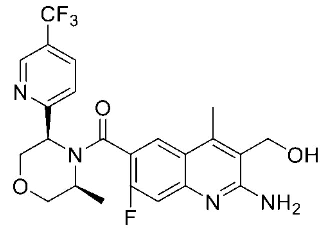, 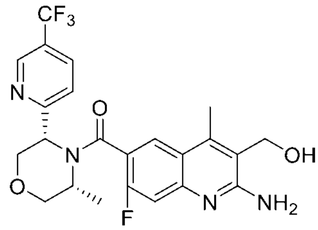,

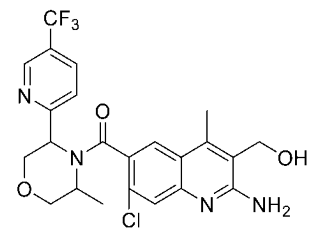, 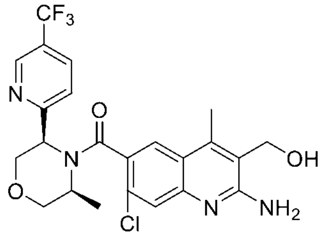, 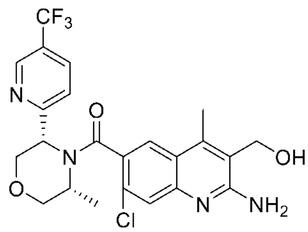,

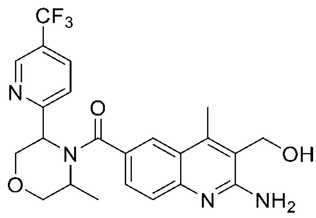, 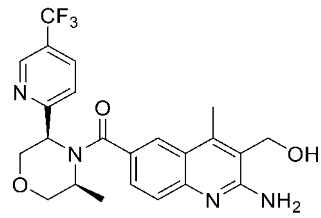, 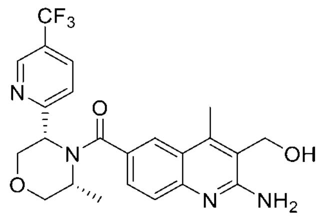,

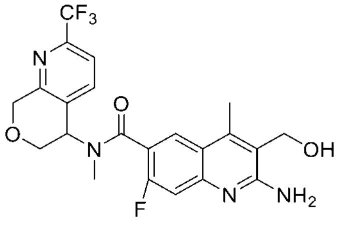, 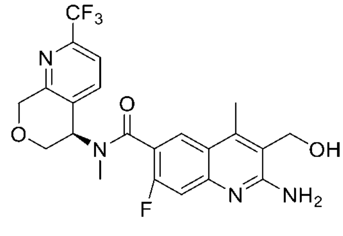, 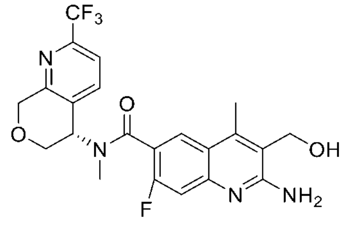,

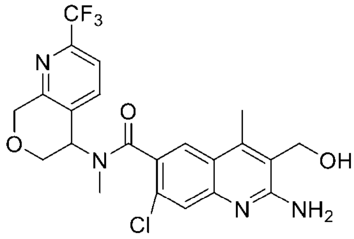,
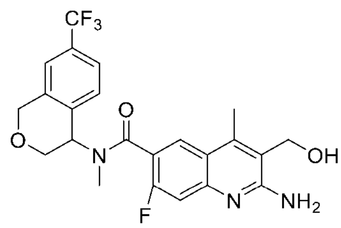,
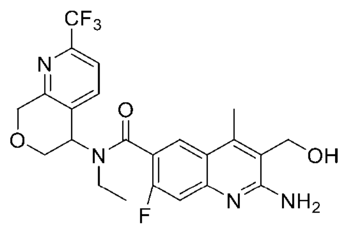,

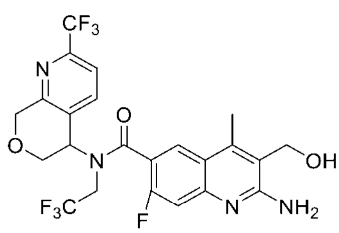,
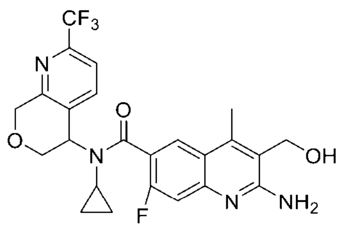,
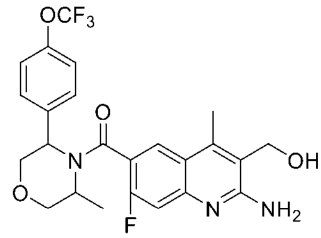,

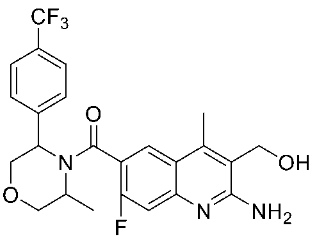,
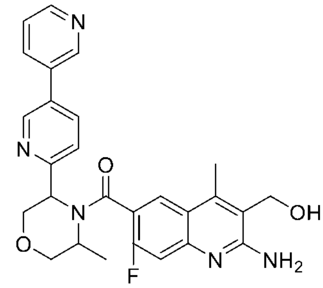,
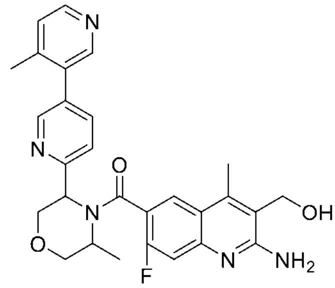,

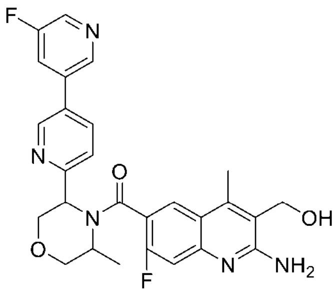,
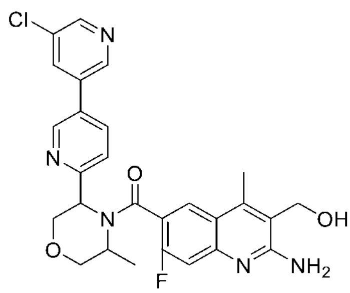,
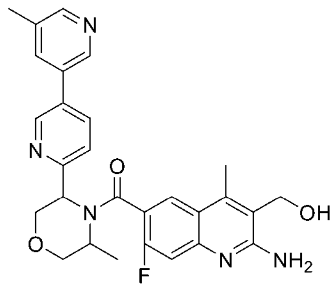,

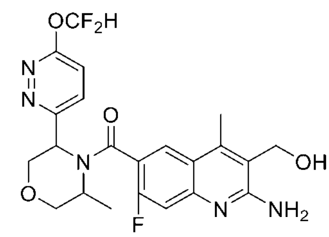,
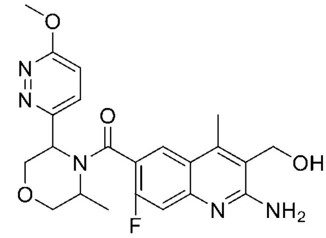,
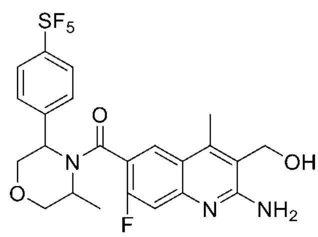,

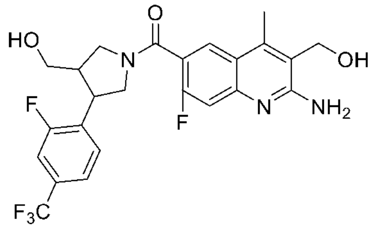,
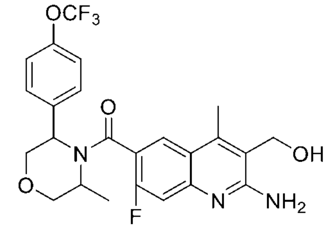,
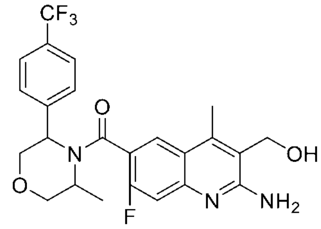,

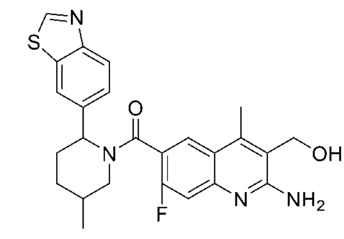 , 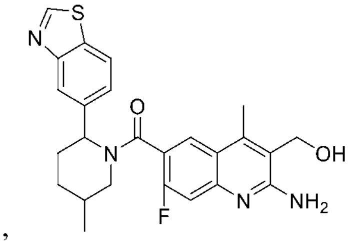 , 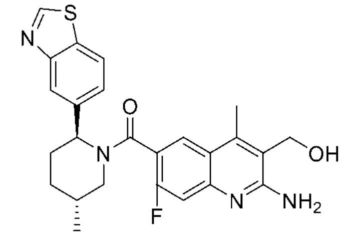 ,

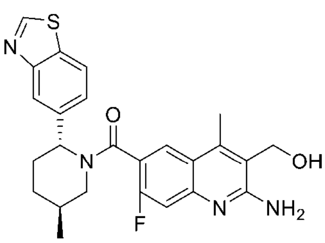 , 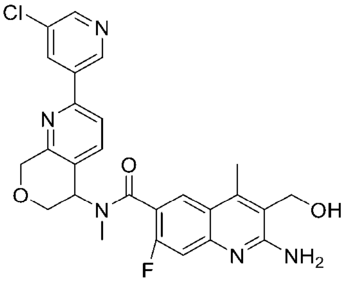 , 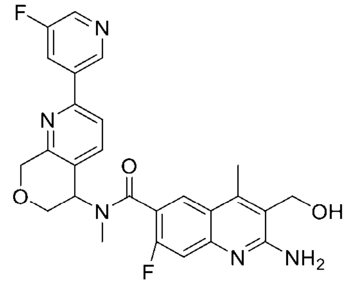 ,

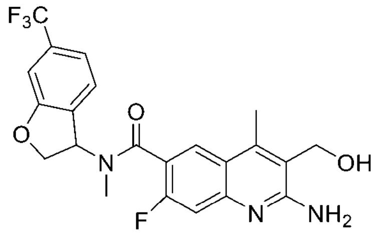 , 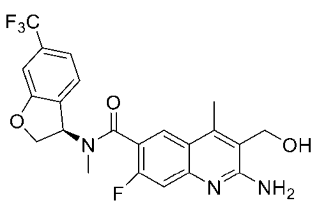 , 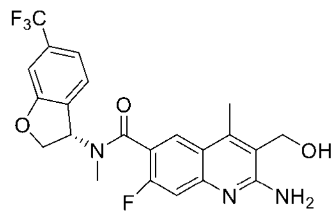 ,

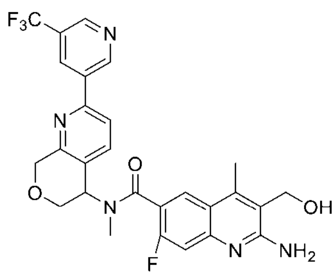 , 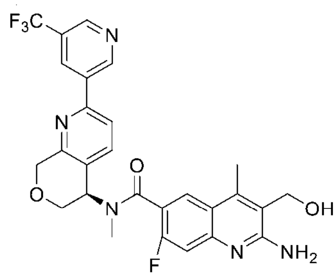 , 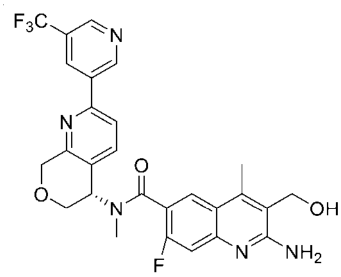 ,

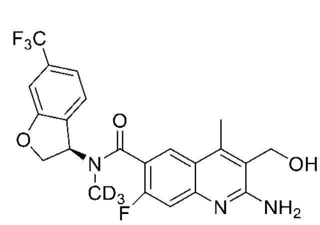 , 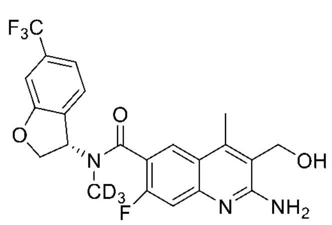 , 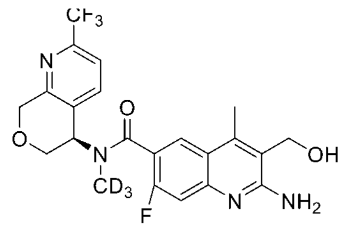 ,

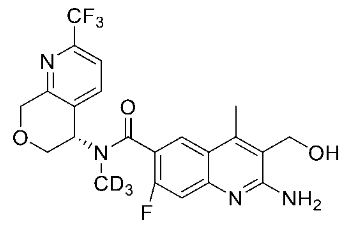 , 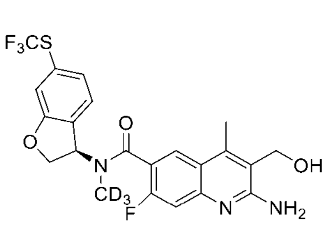 , 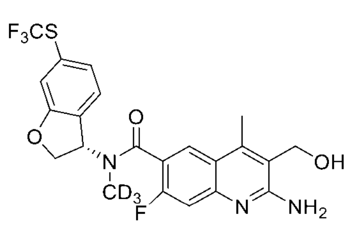 ,

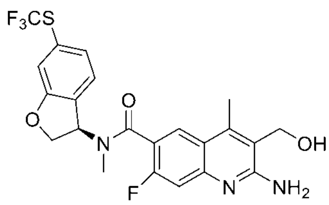 or 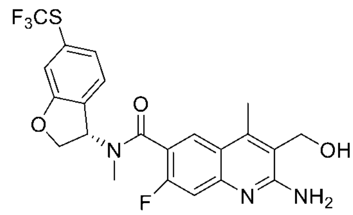 .

The compound of the present invention may include one or more asymmetric centers and therefore may exist in various stereoisomeric forms, such as enantiomers and/or diastereomers. For example, the compound of the present invention may exist as a single enantiomer, diastereomer, or geometric isomer (e.g., cis- and trans-isomers), or it may be in the form of a mixture of stereoisomers, including a racemic mixture and a mixture enriched in one or more stereoisomers. Isomers can be separated from mixtures using methods known to those skilled in the art, including: chiral high-performance liquid chromatography (HPLC) and the formation and crystallization of chiral salts; alternatively, preferred isomers can be prepared through asymmetric synthesis.

The compound of the present invention may also exist as tautomers. For compounds that exist in different tautomeric forms, a given compound is not limited to any specific tautomer but is intended to encompass all tautomeric forms.

The present invention also includes isotopically labeled compounds (isotopologs) that are equivalent to those described in Formula (A), except that one or more atoms are replaced by atoms with atomic mass or mass number different from those commonly found in nature. Examples of isotopes that can be incorporated into the compounds of the present invention include isotopes of hydrogen, carbon, nitrogen, oxygen, phosphorus, sulfur, fluorine, and chlorine, such as $^{2}H$, $^{3}H$, $^{13}C$, $^{11}C$, $^{14}C$, $^{15}N$, $^{18}O$, $^{17}O$, $^{31}P$, $^{32}P$, $^{35}S$, $^{18}F$, and $^{36}Cl$, respectively. Compounds of the present invention containing the aforementioned isotopes and/or other atomic isotopes, their prodrugs, and the pharmaceutically acceptable salts of such compounds or prodrugs, are all within the scope of the present invention. Certain isotopically labeled compounds of the present invention, such as those incorporating radioactive isotopes (e.g., $^{3}H$ and $^{14}C$), may be used for drug and/or substrate tissue distribution studies. Tritium ($^{3}H$) and carbon-14 ($^{14}C$) isotopes are particularly preferred because they are easy to prepare and detect. Furthermore, substitution with heavier isotopes, such as deuterium ($^{2}H$), may provide therapeutic benefits due to increased metabolic stability, such as prolonged in vivo half-life or reduced dosage requirements, and may therefore be preferred in certain cases. Isotopically labeled compounds of Formula (A) of the present invention and their prodrugs can generally be prepared by substituting readily available isotopically labeled reagents for non-isotopically labeled reagents in the following procedures and/or in the processes disclosed in the examples and preparation methods.

Pharmaceutical Composition and Kit

In another aspect, the present invention provides a pharmaceutical composition comprising a compound of the present invention (also referred to as the "active ingredient") and a pharmaceutically acceptable excipient. In some embodiments, the pharmaceutical composition comprises an effective amount of the compound of the present invention. In some embodiments, the pharmaceutical composition comprises a therapeutically effective amount of the compound of the present invention. In some embodiments, the pharmaceutical composition comprises a prophylactically effective amount of the compound of the present invention.

A pharmaceutically acceptable excipient used in the present invention refers to a non-toxic carrier, adjuvant, or medium that does not destroy the pharmacological activity of the compound that is formulated with it. Pharmaceutically acceptable carriers, adjuvants, or media that can be used in the compositions of the present invention include (but are not limited to) ion exchange resins, aluminum oxide, aluminum stearate, lecithin, serum proteins (such as human serum albumin), buffering agents (such as phosphates), glycine, sorbic acid, potassium sorbate, mixtures of partial glycerides of saturated vegetable fatty acids, water, salts or electrolytes (such as protamine sulfate), disodium phosphate, dipotassium phosphate, sodium chloride, zinc salts, silica gel, magnesium trisilicate, polyvinylpyrrolidone, cellulose-based substances, polyethylene glycol, sodium carboxymethyl cellulose, polyacrylates, waxes, polyethylene-polyoxypropylene block polymers, polyethylene glycol, and lanolin.

The present invention also includes a kit (e.g., pharmaceutical packaging). The provided kit may include the compound of the present invention, other therapeutic agents, and first and second containers (e.g., vials, ampoules, bottles, syringes, and/or dispersible packaging or other suitable containers) containing the compound of the present invention and other therapeutic agents. In some embodiments, the provided kit may optionally include a third container containing a pharmaceutical excipient for diluting or suspending the compound of the present invention and/or other therapeutic agents. In some embodiments, the compound of the present invention and other therapeutic agents provided in the first and second containers may be combined to form a single unit dosage form.

Drug Administration

The pharmaceutical composition provided by the present invention can be administered through various routes, including but not limited to: oral administration, parenteral administration, inhalation administration, topical administration, rectal administration, nasal administration, buccal administration, vaginal administration, administration via implants, or other administration methods. For example, as used herein, parenteral administration includes subcutaneous administration, intradermal administration, intravenous administration, intramuscular administration, intra-articular administration, intra-arterial administration, intra-synovial administration, intra-sternal administration, intrathecal administration, intralesional administration, and intracranial injection or infusion techniques.

Generally, an effective amount of the compounds provided herein is administered. As appropriate under related conditions, including the disease being treated, the selected route of administration, the actual compound administered, the individual patient's age, weight, and response, as well as the severity of the patient's symptoms, the actual amount of the compound administered may be determined by a physician.

When used to prevent a disease described in the present invention, the compound provided herein is administered to a subject at risk of developing the disease, typically based on a physician's recommendation and under a physician's supervision, at dosage levels as described above. A subject at risk of developing a specific disease typically includes those with a family history of the disease or those identified as particularly susceptible to developing the disease through genetic testing or screening.

The pharmaceutical composition provided herein may also be administered over a long-term period ("long-term administration"). Long-term administration refers to the administration of a compound or its pharmaceutical composition over an extended period, such as 3 months, 6 months, 1 year, 2 years, 3 years, 5 years, etc., or indefinitely, such as for the subject's lifetime. In some embodiments, long-term administration is intended to provide a stable level of the compound in the bloodstream over an extended period, for example, within the therapeutic window.

Various methods of administration may be used to further deliver the pharmaceutical composition of the present invention. For example, in some embodiments, the pharmaceutical composition may be administered via bolus injection, for instance, to increase the concentration of the compound in the blood to an effective level. The bolus injection dose depends on the target systemic level of the active component in the body. For example, an intramuscular or subcutaneous bolus injection dose allows the active component to be released slowly, whereas direct intravenous bolus injection (e.g., via IV dripping) enables a more rapid delivery, quickly raising the concentration of the active component in the blood to an effective level. In other embodiments, the pharmaceutical composition may be administered in the form of continuous infusion, such as via IV dripping, thereby maintaining a steady-state concentration of the active component in the subject's body. Additionally, in other embodiments, a bolus dose of the pharmaceutical composition may first be administered, followed by continuous infusion.

The composition for oral administration may be in the form of a bulk liquid solution, suspension, or bulk powder. However, more commonly, to facilitate accurate dosing, the composition is provided in a unit dosage form. The term "unit dosage form" refers to a physically discrete unit suitable as a single dose for human patients and other mammals, each unit containing a predetermined amount of the active substance and suitable pharmaceutical excipients that is appropriate for achieving the desired therapeutic effect. Typical unit dosage forms include pre-filled, pre-measured ampoules or syringes for the liquid composition, or tablets, pills, capsules, etc., for the solid composition. In such a composition, the compound typically constitutes a minor component (about 0.1 to about 50% by weight, preferably about 1 to about 40% by weight), with the remaining portion comprising various carriers or excipients and processing aids that are useful for forming the desired dosage form.

For oral dosages, a representative regimen is one to five oral doses, particularly two to four doses, typically three oral doses per day. Using these dosage forms, each dose provides about 0.01 to about 20 mg/kg of the compound of the present invention, and a preferred dose provides about 0.1 to about 10 mg/kg, especially about 1 to about 5 mg/kg thereof.

To achieve blood levels similar to or lower than those obtained with injectable doses, a transdermal dose is typically chosen, ranging from about 0.01 to about 20% by weight, preferably about 0.1 to about 20% by weight, more preferably about 0.1 to about 10% by weight, and most preferably about 0.5 to about 15% by weight.

For about 1 to about 120 hours, especially 24 to 96 hours, the injection dose level ranges from about 0.1 mg/kg/hour to at least 10 mg/kg/hour. To achieve adequate steady-state levels, a pre-loading bolus dose of about 0.1 mg/kg to about 10 mg/kg or more may also be administered. For human patients weighing 40 to 80 kg, the maximum total dose should not exceed about 2 g/day.

A liquid form suitable for oral administration may include an appropriate aqueous or non-aqueous carrier, as well as a buffering agent, suspending agent and dispersing agent, colorant, flavoring agent, etc. A solid form may include, for example, any of the following components or compounds with similar properties: a binder such as microcrystalline cellulose, tragacanth gum, or gelatin; an excipient such as starch or lactose; a disintegrant such as alginic acid, Primogel, or corn starch; a lubricant such as magnesium stearate; a glidant such as colloidal silicon dioxide; a sweetener such as sucrose or saccharin; or a flavoring agent such as mint, methyl salicylate, or orange flavor.

An injectable composition is typically based on sterile saline or phosphate-buffered saline for injection, or other injectable excipients known in the field. As mentioned above, in such a composition, the active compound typically constitutes a minor component, often at about 0.05 to 10% by weight, with the remainder consisting of injectable excipients, etc.

Typically, a transdermal composition is formulated as a topical ointment or cream containing the active ingredient. When formulated as an ointment, the active ingredient is typically combined with paraffin or may be combined with a water-miscible ointment matrix. Alternatively, the active ingredient may be formulated as a cream, for example, with a water-encapsulated-oil cream matrix. Such transdermal formulations are well known in the field and generally include other components to enhance the stability and skin penetration of the active ingredient or formulation. All such known transdermal formulations and components are included in the scope of the present invention.

The compound of the present invention may also be administered via a transdermal device. Thus, transdermal administration can be achieved using a reservoir or porous membrane-type patch, or patch with various solid matrices.

The above-mentioned components for the composition for oral, injection, or topical administration are merely representative. Other materials and processing techniques are described in Part 8 of Remington's Pharmaceutical Sciences, 17th edition, 1985, Mack Publishing Company, Easton, Pennsylvania, which is incorporated herein by reference.

The compound of the present invention may also be administered in a sustained-release form or from a sustained-release drug delivery system. Descriptions of representative sustained-release materials can be found in Remington's Pharmaceutical Sciences.

The present invention also relates to pharmaceutically acceptable formulations of the compounds of the present invention. In one embodiment, the formulation contains water. In another embodiment, the formulation contains a cyclodextrin derivative. The most common cyclodextrins are α-, β-, and γ-cyclodextrins, which consist of six, seven, and eight α-1,4-linked glucose units, respectively, and optionally include one or more substituents on the linked sugar portion, including but not limited to: methylated, hydroxyalkylated, acylated, and sulfoalkyl ether substitutions. In some embodiments, the cyclodextrin is a sulfoalkyl ether β-cyclodextrin, such as sulfobutyl ether β-cyclodextrin, also known as Captisol. See, for example, U.S. 5,376,645. In some embodiments, the formulation includes hexapropyl-β-cyclodextrin (e.g., 10-50% in water).

## Examples

The reagents used in the present invention are commercially available reagents purchased directly or synthesized by commonly known methods in the field.

Interpretation of Common Abbreviations:

PE = petroleum ether; EA = ethyl acetate; MeOH = methanol; DCM = dichloromethane; DCE = dichloroethane; $CH_3CN$ = acetonitrile; 1,4-dioxane = 1,4-dioxane; DMSO = dimethyl sulfoxide; HFIP = hexafluoroisopropanol; DMF = N,N-dimethylformamide; Hex = n-hexane; IPA = isopropanol; NMP = N-methylpyrrolidone; NMO = N-methylmorpholine-N-oxide; TEA = triethylamine; DIEA = diisopropylethylamine; CuI = copper(I) iodide; CuCN = copper(I) cyanide; triphosgene = triphosgene; p-TsOH = p-toluenesulfonic acid; TBAS = tetrabutylammonium hydrogen sulfate.

The following specific reaction routes or steps are used in the present invention, as detailed below:

### Example 1

### Preparation of Key Intermediates a1-a6

Synthesis of Intermediates **a1, a2**

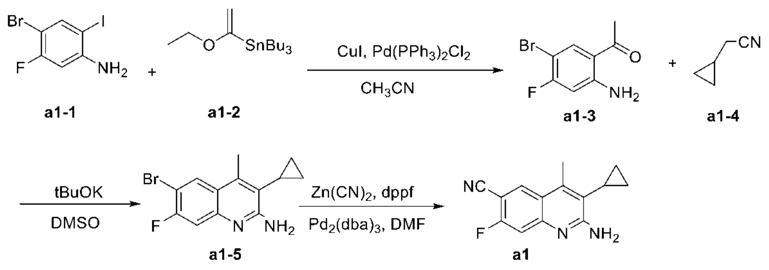

a1-1 a1-2 a1-3 a1-4 a1-5 a1

Step 1: Under nitrogen protection, 2-iodo-4-bromo-5-fluoroaniline **a1-1** (10.0 g, 31.6 mmol), tributyl(1-ethoxyvinyl)tin **a1-2** (13.0 mL), and CuI (600 mg, 3.2 mmol) were dissolved in 200 mL of acetonitrile, and the catalyst $Pd(PPh_3)_3Cl_2$ (2.2 g, 3.17 mmol) was added, and the mixture was allowed to react at 80°C for 2 hours before the reaction was stopped. The reaction mixture was filtered, and the solvent was removed under reduced pressure. The crude product was directly purified by flash column chromatography (PE/EA, 10/1) to obtain the compound **a1-3** (5.0 g), with a yield of: 68%. LCMS ESI-MS m/z: 232 $[M+H]^+$.

Step 2: The previous step's intermediate **a1-3** (5.0 g, 21.5 mmol) and cyclopropyl acetonitrile **a1-4** (3.5 g, 43.1 mmol) were dissolved in 50 mL of DMSO. Potassium tert-butoxide (4.8 g, 43.1 mmol) was added, and the mixture was allowed to react at 50°C for 2 hours before the reaction was stopped. 200 mL of water was added to the reaction system, followed by extraction with ethyl acetate. The organic phase was dried over anhydrous sodium sulfate and concentrated. The crude product was directly purified by flash column chromatography (PE/EA, 1/1) to obtain the compound **a1-5** (1.2 g), with a yield of: 19%. LCMS ESI-MS m/z: 295 $[M+H]^+$.

Step 3: Under nitrogen protection, the previous step's compound **a1-5** (1.2 g, 4.1 mmol), $Zn(CN)_2$ (600 mg, 4.88 mmol), and 1,1'-bis(diphenylphosphino)ferrocene (dppf) (200 mg, 0.41 mmol) were dissolved in 24 mL of DMF. The catalyst $Pd_2(dba)_3$ (0.04 mL, 0.13 mmol) was added, and the mixture was heated to 100°C and allowed to react for 2 hours before the reaction was stopped. 100 mL of water was added to the reaction system, followed by extraction with ethyl acetate. The organic phase was dried over anhydrous sodium sulfate and concentrated. The crude product was directly purified by flash column chromatography (acetonitrile/water, 4/5) to obtain the compound **a1** (150 mg), with a yield of: 15%. LCMS ESI-MS m/z: 242 $[M+H]^+$.

By referring to the synthetic route of the intermediate **a1**, the following intermediate was synthesized.

| Target Intermediate | Intermediate Structure | LC-MS $[M+H]^+$ |
|---|---|---|
| **a2** | 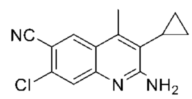 | 258 |

Synthesis of Intermediates **a3-a5:**

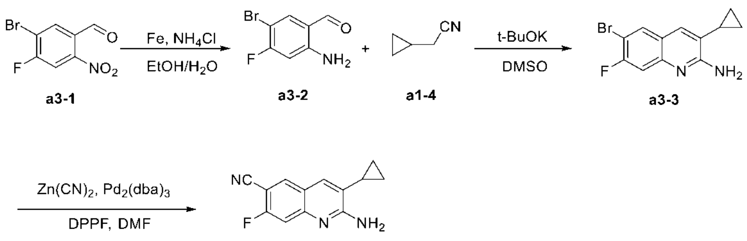

a3-1 a3-2 + a1-4 a3-3

a3

Step 1: The compound **a3-1** (7.0 g, 28.2 mmol) was dissolved in a mixed solution of 70 mL ethanol and 70 mL water. Reduced iron powder (7.9 g, 141 mmol) and ammonium chloride (15.1 g, 282 mmol) were added. The reaction mixture was heated to reflux reaction for 3 hours before the reaction was stopped, followed by filtering. 400 mL of ice water was added to the reaction system, followed by extraction with ethyl acetate. The organic phase was concentrated, and the crude product was purified by column chromatography to obtain a yellow solid **a3-2** (5.3 g, 24.3 mmol), with a yield of: 86%. LCMS ESI-MS m/z: 218 $[M+H]^+$.

Step 2: The previous step's intermediate **a3-2** (5.3 g, 24.3 mmol) and cyclopropyl acetonitrile **a1-4** (3.9 g, 48.6 mmol) were dissolved in 55 mL of DMSO. After stirring for 5 minutes, potassium tert-butoxide (5.5 g, 48.6 mmol) was added to the reaction system, and the mixture was heated to 50°C to react for 2 hours. 150 mL of ice water was added to the reaction system, followed by extraction with ethyl acetate. The organic phase was dried over anhydrous sodium sulfate and concentrated. The crude product was purified by column chromatography to obtain a yellow solid, **a3-3** (3.3 g, 11.7 mmol), with a yield of: 48%. LCMS ESI-MS m/z: 281 $[M+H]^+$.

Step 3: Under nitrogen protection, the previous step's intermediate **a3-3** (3.3 g, 11.7 mmol), catalyst $Pd_2(dba)_3$ (0.5 g, 0.59 mmol), and dppf (0.7 g, 1.2 mmol) were dissolved in 66 mL of DMF. $Zn(CN)_2$ (1.7 g, 14.1 mmol) was added, and the mixture was heated to 100°C to react for 2 hours, followed by cooling to room temperature and filtering. 200 mL of ice water was added to the reaction system, followed by extraction with ethyl acetate. The organic phase was dried over anhydrous sodium sulfate and concentrated. The crude product was purified by flash column chromatography to obtain a

yellow solid **a3** (1.1 g, 4.84 mmol). Yield: 41%. LCMS ESI-MS m/z: 228 [M+H]$^+$.

By referring to the synthetic route of the intermediate **a3,** the following intermediates were synthesized.

| Target Intermediate | Intermediate Structure | LC-MS [M+H]$^+$ |
|---|---|---|
| **a4** | 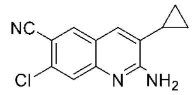 | 244 |
| **a5** | 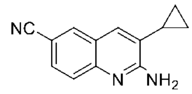 | 210 |

Synthesis of Intermediate **a6:**

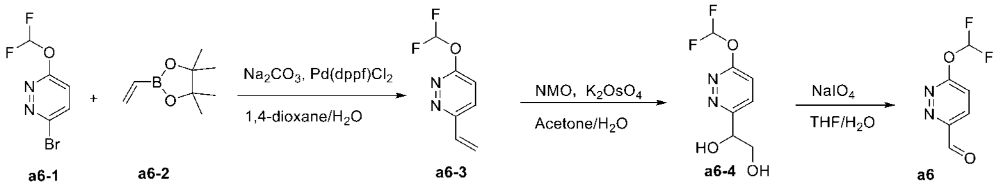


a6-1 a6-2 a6-3 a6-4 a6

Step 1: Under nitrogen protection, the starting materials 3-bromo-6-difluoromethoxypyrazine **a6-1** (1.0 g, 4.5 mmol), vinylboronic acid pinacol ester **a6-2** (2.0 g, 13.5 mmol), and sodium carbonate (1.4 g, 13.6 mmol) were dissolved in 20 mL of a mixed solution of 1,4-dioxane and water (v/v, 8/1). Catalyst Pd(dppf)$Cl_2$ (0.3 g, 0.45 mmol) was added, and the mixture was heated to 80°C to react for 12 hours, followed by cooling to room temperature and filtering. 100 mL of ice water was added to the reaction system, followed by extraction with dichloromethane. The organic phase was dried over anhydrous sodium sulfate and concentrated. The crude product was purified by flash column chromatography (PE/EA, 5/1) to obtain a white solid **a6-3** (730 mg), with a yield of: 94%. LCMS ESI-MS m/z: 262 [M+H]$^+$.

Step 2: The previous step's intermediate **a6-3** (720 mg, 4.2 mmol) was dissolved in 12 mL of a mixed solution of acetone and water (v/v, 5/1). N-methylmorpholine N-oxide (NMO) (1.7 g, 14.6 mmol) and $K_2OsO_4 \cdot 2H_2O$ (154 mg, 0.41 mmol) were added, and the reaction was carried out at room temperature for 1 hour, and then the reaction was stopped and filtering was performed. 100 mL of ice water was added to the reaction system, followed by extraction with dichloromethane. The organic phase was dried over anhydrous sodium sulfate and concentrated. The crude product was purified by flash column chromatography (PE/EA, 2/1) to obtain a white solid **a6-4** (360 mg), with a yield of: 42%. LCMS ESI-MS m/z: 207 [M+H]$^+$.

Step 3: The previous step's intermediate **a6-4** (360 mg, 1.74 mmol) was dissolved in 17 mL of a mixed solution of tetrahydrofuran and water (v/v, 11/1). Sodium periodate ($NaIO_4$) (11.2 g, 5.24 mmol) was added, and the reaction was carried out at room temperature for 1 hour, and then the reaction was stopped and filtering was performed. 50 mL of ice water was added to the reaction system, followed by extraction with dichloromethane. The organic phase was dried over anhydrous sodium sulfate and concentrated to obtain a white solid **a6** (290 mg), with a yield of: 95%. LCMS ESI-MS m/z: 175 [M+H]$^+$.

**Preparation of Key Intermediates b1-b7**

Synthesis of Intermediates **b1-b5**

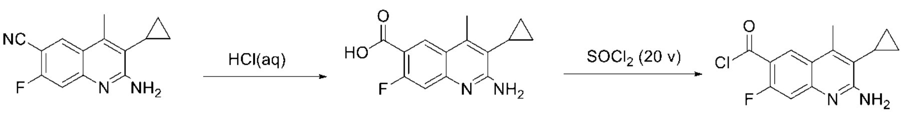


a1 b1-1 b1

Step 1: The intermediate **a1** (150 mg, 0.62 mmol) was dissolved in 3 mL of concentrated hydrochloric acid (35%)

and heated to 100°C to react for 2 hours. The solvent was removed under reduced pressure, and the crude product was purified by flash reverse-phase column chromatography (acetonitrile/water) to obtain a yellow solid **b1-1** (100 mg), with a yield of: 62%. LCMS ESI-MS m/z: 261 $[M+H]^+$.

Step 2: The previous step's intermediate **b1-1** (100 mg, 0.38 mmol) was dissolved in 2 mL of thionyl chloride, and the reaction mixture was heated to 70°C to react for 2 hours, and then the reaction was stopped. The solvent was removed under reduced pressure, yielding a yellow solid **b1** (80 mg), which was directly used for the next reaction step, with a yield of: 75%.

By referring to the synthetic route of the intermediate **b1**, the following intermediates were synthesized.

| Target Intermediate | Intermediate Structure |
|---|---|
| **b2** | 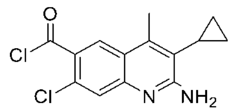 |
| **b3** | 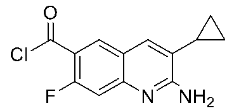 |
| **b4** | 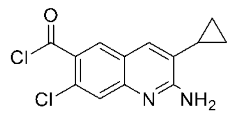 |
| **b5** | 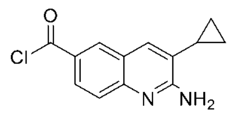 |

Synthesis of Intermediate **b6**

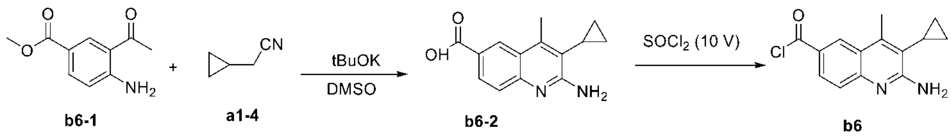

b6-1 a1-4 b6-2 b6

Step 1: The starting materials **b6-1** (1.2 g, 6.2 mmol) and cyclopropyl acetonitrile **a1-4** (1.0 g, 12.3 mmol) were dissolved in 24 mL of DMSO. After stirring for 5 minutes, potassium tert-butoxide (1.4 g, 12.4 mmol) was added, and the mixture was heated to 50°C to react for 2 hours. 80 mL of ice water was added to the reaction system, and the pH was adjusted to approximately 5 with dilute hydrochloric acid. The mixture was extracted with ethyl acetate, concentrated, and the crude product was purified by flash reverse-phase column chromatography (acetonitrile/water) to obtain a yellow solid **b6-2** (50 mg), with a yield of: 4%. LCMS ESI-MS m/z: 243 $[M+H]^+$.

Step 2: The intermediate **b6-2** (50 mg, 0.21 mmol) was dissolved in 0.5 mL of thionyl chloride, and the reaction mixture was heated to 70°C for 2 hours before stopping the reaction. The solvent was removed under reduced pressure, yielding a yellow solid **b6** (50 mg), which was directly used for the next reaction step. Yield: 93%.

Synthesis of Intermediate **b7**

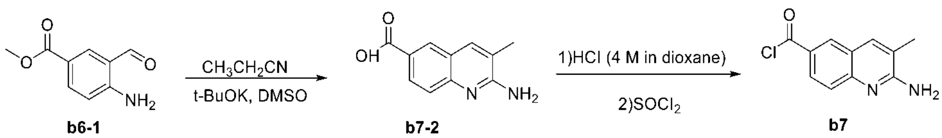

b6-1 b7-2 b7

Step 1: Under nitrogen protection, the starting material **b6-1** (3.5 g, 19.5 mmol) and propionitrile (2.1 g, 39.0 mmol) were dissolved in 35 mL of DMSO. tBuOK (4.3 g, 39.0 mmol) was added, and the reaction mixture was heated to 50°C to react for 2 hours before cooling to room temperature. 80 mL of ice water was added to the reaction system, followed by washing with ethyl acetate. The aqueous phase was adjusted to approximately pH 7 using 1M dilute hydrochloric acid, followed by filtering under vacuum. The filter cake was dried to obtain a yellow solid **b7-2** (3.5 g, 17.3 mmol), with a yield of: 89%. LCMS ESI-MS m/z: 203 $[M+H]^+$.

Step 2: The previous step's intermediate **b7-2** (500 mg, 2.4 mmol) was dissolved in 10 mL of dichloromethane, and 1,4-dioxane hydrochloride solution (0.7 mL, 4M) was added. The reaction was carried out at room temperature for 30 minutes, and the solvent was removed under reduced pressure. $SOCl_2$ (4412 mg, 37.0 mmol) was added to the reaction system, and the mixture was heated to 55°C to react for 1 hour before cooling to room temperature. The reaction mixture was diluted with 10 mL of dichloromethane, filtered under vacuum, washed with n-hexane, and dried to obtain the intermediate b7 (400 mg), with a yield of: 73%.

**Preparation of Key Intermediates c1-c9**

Synthesis of Intermediates **c1-c9**

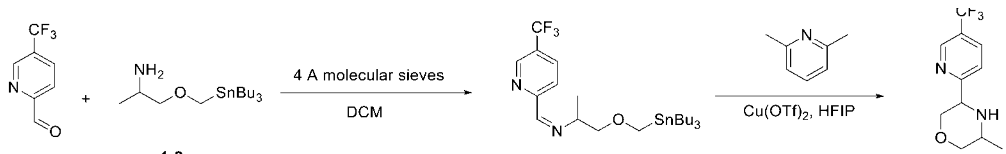


c1-1 c1-2 c1-3 c1 (cis)

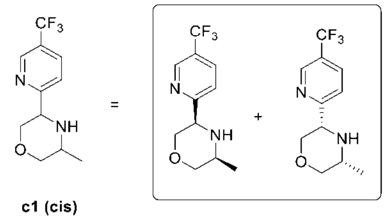

c1 (cis)

Step 1: Under nitrogen protection, the starting material 5-trifluoromethyl-pyridine-2-carbaldehyde **c1-1** (558 mg, 3.53 mmol) and the starting material **c1-2** (508 mg, 1.34 mmol) were dissolved in 5.0 mL of dichloromethane. 4A molecular sieves (1.0 g) were added, and the reaction was carried out at room temperature for 12 hours before it was stopped. The reaction mixture was filtered, and the filtrate was concentrated under reduced pressure to obtain a yellow oily substance c1-3 (600 mg). Yield: 84%. LCMS ESI-MS m/z: 537 $[M+H]^+$.

Step 2: Under nitrogen protection, 2,6-dimethylpyridine (48 mg, 0.45 mmol) and catalyst $Cu(OTf)_2$ (162 mg, 0.45 mmol) were dissolved in 3 mL of hexafluoroisopropanol and allowed to react at room temperature for 7 hours. The intermediate **c1-3** (600 mg, 1.12 mmol) was added dropwise to the reaction solution, and after completion of the addition, the reaction was continued for another 10 hours before it was stopped. 6 mL of aqueous ammonia was added to the reaction solution, and the mixture was stirred for 1 hour. The organic phase was extracted with dichloromethane, dried over anhydrous sodium sulfate, and concentrated. The crude product was purified by flash column chromatography (PE/EA, 1/1) to obtain the intermediate cis-isomer **c1** (140 mg), with a yield of: 51%. LCMS ESI-MS m/z: 247 $[M+H]^+$.

By referring to the synthetic route of the compound **c1**, similar scaffold structures were used to synthesize the following target molecules. Cis = Cis-enantiomer

| Target Intermediate | Intermediate Structure | LC-MS $[M+H]^+$ |
|---|---|---|
| **c2** | 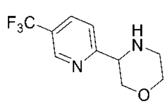 (Corresponding isomer) | 233 |

(continued)

| Target Intermediate | Intermediate Structure | LC-MS [M+H]+ |
|---|---|---|
| **c3 (cis)** | 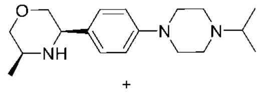<br>+<br>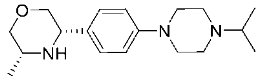 | 304 |
| **c4 (cis)** | 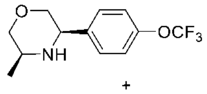<br>+<br>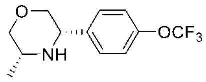 | 262 |
| **c5 (cis)** | 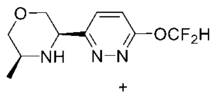<br>+<br>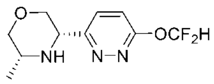 | 246 |
| **c6 (cis)** | 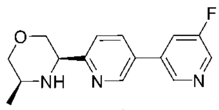<br>+<br>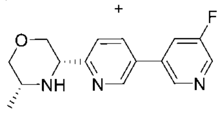 | 274 |
| **c7 (cis)** | 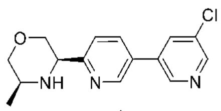<br>+<br>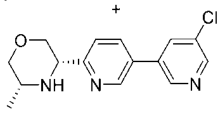 | 290 |
| **c8 (cis)** | 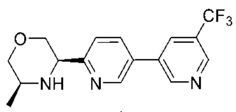<br>+<br>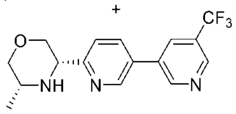 | 324 |
| **c9 (cis)** | 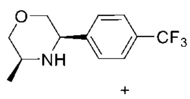<br>+<br>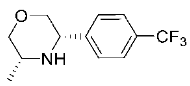 | 246 |

**Preparation of Key Intermediates d1-d7**

Synthesis of Intermediates **d1-d3**

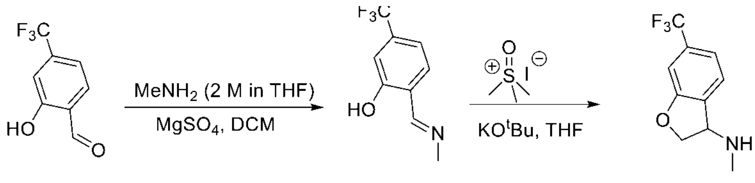


d1-1 d1-2 d1

Step 1: Under nitrogen protection, the starting material 2-hydroxy-4-trifluoromethylbenzaldehyde **d1-1** (1.0 g, 5.26 mmol) and a tetrahydrofuran solution of methylamine (5.3 mL, 2M) were dissolved in 20 mL of anhydrous dichloromethane. Magnesium sulfate (2.5 g, 21.0 mmol) was added, and the reaction was carried out at room temperature for 12 hours before it was stopped. The reaction mixture was filtered, and the filtrate was concentrated under reduced pressure to obtain a yellow solid **d1-2** (470 mg), with a yield of: 44%. LCMS ESI-MS m/z: 204 $[M+H]^+$.

Step 2: Under nitrogen protection, trimethyl sulfoxonium iodide (1.27 g, 5.78 mmol) and potassium tert-butoxide (649 mg, 5.78 mmol) were dissolved in 13 mL of anhydrous tetrahydrofuran and stirred for 30 minutes. The intermediate **d1-2** (470 mg, 2.31 mmol) was added to the reaction solution, and the mixture was heated to 50°C to react for 4 hours, followed by cooling to room temperature. Additional potassium tert-butoxide (260 mg, 2.31 mmol) was added to the reaction solution, and the reaction was continued at room temperature for 12 hours before it was stopped, and filtering was performed. The solvent was removed under reduced pressure, and the crude product was purified by flash reverse-phase column chromatography (acetonitrile/water, 1/1) to obtain white solid **d1** (40 mg), with a yield of: 8%. LCMS ESI-MS m/z: 218 $[M+H]^+$.

By referring to the synthetic route of the intermediate **d1,** the following intermediates were synthesized.

| Target Intermediate | Intermediate Structure | LC-MS $[M+H]^+$ |
|---|---|---|
| **d2** | 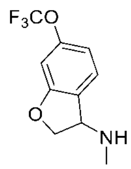 | 234 |
| **d3** | 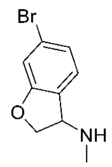 | 228 |

Synthesis of Intermediate **d4:**

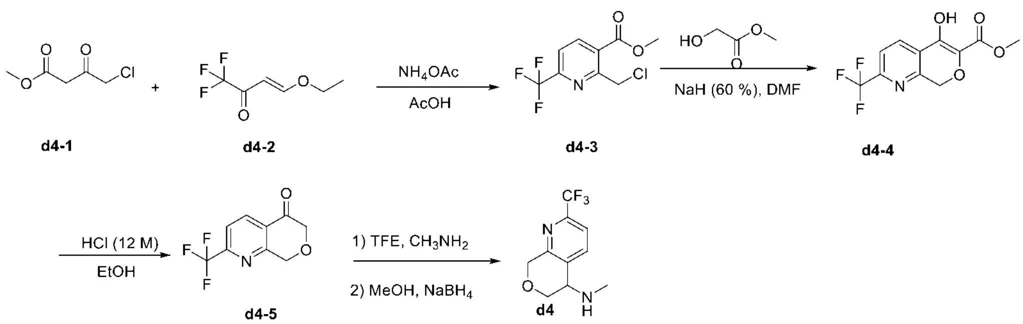


d4-1 d4-2 d4-3 d4-4 d4-5 d4

Step 1: Under nitrogen protection, the starting material **d4-1** (12.2 g, 81.0 mmol) and the starting material **d4-2** (13.6 g, 81.0

mmol) were dissolved in 20 mL of acetic acid. Ammonium acetate (12.5 g, 162 mmol) was added, and the reaction mixture was heated to 120°C to react for 1 hour, followed by cooling to room temperature. 100 mL of ice water was added to the reaction mixture, followed by extraction with methyl tert-butyl ether (MTBE) three times. The organic phases were combined, dried over anhydrous sodium sulfate, and concentrated. The crude product was purified by flash column chromatography (PE/EA, 20/1) to obtain a white solid **d4-3** (10.5 g), with a yield of: 51%. LCMS ESI-MS m/z: 254 $[M+H]^+$.

Step 2: Under nitrogen protection, the previous step's intermediate **d4-3** (10.5 g, 41.4 mmol) and methyl glycolate (2-hydroxyacetic acid methyl ester) (7.5 g, 82.8 mmol) were dissolved in 105 mL of DMF. NaH (3.3 g, 82.8 mmol, 60%) was added, and the reaction was carried out at room temperature for 2 hours. 300 mL of ice water was added to the reaction mixture, followed by extraction with ethyl acetate three times. The organic phases were combined, dried over anhydrous sodium sulfate, and concentrated to obtain a yellow oily substance **d4-4** (5.0 g), with a yield of: 44%. LCMS ESI-MS m/z: 276 $[M+H]^+$.

Step 3: The previous step's intermediate **d4-4** (5.0 g, 18.2 mmol) was dissolved in 50 mL of ethanol, and concentrated hydrochloric acid (100 mL, 12M) was added. The reaction mixture was heated to 100°C to react for 1 hour, followed by cooling to room temperature. 300 mL of aqueous ammonia was added dropwise to the reaction mixture, followed by extraction with ethyl acetate three times. The organic phases were combined, dried over anhydrous sodium sulfate, and concentrated. The crude product was purified by flash column chromatography (PE/EA, 10/1) to obtain a yellow solid **d4-5** (366 mg), with a yield of: 9%. LCMS ESI-MS m/z: 218 $[M+H]^+$.

Step 4: Under nitrogen protection, the previous step's intermediate **d4-5** (110 mg, 0.51 mmol) and a tetrahydrofuran solution of methylamine (1.3 mL, 2M) were dissolved in 1 mL of trifluoroethanol and stirred at room temperature for 16 hours. Sodium borohydride ($NaBH_4$) (96 mg, 2.5 mmol) and 0.25 mL of methanol were added to the reaction mixture, and the reaction was continued at room temperature for 1 hour before it was stopped. The reaction mixture was filtered, and the filtrate was concentrated under reduced pressure. The crude product was purified by flash reverse-phase column chromatography (acetonitrile/water, 7/10) to obtain a white solid d4 (60 mg), with a yield of: 51%. LCMS ESI-MS m/z: 233 $[M+H]^+$.

Synthesis of Intermediate d5:

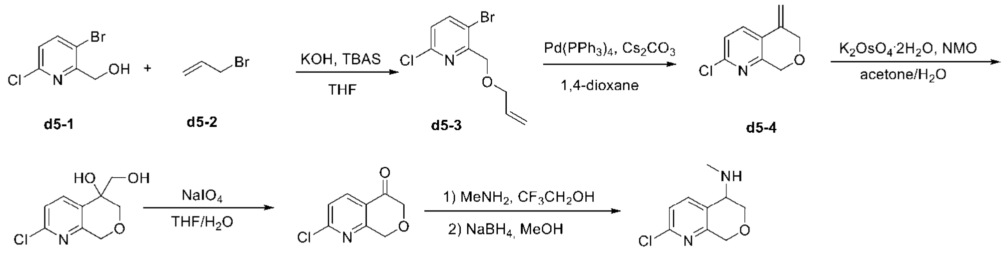


d5-1 d5-2 d5-3 d5-4

d5-5 d5-6 d5

Step 1: Under nitrogen protection, the starting material 3-bromo-6-chloropyridine-2-methanol **d5-1** (6.0 g, 27.0 mmol) and allyl bromide **d5-2** (4.9 g, 40.5 mmol) were dissolved in 120 mL of tetrahydrofuran. KOH (3.0 g, 53.9 mmol) and TBAS (1.4 g, 4.1 mmol) were added, and the reaction was carried out at room temperature for 12 hours before it was stopped. 100 mL of ice water was added to the reaction mixture, followed by extraction with ethyl acetate. The organic phases were combined, dried over anhydrous sodium sulfate, and concentrated. The crude product was purified by flash column chromatography (PE/EA, 80/1) to obtain a colorless oily substance **d5-3** (5.0 g), with a yield of: 71%. LCMS ESI-MS m/z: 262 $[M+H]^+$.

Step 2: Under nitrogen protection, the previous step's intermediate **d5-3** (5.0 g, 19.1 mmol) and cesium carbonate (7.5 g, 22.9 mmol) were dissolved in 105 mL of 1,4-dioxane. Catalyst $Pd(PPh_3)_4$ (4.4 g, 3.8 mmol) was added, and the reaction mixture was heated to 100°C to react for 12 hours, followed by cooling to room temperature and filtering. 100 mL of ice water was added to the filtrate, followed by extraction with ethyl acetate three times. The organic phases were combined, dried over anhydrous sodium sulfate, and concentrated. The crude product was purified by flash column chromatography (PE/EA, 10/1) to obtain a light yellow solid **d5-4** (1.3 g), with a yield of: 38%. LCMS ESI-MS m/z: 182 $[M+H]^+$.

Step 3: The previous step's intermediate **d5-4** (1.3 g, 7.2 mmol) was dissolved in 30 mL of a mixed solution of acetone and water (v/v, 5/1). $K_2OsO_4 \cdot 2H_2O$ (264 mg, 0.72 mmol) and NMO (2.9 g, 25.1 mmol) were added, and the reaction was carried out at room temperature for 2 hours. The reaction was quenched by adding a saturated aqueous solution of sodium bisulfite, followed by extraction with ethyl acetate three times. The organic phases were combined, dried over anhydrous sodium sulfate, and concentrated to obtain an oily product **d5-5** (1.3 g), with a yield of: 84%. LCMS ESI-MS

m/z: 216 [M+H]+.

Step 4: Under nitrogen protection, the previous step's intermediate **d5-5** (1.3 g, 6.0 mmol) was dissolved in 25 mL of a mixed solution of tetrahydrofuran and water (v/v, 5/1). Sodium periodate ($NaIO_4$) (3.2 g, 15.1 mmol) was added, and the reaction mixture was stirred at room temperature for 1 hour before stopping the reaction. 50 mL of water was added to the reaction mixture, followed by extraction with ethyl acetate. The organic phase was dried over anhydrous sodium sulfate and concentrated. The crude product was purified by flash reverse-phase column chromatography (acetonitrile/water, 3/8) to obtain a white solid **d5-6** (900 mg), with a yield of: 51%. LCMS ESI-MS m/z: 184 [M+H]+.

Step 5: Under nitrogen protection, the previous step's intermediate **d5-6** (900 mg, 4.9 mmol) and an aqueous solution of methylamine (24.5 mmol, 2.5 mL) were dissolved in 6 mL of trifluoroethanol and stirred at room temperature for 4 hours. Methanol (3.6 mL) and reducing agent $NaBH_4$ (927 mg, 24.5 mmol) were added to the reaction solution, and the reaction was carried out in an ice bath for 1 hour. The reaction was quenched by adding a saturated aqueous solution of sodium bicarbonate. The mixture was extracted with ethyl acetate, dried over anhydrous sodium sulfate, and concentrated. The crude product was purified by flash reverse-phase column chromatography (acetonitrile/water, 7/10) to obtain a white solid **d5** (500 mg), with a yield of: 51%. LCMS ESI-MS m/z: 199 [M+H]+.

Synthesis of Intermediates **d6-d8:**

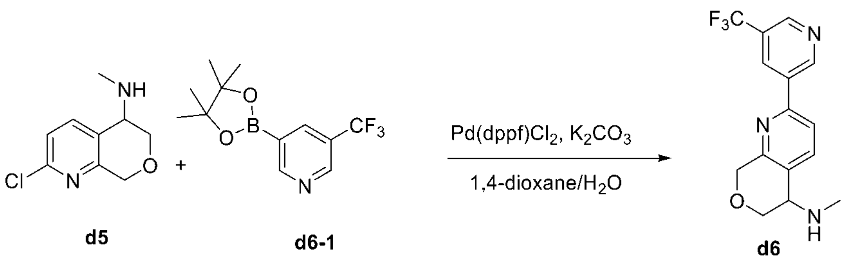


d5 d6-1 d6

Step: Under nitrogen protection, the intermediate **d5** (80 mg, 0.4 mmol), the starting material **d6-1** (218 mg, 0.8 mmol), and potassium carbonate (112 mg, 0.8 mmol) were dissolved in 2 mL of a mixed solution of 1,4-dioxane and water (v/v, 4/1). Catalyst Pd(dppf)$Cl_2$ (30 mg, 0.04 mmol) was added, and the mixture was heated to 80°C to react for 1 hour, followed by cooling to room temperature and filtering. 20 mL of ice water was added to the filtrate, followed by extraction with ethyl acetate three times. The organic phases were combined, dried over anhydrous sodium sulfate, and concentrated. The crude product was purified by flash reverse-phase column chromatography (acetonitrile/water, 3/5) to obtain a light yellow solid **d6** (80 mg), with a yield of: 51%. LCMS ESI-MS m/z: 310 [M+H]+.

By referring to the synthetic route of the intermediate **d6,** the following intermediate were synthesized.

| Target Intermediate | Intermediate Structure | LC-MS [M+H]+ |
|---|---|---|
| **d7** | 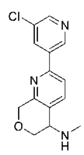 | 276 |
| **d8** | 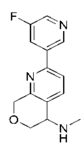 | 260 |

**Preparation of Key Intermediates e1-e6**

Synthesis of Intermediates **e1-e3**

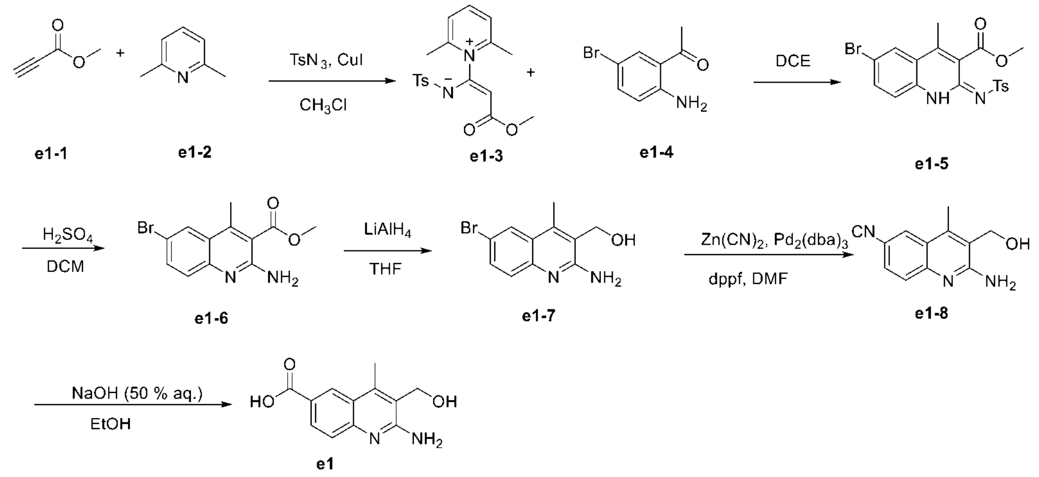


Step 1: In an ice bath under nitrogen protection, the starting material methyl propiolate e1-1 (2.1 g, 24.9 mmol) and the starting material 2,6-dimethylpyridine e1-2 (2.7 g, 24.9 mmol) were dissolved in 42 mL of chloromethane. $TsN_3$ (4.1 g, 20.8 mmol) and catalyst CuI (400 mg, 2.08 mmol) were added slowly, and the reaction was carried out in the ice bath for 4 hours before it was stopped. The solvent was removed under reduced pressure, and the residue was dissolved in ethyl acetate. The solid was precipitated, filtered under vacuum, and dried to obtain a white solid e1-3 (2.5 g). Yield: 33%. LC-MS: $[M+H]^+$ = 361.

Step 2: Under nitrogen protection, the previous step's intermediate e1-3 (2.5 g, 6.94 mmol) and 2-amino-4-bromoacetophenone e1-4 (1.5 g, 6.94 mmol) were dissolved in 50 mL of dichloroethane. The reaction mixture was heated to 90°C to react for 4 hours, and then the reaction was stopped. The solvent was removed under reduced pressure to obtain a crude product **e1-5** (3.0 g). LC-MS: $[M+H]^+$ = 450.

Step 3: In an ice bath, the previous step's crude product **e1-5** (3.0 g, 6.68 mmol) was dissolved in 63 mL of dichloromethane. Concentrated sulfuric acid (1.3 g, 13.4 mmol) was added dropwise, and after completion of the addition, the reaction was carried out at room temperature for 1 hour before it was stopped. The reaction mixture was slowly poured into ice water, and the pH was adjusted to approximately 9 with a saturated sodium bicarbonate aqueous solution. The mixture was extracted with dichloromethane, dried over anhydrous sodium sulfate, and concentrated. The crude product was purified by flash reverse-phase column chromatography (acetonitrile/water, 4/5) to obtain a yellow solid **e1-6** (1.5 g), with a yield of: 76%. LC-MS: $[M+H]^+$ = 297.

Step 4: In an ice bath under nitrogen protection, the previous step's intermediate **e1-6** (3.0 mL, 2.5M) was dissolved in 30 mL of anhydrous tetrahydrofuran. A tetrahydrofuran solution of $LiAlH_4$ (1.3 g, 13.4 mmol) was added slowly, and after completion of the addition, the reaction was carried out at room temperature for 1 hour, and then it was stopped. The reaction mixture was slowly poured into ice water, filtered, and the filtrate was concentrated under reduced pressure to obtain a yellow oily product **e1-7** (1.0 g), with a yield of: 74%. LC-MS: $[M+H]^+$ = 268.

Step 5: Under nitrogen protection, the previous step's intermediate **e1-7** (1.0 g, 3.74 mmol) and zinc cyanide (500 mg, 4.49 mmol) were dissolved in 20 mL of anhydrous DMF. Ligand dppf (200 mg, 0.37 mmol) and catalyst $Pd_2(dba)_3$ (200 mg, 0.19 mmol) were added, and the reaction mixture was heated to 100°C to react for 2 hours before it was stopped. 100 mL of ice water was added to the reaction mixture, followed by extraction with ethyl acetate. The organic phase was dried over anhydrous sodium sulfate and concentrated. The crude product was purified by flash reverse-phase column chromatography (acetonitrile/10M ammonium bicarbonate aqueous solution, 4/5) to obtain a yellow oily product **e1-8** (400 mg), with a yield of: 50%. LC-MS: $[M+H]^+$ = 214.

Step 6: The previous step's intermediate **e1-8** (400 mg, 1.88 mmol) was dissolved in 8 mL of ethanol, and an aqueous NaOH solution (8.0 mL, 50%) was added slowly. The reaction mixture was heated to 80°C to react for 4 hours before the reaction was stopped. The reaction mixture was slowly poured into ice water, and the pH was adjusted to approximately 3 with dilute hydrochloric acid. The mixture was extracted with ethyl acetate, and the crude product was purified by flash reverse-phase column chromatography (acetonitrile/water, 3/5) to obtain a gray solid **e1** (300 mg), with a yield of: 69%. LC-MS: $[M+H]^+$ = 233.

By referring to the synthetic route of the compound **e1,** the following target molecular intermediates were synthesized using similar starting materials/intermediates.

| Target Intermediate | Intermediate Structure | LC-MS [M+H]$^+$ |
|---|---|---|
| **e2** | 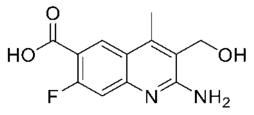 | 251 |
| **e3** | 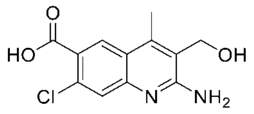 | 267 |

Synthesis of Intermediates e4-e6

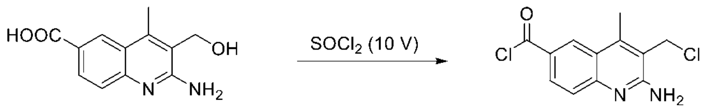


e1 e4

Step: Under nitrogen protection, the intermediate e1 (320 mg, 1.37 mmol) was dissolved in 3.2 mL of thionyl chloride, and the reaction mixture was heated to 70°C to react for 1 hour before the reaction was stopped. The solvent was removed under reduced pressure to obtain a yellow solid e4 (320 mg), with a yield of: 86%. The product was directly used for the next reaction.

By referring to the synthetic route of compound d4, the following target molecular intermediates were synthesized using similar starting materials/intermediates.

| Target Intermediate | Intermediate Structure |
|---|---|
| **e5** | 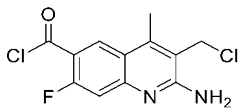 |
| **e6** | 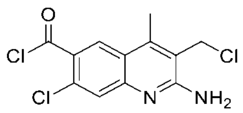 |

**Preparation of Key Intermediates f1-f2**

Synthesis of Intermediates **f1-f2**

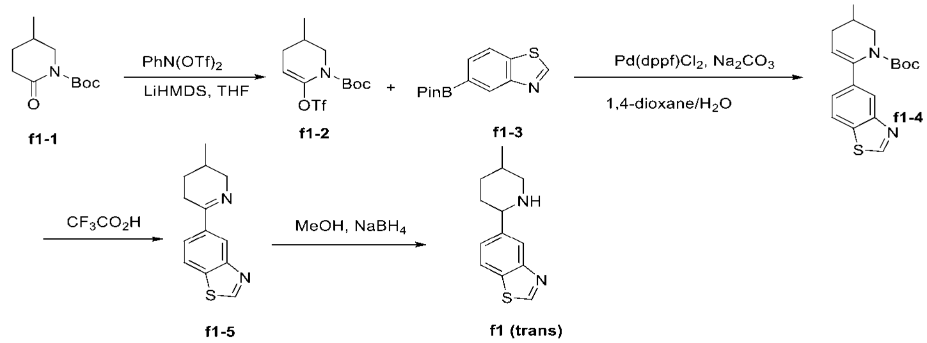


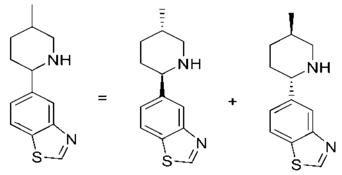

f1 (trans)

Step 1: At -78°C under nitrogen protection, the starting material **f1-1** (8.8 g, 41.3 mmol) was dissolved in 88 mL of anhydrous tetrahydrofuran, and a tetrahydrofuran solution of LiHMDS (10.4 g, 61.9 mmol, 61.9 mL) was slowly added dropwise. After the addition was complete, the reaction mixture was stirred at -78°C for 1.5 hours. N-Phenyl-bis(trifluoromethanesulfonyl)imide (20.1 g, 51.6 mmol) was added to the reaction solution, and the mixture was heated to room temperature to react for 2 hours before the reaction was stopped. 50 mL of ice water was added to the reaction solution, followed by extraction with methyl tert-butyl ether. The organic phase was dried over anhydrous sodium sulfate and concentrated. The crude product was purified by flash reverse-phase column chromatography (acetonitrile) to obtain a red oily substance **f1-2** (10.1 g), with a yield of: 71%. LCMS ESI-MS m/z: 346 $[M+H]^+$.

Step 2: Under nitrogen protection, the previous step's oily product **f1-2** (2.1 g, 6.08 mmol), 5-boronic acid pinacol ester-1,3-benzothiazole **f1-3** (1.1 g, 7.62 mmol), and sodium carbonate (1.9 g, 18.2 mmol) were dissolved in 40 mL of a mixed solution of 1,4-dioxane and water (v/v, 3/1). Catalyst $Pd(dppf)Cl_2$ (220 mg, 0.30 mmol) was added, and the mixture was heated to 80°C to react for 2 hours, and then it was stopped. The reaction mixture was filtered, and 100 mL of water was added. The organic phase was extracted with ethyl acetate, dried over anhydrous sodium sulfate, and concentrated to obtain a white solid **f1-4** (2.7 g), with a yield of: 96%. LCMS ESI-MS m/z: 331 $[M+H]^+$.

Step 3: The previous step's white solid **f1-4** (2.7 g, 8.17 mmol) was dissolved in 15 mL of trifluoroacetic acid to react at room temperature for 1 hour, and then the reaction was stopped. The solvent was removed under reduced pressure, and the reaction mixture was adjusted to approximately pH 8 with a saturated aqueous solution of sodium bicarbonate. The mixture was extracted with ethyl acetate, dried over anhydrous sodium sulfate, and concentrated to obtain a yellow oily product **f1-5** (1.8 g), with a yield of: 96%. LCMS ESI-MS m/z: 231 $[M+H]^+$.

Step 4: In an ice bath, the previous step's oily product **f1-5** (1.0 g, 4.34 mmol) was dissolved in 20 mL of methanol. Reducing agent $NaBH_4$ (250 mg, 6.51 mmol) was added, and the reaction was carried out in the ice bath for 1 hour, and then it was stopped. 100 mL of ice water was added to the reaction mixture, followed by extraction with ethyl acetate. The organic phase was dried over anhydrous sodium sulfate and concentrated. The crude product was purified by flash reverse-phase column chromatography (acetonitrile/water, 3/5) to obtain a yellow oily product **f1** (370 mg), with a yield of: 37%. LCMS ESI-MS m/z: 233 $[M+H]^+$.

By referring to the synthetic route of the compound **f1,** the following target intermediate was synthesized using similar starting materials/intermediates. Trans = Trans-enantiomer.

| Target Intermediate | Intermediate Structure | LC-MS $[M+H]^+$ |
|---|---|---|
| **f2 (trans)** | 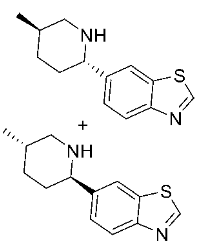 | 233 |

**Chiral Resolution of** Key **Intermediates f3-f4**

Synthesis of Intermediates f3-f4

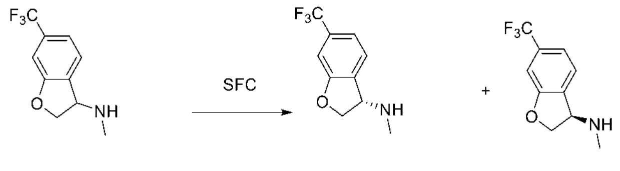


d1 f3 (*S*) f4 (*R*)

Resolution conditions: column: CHIRALPAK IF, 5×25 cm, 5 μm; mobile phase A: Hex (0.5% 2M $NH_3$ in methanol solution); mobile phase B (EtOH); flow rate: 20 mL/min;);

f1 (retention time: 4.807 min);

f2 (retention time: 5.943 min).

**Example 2:** Synthesis of **the Target Molecules P1-P12**

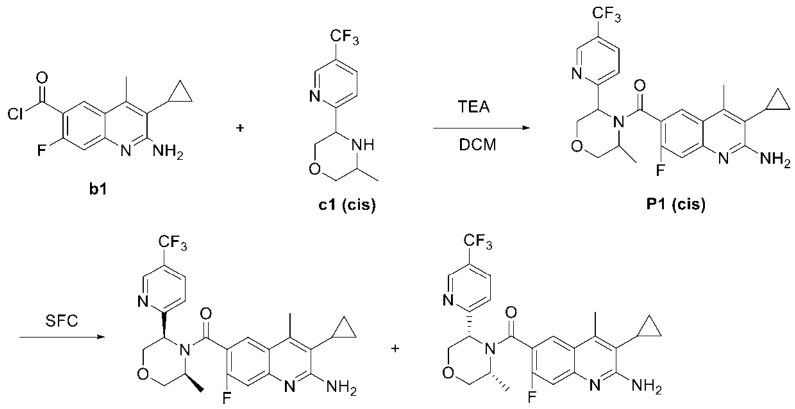


b1 c1 (cis) P1 (cis)

P1a P1b

Step 1: At room temperature, the intermediate **c1** (71 mg, 0.29 mmol) and triethylamine (145 mg, 1.44 mmol) were dissolved in 2 mL of dichloromethane. A dichloromethane solution of the intermediate **b1** (81 mg, 0.29 mmol, 0.5 mL) was added dropwise, and the mixture was stirred at room temperature to react for 1 hour. The solvent was removed under reduced pressure, and the crude product was purified by preparative HPLC (mobile phase: acetonitrile/water, 4/5) to obtain the compound **P1** (40 mg), with a yield of: 29%. LCMS ESI-MS m/z: 489 $[M+H]^+$.

Step 2: The compound **P1** was separated by SFC chiral column chromatography to obtain compounds **P1a** and **P1b.**

Resolution conditions: (column: CHIRAL ART Cellulose-SC 2×25 cm, 5 μm; mobile phase A: Hex: DCM=3: 1 (0.5% 2M $NH_3$ in methanol); mobile phase B: IPA; flow rate: 20 mL/min).

Retention time: 17.604 min **(P1a);** 20.403 min **(P1b).**

**P1a:** $^1$H NMR (400 MHz, DMSO-$d_6$) δ 9.11 - 8.96 (m, 1H), 8.25 (s, 1H), 7.97 (d, *J* = 7.5 Hz, 1H), 7.79 (s, 1H), 7.24 (s, 1H), 6.72 (s, 2H), 5.73 (s, 1H), 5.12 (s, 1H), 3.88 - 3.49 (m, 4H), 2.78 - 2.54 (m, 3H), 1.63 (s, 1H), 1.20 (d, *J= 31.9* Hz, 3H), 0.78 (s, 2H), 0.48 (s, 2H).

**P1b:** $^1$H NMR (400 MHz, DMSO-$d_6$) δ 9.11 - 8.96 (m, 1H), 8.25 (s, 1H), 7.97 (d, *J*= 7.5 Hz, 1H), 7.79 (s, 1H), 7.24 (s, 1H), 6.72 (s, 2H), 5.73 (s, 1H), 5.12 (s, 1H), 3.88 - 3.49 (m, 4H), 2.78 - 2.54 (m, 3H), 1.63 (s, 1H), 1.20 (d, *J= 31.9* Hz, 3H), 0.78 (s, 2H), 0.48 (s, 2H).

By referring to the synthetic route of the compound **P1,** the following target molecules were synthesized using similar starting materials or intermediates.

Cis indicates that the compound has a cis configuration and is not resolved;
* Indicates a chiral center and that it is not resolved.

| Compound | Target molecular structure | Resolution conditions: /$^1$H NMR/ | LC-MS [M+H]$^+$ |
|---|---|---|---|
| **P2 (cis)** | 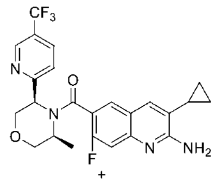 + 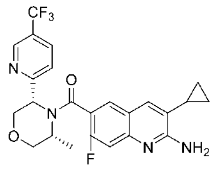 | $^1$H NMR: (400 MHz, DMSO-$d_6$) $\delta$ 8.96 - 8.90 (m, 1H), 8.17 (dd, $J$ = 8.4, 2.4 Hz, 1H), 7.77 (d, $J$ = 7.7 Hz, 1H), 7.73 - 7.67 (m, 1H), 7.63 (s, 1H), 7.18 (d, $J$ = 11.7 Hz, 1H), 6.53 (s, 2H), 5.98 (s, 1H), 4.98 (d, $J$ = 12.0 Hz, 1H), 3.92 (s, 1H), 3.76 (dd, $J$ = 12.0, 4.3 Hz, 1H), 3.67 - 3.52 (m, 2H), 1.83 - 1.74 (m, 1H), 0.99 - 0.90 (m, 2H), 0.76 (d, $J$ = 7.0 Hz, 3H), 0.64 - 0.57 (m, 2H). | 475 |
| **P3** | 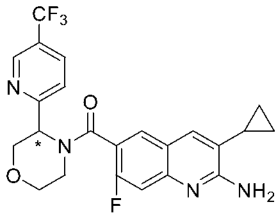 | $^1$H NMR (400 MHz, DMSO-$d_6$) $\delta$ 8.94 (s, 1H), 8.18 (d, $J$ = 8.3 Hz, 1H), 7.74 - 7.57 (m, 3H), 7.16 (d, $J$ = 11.8 Hz, 1H), 6.48 (s, 2H), 4.62 (s, 1H), 3.86 (dd, $J$ = 11.9, 3.8 Hz, 1H), 3.75 (s, 1H), 3.51 (t, $J$ = 11.5 Hz, 4H), 1.79 (dq, $J$ = 13.9, 7.0, 6.2 Hz, 1H), 0.95 (dt, $J$ = 8.6, 2.6 Hz, 2H), 0.62 (d, $J$ = 4.9 Hz, 2H). | 461 |
| **P4 (cis)** | 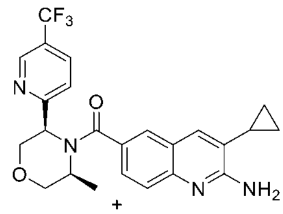 + 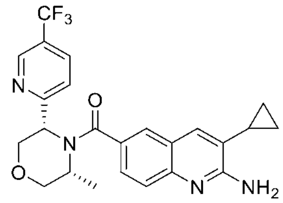 | $^1$H NMR: (400 MHz, DMSO-$d_6$) $\delta$ 8.99 (d, $J$ = 2.3 Hz, 1H), 8.21 (dd, $J$ = 8.5, 2.4 Hz, 1H), 7.85 - 7.66 (m, 3H), 7.58 (dd, $J$ = 8.5, 2.0 Hz, 1H), 7.49 (d, $J$ = 8.5 Hz, 1H), 6.63 (s, 2H), 5.51 (s, 1H), 5.02 (d, $J$ = 11.9 Hz, 1H), 4.08 (s, 1H), 3.83 (dd, $J$ = 12.0, 4.1 Hz, 1H), 3.69 (dd, $J$ = 11.4, 3.4 Hz, 1H), 3.57 (d, $J$ = 11.4 Hz, 1H), 1.83 (t, $J$ = 5.2 Hz, 1H), 0.97 (dt, $J$ = 8.8, 3.0 Hz, 2H), 0.79 (d, $J$ = 6.9 Hz, 3H), 0.66 (td, $J$ = 5.8, 3.8 Hz, 2H). | 457 |
| **P5 (cis)** | 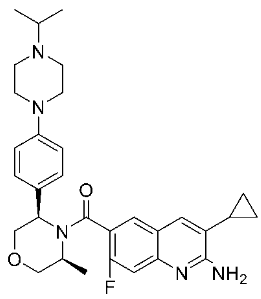 + 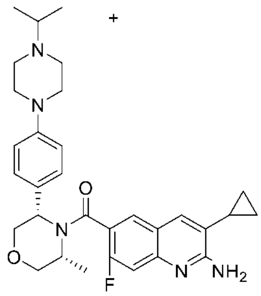 | $^1$H NMR: (300 MHz, DMSO-$d_6$) $\delta$ 7.72 (d, $J$ = 7.3 Hz, 2H), 7.38 (s, 2H), 7.21 (d, $J$ = 11.7 Hz, 1H), 6.91 (d, $J$ = 8.3 Hz, 2H), 6.75 (s, 2H), 4.64 (d, $J$ = 12.4 Hz, 1H), 3.96 - 3.54 (m, 4H), 3.13 (s, 4H), 2.61 (s, 5H), 1.80 (p, $J$ = 7.9 Hz, 1H), 1.32 - 0.91 (m, 9H), 0.77 (d, $J$ = 6.8 Hz, 3H), 0.72 - 0.53 (m, 2H). | 532 |

(continued)

| Compound | Target molecular structure | Resolution conditions: /$^1$H NMR/ | LC-MS $[M+H]^+$ |
|---|---|---|---|
| **P6 (cis)** | 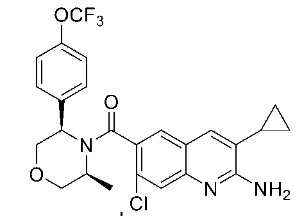 + 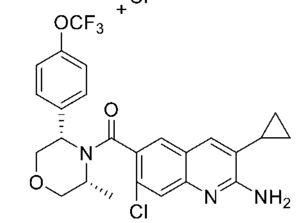 | $^1$H NMR (400 MHz, DMSO-$d_6$) $\delta$ 7.86 - 7.62 (m, 4H), 7.53 (d, *J* = 4.6 Hz, 1H), 7.40 (d, *J* = 8.3 Hz, 2H), 6.81 (d, *J* = 9.5 Hz, 2H), 5.75 (d, *J* = 3.6 Hz, 1H), 4.73 (t, *J* = 11. 7 Hz, 1H), 3.88 - 3.46 (m, 4H), 1.81 (tq, *J* = 12.7, 7.3, 6.1 Hz, 1H), 0.97 (ddt, *J* = 8.4, 3.9, 2.6 Hz, 2H), 0.82 - 0.38 (m, 5H). | 506 |
| **P7a** | 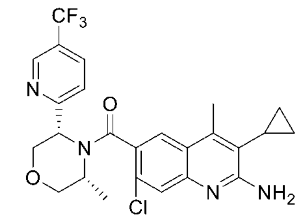 | Resolution conditions: column: WelFlash CHIRAL ART Amylose-C NEO, 2×25 cm, 5 μm; mobile phase A: Hex (0.5 % 2M $NH_3$-MeOH), mobile phase B: EtOH; flow rate: 20 mL/min; retention time: 9.71 min; $^1$H NMR (400 MHz, DMSO-$d_6$) $\delta$ 9.03 (d, *J*= *6.2* Hz, 1H), 8.37 - 8.11 (m, 1H), 8.03 - 7.85 (m, 2H), 7.62 - 7.43 (m, 1H), 6.87 (s, 2H), 5.72 (d, *J*= 4.2 Hz 1H), 5.14 (d, *J* = 11.7 Hz, 1H), 4.63 (dd, *J* = 73.8, 54.6 Hz, 1H), 3.93 - 3.63 (m, 3H), 3.63 - 3.41 (m, 1H), 2.70 (d, *J* = 11.1 Hz, 2H), 1.66 (s, 1H), 1.17 (s, 2H), 0.90 (s, 1H), 0.76 (d, *J* = 7.0 Hz, 2H), 0.52 (s, 2H). | 505 |
| **P7b** | 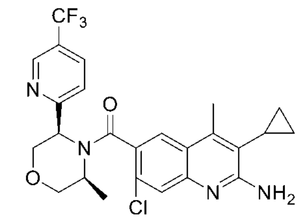 | Resolution conditions: same as above; retention time: 13.36 min; $^1$H NMR (400 MHz, DMSO-$d_6$) $\delta$ 9.03 - 8.93 (m, 1H), 8.38 - 8.08 (m, 1H), 7.97 - 7.84 (m, 2H), 7.54 (d, *J* = 6.8 Hz, 1H), 6.57 (s, 2H), 5.72 (s, 1H), 5.17 (dd, *J* = 17.8, 11.5 Hz, 1H), 4.88 - 4.34 (m, 1H), 4.02 - 3.37 (m, 4H), 2.68 (d, *J* = 11.8 Hz, 2H), 1.67 - 1.61 (m, 1H), 1.33 - 1.03 (m, 2H), 0.83 (dd, *J* = 17.8, 11.5 Hz, 3H), 0.59 - 0.27 (m, 2H). | 505 |

(continued)

| Compound | Target molecular structure | Resolution conditions: /$^1$H NMR/ | LC-MS $[M+H]^+$ |
|---|---|---|---|
| **P8 (cis)** | 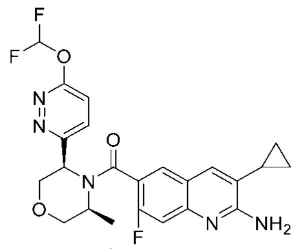 + 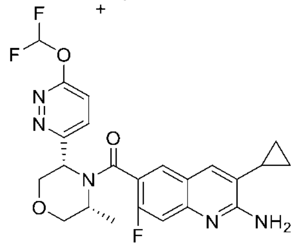 | $^1$H NMR (400 MHz, DMSO-$d_6$) $\delta$ 8.19 - 7.81 (m, 3H), 7.77 (s, 1H), 7.59 (s, 1H), 7.24 (d, $J$ = 11.8 Hz, 1H), 6.80 (s, 2H), 5.80 (s, 1H), 5.06 (d, $J$ = 32.2 Hz, 1H), 3.87 - 3.49 (m, 4H), 1.81 (td, $J$ = 8.2, 4.2 Hz, 1H), 0.97 (dd, $J$ = 8.3, 2.0 Hz, 2H), 0.68 (dd, $J$ = 32.3, 6.0 Hz, 5H). | 474 |
| **P9 (cis)** | 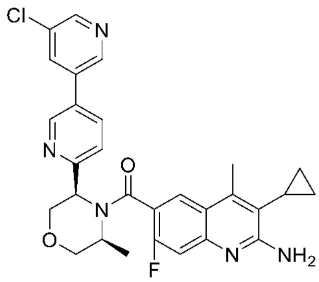 + 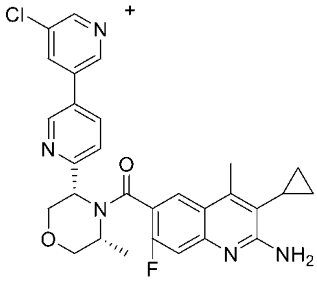 | $^1$H NMR (400 MHz, DMSO-$d_6$) $\delta$ 9.01 (d, $J$ = 24.1 Hz, 2H), 8.69 (d, $J$ = 2.3 Hz, 1H), 8.41 (s, 1H), 8.27 (s, 1H), 7.96 (s, 1H), 7.67 (s, 1H), 7.21 (s, 1H), 6.53 (s, 2H), 5.43 (d, $J$ = 203.1 Hz, 1H), 3.74 (d, $J$ = 36.6 Hz, 4H), 2.66 (d, $J$ = 11.2 Hz, 3H), 1.62 (s, 1H), 1.14 (s, 2H), 0.86 (s, 4H), 0.47 (s, 2H). | 533 |
| **P10 (cis)** | 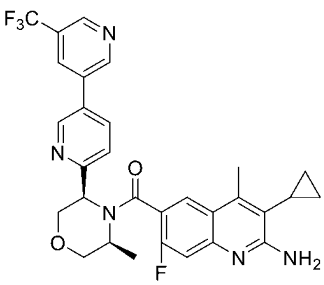 + 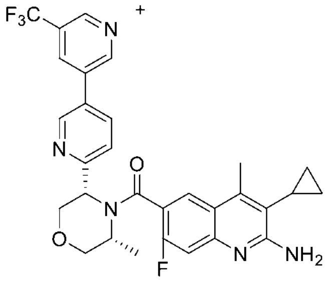 | $^1$H NMR (300 MHz, DMSO-$d_6$) $\delta$ 9.32 (s, 1H), 9.07 (d, $J$ = 18.6 Hz, 2H), 8.63 (s, 1H), 8.34 (s, 1H), 7.97 (s, 1H), 7.70 (s, 1H), 7.21 (s, 1H), 6.54 (s, 2H), 5.70 (s, 1H), 5.18 (s, 1H), 4.96 - 4.50 (m, 1H), 3.74 (d, $J$ = 28.1 Hz, 3H), 2.65 (s, 3H), 1.62 (s, 1H), 1.15 (s, 2H), 0.86 (s, 3H), 0.47 (s, 2H). | 566 |

(continued)

| Compound | Target molecular structure | Resolution conditions: /¹H NMR/ | LC-MS $[M+H]^+$ |
|---|---|---|---|
| **P11 (cis)** | 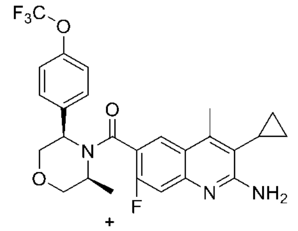 + 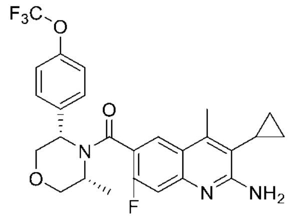 | | 504 |
| **P12 (cis)** | 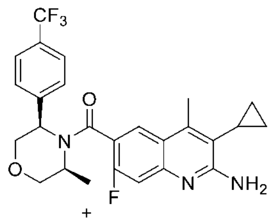 + 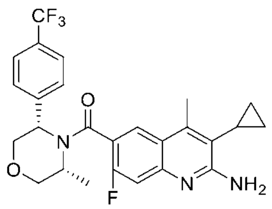 | | 488 |

**Example** 3: Synthesis of the **Target Molecules H1-H3, H6-H7**

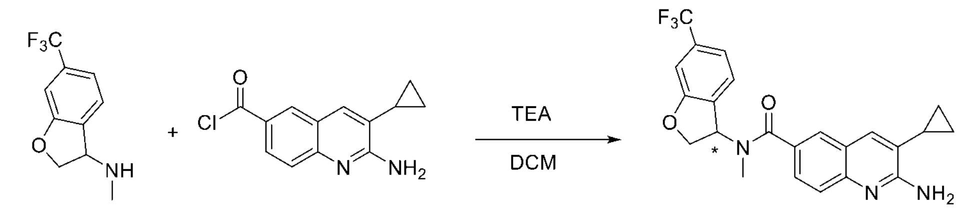


d1 b5 H1

Step 1: Under nitrogen protection, the intermediate **d1** (41 mg, 0.18 mmol) and triethylamine (93 mg, 0.92 mmol) were dissolved in 2 mL of dichloromethane. A dichloromethane solution of intermediate **b5** (41 mg, 0.18 mmol, 0.5 mL) was added dropwise, and the mixture was stirred at room temperature to react for 2 hours. The solvent was removed under reduced pressure, and the crude product was purified by preparative HPLC (mobile phase: acetonitrile/water, 4/5) to obtain the target molecule **H1** (12 mg), with a yield of: 15%. LCMS ESI-MS m/z: 428 $[M+H]^+$.

$^1H$ NMR (400 MHz, DMSO-$d_6$) $\delta$ 7.79 (s, 1H), 7.70 (s, 1H), 7.62 (d, *J*= 7.8 Hz, 1H), 7.57 - 7.46 (m, 2H), 7.33 (d, *J*= *7.8* Hz, 1H), 7.26 (s, 1H), 6.65 (s, 2H), 6.35 (s, 1H), 4.80 (s, 1H), 4.71 (dd, *J*= 10.4, 4.5 Hz, 1H), 2.65 (s, 3H), 1.89 - 1.76 (m, 1H), 1.02 - 0.90 (m, 2H), 0.67 (dt, *J* = 5.6, 2.8 Hz, 2H).

By referring to the synthetic route of compound **H1,** the following target molecules were synthesized using similar starting materials or intermediates.

* Indicates a chiral center and that it is not resolved.

| Compound | Target molecular structure | Resolution conditions: /$^1$H NMR/ | LC-MS $[M+H]^+$ |
|---|---|---|---|
| **H2** | 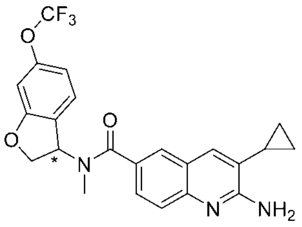 | $^1$H NMR (300 MHz, DMSO-$d_6$) $\delta$ 7.78 (d, $J$ = 1.8 Hz, 1H), 7.69 (s, 1H), 7.59 - 7.40 (m, 3H), 6.95 (s, 2H), 6.63 (s, 2H), 6.22 (s, 1H), 4.78 (s, 1H), 4.68 (dd, $J$ = 10.4, 4.3 Hz, 1H), 2.63 (s, 3H), 1.82 (td, $J$ = 8.2, 4.2 Hz, 1H), 1.02 - 0.95 (m, 2H), 0.66 (td, $J$ = 5.8, 3.9 Hz, 2H). | 444 |
| **H3** | 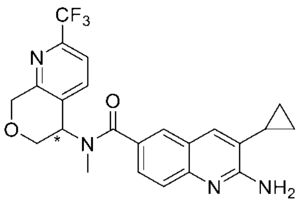 | $^1$H NMR (400 MHz, DMSO-$d_6$) $\delta$ 8.08 (s, 1H), 7.90 - 7.81 (m, 2H), 7.68 (s, 1H), 7.58 (dd, $J$ = 8.6, 2.0 Hz, 1H), 7.49 (d, $J$ = 8.6 Hz, 1H), 6.65 (s, 2H), 5.82 (s, 1H), 5.11 - 4.82 (m, 2H), 4.18 (s, 2H), 2.77 (s, 3H), 1.83 (td, $J$ = 8.2, 4.2 Hz, 1H), 0.97 (dt, $J$ = 10.3, 3.0 Hz, 2H), 0.69 - 0.62 (m, 2H). | 443 |
| **H6** | 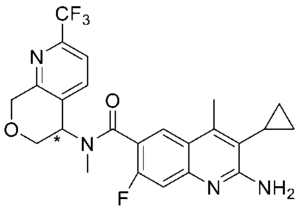 | $^1$H NMR (300 MHz, DMSO-$d_6$) $\delta$ 8.03 (d, $J$ = 8.1 Hz, 1H), 7.92 (d, $J$ = 7.9 Hz, 2H), 7.28 (s, 2H), 7.18 (dd, $J$ = 11.7, 6.4 Hz, 1H), 6.55 (s, 2H), 5.95 - 5.84 (m, 1H), 4.92 - 4.60 (m, 2H), 4.19 (d, $J$ = 5.0 Hz, 1H), 2.79 (s, 1H), 2.66 (dd, J= 8.9, 2.0 Hz, 4H), 1.63 (d, $J$= 8.0 Hz, 1H), 1.15 (dq, $J$ = 8.2, 3.8 Hz, 2H), 0.48 (dq, $J$ = 5.8, 3.8 Hz, 2H). | 475 |
| **H7** | 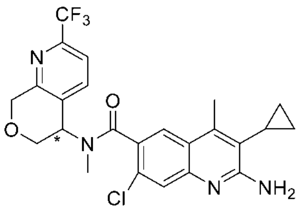 | $^1$H NMR (400 MHz, DMSO-$d_6$) $\delta$ 8.14 - 8.01 (m, 1H), 7.96 - 7.88 (m, 2H), 7.52 - 7.47 (m, 1H), 6.53 (s, 2H), 5.93 (s, 1H), 4.93 - 4.72 (m, 2H), 4.23 - 3.99 (m, 2H), 2.80 (d, $J$ = 12.5 Hz, 1H), 2.62 (d, $J$ = 24.2 Hz, 5H), 1.63 (d, $J$ = 6.3 Hz, 1H), 1.24 (s, 1H), 1.19 - 1.12 (m, 2H), 0.49 (d, $J$ = 4.9 Hz, 2H). | 491 |

**Example** 4: Synthesis of the **Target Molecules H4-H5**

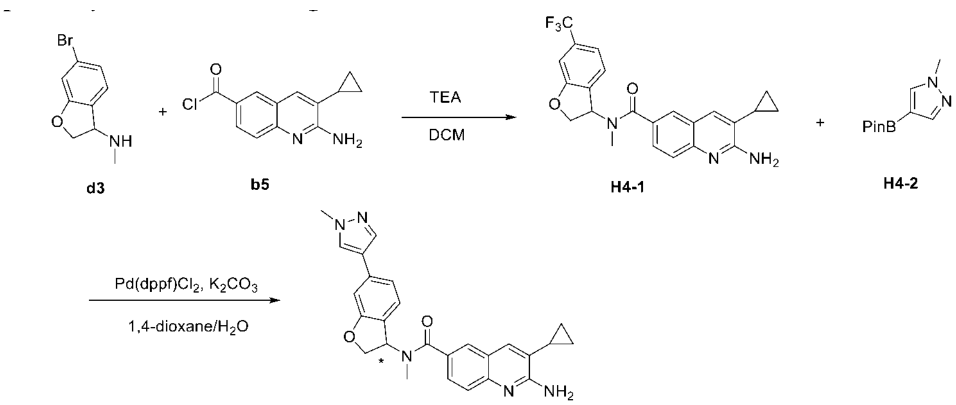


d3 b5 H4-1 H4-2

H4

Step 1: Under nitrogen protection, the intermediate **d3** (500 mg, 2.19 mmol) and triethylamine (1.11 g, 11.0 mmol) were dissolved in 12 mL of dichloromethane. A dichloromethane solution of the intermediate **b5** (541 mg, 2.19 mmol, 2 mL) was added dropwise, and the mixture was stirred at room temperature to react for 2 hours. The solvent was removed under reduced pressure, and the crude product was purified by preparative HPLC (mobile phase: acetonitrile/water, 9/10) to

obtain a white solid **H4-1** (512 mg), with a yield of: 52%. LCMS ESI-MS m/z: 438 $[M+H]^+$.

Step 2: Under nitrogen protection, the previous step's compound **H4-1** (70 mg, 0.16 mmol), 1-N-methyl-4-pyrazole boronic acid pinacol ester **H4-2** (67 mg, 0.32 mmol), and potassium carbonate (66 mg, 0.48 mmol) were dissolved in 2 mL of a mixed solution of 1,4-dioxane and water (v/v, 3/1). Catalyst $Pd(dppf)Cl_2$ (23 mg, 0.03 mmol) was added, and the mixture was stirred for 5 minutes, heated to 90°C to react for 2 hours, and then cooled to room temperature. The solvent was removed under reduced pressure, and the crude product was purified by flash reverse-phase column chromatography (acetonitrile/water, 9/10) to obtain target molecule **H4** (60 mg), with a yield of: 83%. LCMS ESI-MS m/z: 440 $[M+H]^+$.

$^1$H NMR (400 MHz, DMSO-$d_6$) $\delta$ 8.15 (s, 1H), 7.87 (s, 1H), 7.78 (s, 1H), 7.70 (s, 1H), 7.54 (dd, *J*= 8.5, 1.9 Hz, 1H), 7.49 *(d, J=* 8.7 Hz, 1H), 7.34 (d, *J* = 7.8 Hz, 1H), 7.18 (d, *J* = 7.7 Hz, 1H), 7.10 (s, 1H), 6.63 (s, 2H), 4.69 (s, 1H), 4.59 (dd, *J* = 10.3, 4.0 Hz, 1H), 3.86 (s, 3H), 3.83 (d, *J* = 7.3 Hz, 1H), 2.64 (s, 3H), 1.91 - 1.79 (m, 1H), 1.04 - 0.94 (m, 2H), 0.70 - 0.61 (m, 2H).

By referring to the synthetic route of compound **H4,** the following target molecules were synthesized using similar starting materials or intermediates.

* Indicates a chiral center and that it is not resolved.

| Compound | Target molecular structure | Resolution conditions: /$^1$H NMR/ | LC-MS $[M+H]^+$ |
|---|---|---|---|
| **H5** | 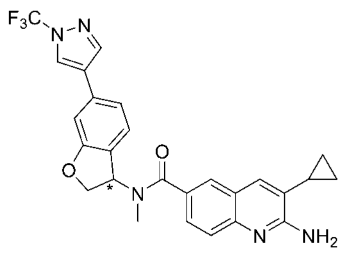 | $^1$H NMR (300 MHz, DMSO-$d_6$) $\delta$ 9.02 (s, 1H), 8.50 (s, 1H), 7.79 (s, 1H), 7.70 (s, 1H), 7.58 - 7.30 (m, 5H), 6.64 (s, 2H), 4.73 (s, 1H), 4.63 (d, *J* = 5.9 Hz, 1H), 2.64 (s, 3H), 1.83 (d, *J* = 5.1 Hz, 1H), 0.98 (d, *J* = 7.8 Hz, 2H), 0.66 (d, *J* = 5.0 Hz, 2H). | 494 |

**Example 5:** Synthesis of the **Target Molecules A1-A13**

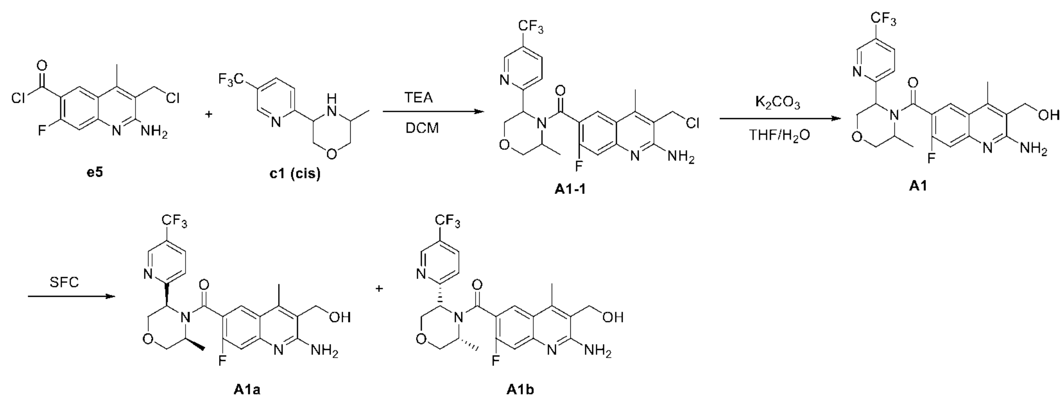


Step 1: Under an ice bath, the intermediate **c1** (43 mg, 0.17 mmol) and triethylamine (88 mg, 0.87 mmol) were dissolved in 2 mL of dichloromethane, then a dichloromethane solution of the intermediate e5 (51 mg, 0.17 mmol, 1 mL) was added dropwise. The mixture was allowed to react at room temperature for 1 hour. The solvent is evaporated under reduced pressure, and the crude product is purified by flash reverse-phase column chromatography (acetonitrile/water, 4/5), yielding a yellow solid **A1-1** (71 mg), with a yield of: 81%. LCMS ESI-MS m/z: 497 $[M+H]^+$.

Step 2: Under nitrogen protection, the previous step's compound **A1-1** (70 mg, 0.14 mmol) was dissolved in a mixed solution of 3 mL tetrahydrofuran and water (v/v, 1/1). $K_2CO_3$ (78 mg, 0.56 mmol) was added and stirred for 5 minutes, and then the mixture was heated to 60°C to react for 10 hours, followed by cooling to room temperature. The solvent was removed under reduced pressure, and the crude product was purified by flash reverse-phase column chromatography (acetonitrile/water, 4/5) to obtain the target molecule **A1** (55 mg), with a yield of: 82%. LCMS ESI-MS m/z: 479 $[M+H]^+$.

Step 3: The compound **A1** (55 mg) was separated by SFC chiral column chromatography under the following conditions of: chromatographic column: Chiral ART Cellulose-SA, 2*25 cm, 5 $\mu$m; mobile phase A: Hex(0.5% 2 M $NH_3$-MeOH), and mobile phase B: EtOH; flow rate: 20 mL/min; **A1a** retention time: 11.935 min; **A1b** retention time: 15.066

min.

**A1a:** $^1$H NMR (300 MHz, DMSO-$d_6$) $\delta$ 9.00 (s, 1H), 8.23 (s, 1H), 8.00 (d, $J$ = 7.6 Hz, 1H), 7.79 (s, 1H), 7.21 (s, 1H), 6.49 (s, 2H), 5.71 (s, 1H), 5.11 (s, 2H), 4.57 (d, $J$ = 5.2 Hz, 2H), 3.89 - 3.50 (m, 3H), 2.71 - 2.49 (m, 3H), 0.77 (s, 3H).

**A1b:** $^1$H NMR (300 MHz, DMSO-$d_6$) $\delta$ 9.00 (s, 1H), 8.23 (s, 1H), 8.00 (d, $J$ = 7.7 Hz, 1H), 7.79 (s, 1H), 7.21 (s, 1H), 6.49 (s, 2H), 5.71 (s, 1H), 5.13 (d, $J$ = 5.4 Hz, 2H), 4.57 (d, $J$ = 4.9 Hz, 2H), 3.93 - 3.53 (m, 3H), 2.72 - 2.49 (m, 3H), 0.77 (s, 3H).

By referring to the synthetic route of the compound **A1,** the following target molecules were synthesized using similar starting materials or intermediates.

* Indicates a chiral center and that it is not resolved.

| Compound | Target molecular structure | Resolution conditions: /$^1$H NMR/ | LC-MS $[M+H]^+$ |
|---|---|---|---|
| **A2a** | 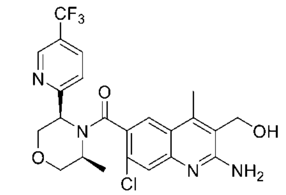 | Separation conditions: (chromatographic column: CHIRALPAK AD 2*25 cm, 5 μm; mobile phase A: HEX (0.5 % 2M $NH_3$-MeOH), and mobile phase B: ETOH; flow rate: 20 mL/min; retention time: 18.8 min; $^1$H NMR (400 MHz, DMSO-$d_6$) $\delta$ 9.03 (d, $J$= 7.5 Hz, 1H), 8.24 (s, 1H), 8.02 (s, 1H), 7.91 (d, $J$ = 8.5 Hz, 1H), 7.52 (d, $J$ = 49.5 Hz, 1H), 6.58 (s, 2H), 5.72 (s, 1H), 5.17 (d, $J$ = 5.9 Hz, 1H), 4.62 (d, $J$ = 4.9 Hz, 2H), 3.92 - 3.61 (m, 3H), 3.59 - 3.38 (m, 1H), 2.61 (d, $J$ = 14.6 Hz, 2H), 2.40 (s, 1H), 1.24 (s, 1H), 0.95 - 0.61 (m, 3H). | 495 |
| **A2b** | 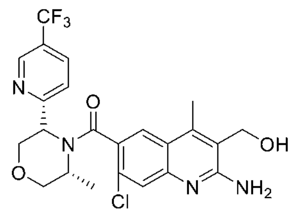 | Separation conditions: same as above; retention time: 27.098 min; $^1$H NMR (400 MHz, DMSO-$d_6$) $\delta$ 9.03(d, $J$ = 7.5 Hz, 1H), 8.40 - 8.13 (m,1H), 8.02 (s, 1H), 7.91 (d, $J$ = 8.4 Hz, 1H), 7.52 (d, $J$ = 49.5 Hz, 1H), 6.55 (d, $J$ = 26.8 Hz, 2H), 5.72 (s, 1H), 5.21 - 5.11 (m, 1H), 4.58 (d, $J$ = 33.4 Hz, 2H), 4.00 - 3.38 (m, 4H), 2.62 (d, $J$ = 14.6 Hz, 2H), 2.41 (s, 1H), 1.43 - 0.95 (m,1H), 0.95 - 0.64 (m, 3H). | 495 |
| **A3** | 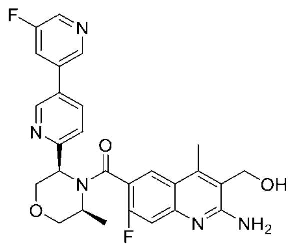 + 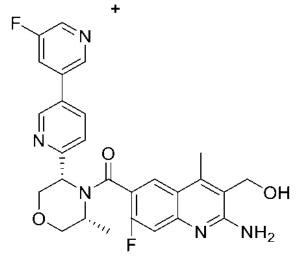 | $^1$H NMR (400 MHz, DMSO-$d_6$) $\delta$ 9.05 (s, 1H), 8.92 (s, 1H), 8.65 (d, $J$ = 2.7 Hz, 1H), 8.25 (s, 2H), 8.05 (s, 1H), 7.70 (s, 1H), 7.24 (s, 1H), 6.51 (s, 2H), 5.69 (s, 1H), 5.14 (s, 2H), 4.59 (s, 2H), 3.75 (d, $J$ = 34.5 Hz, 3H), 2.57 (d, $J$ = 14.5 Hz, 3H), 0.86 (s, 3H). | 506 |

(continued)

| Compound | Target molecular structure | Resolution conditions: /¹H NMR/ | LC-MS [M+H]⁺ |
|---|---|---|---|
| **A4** | 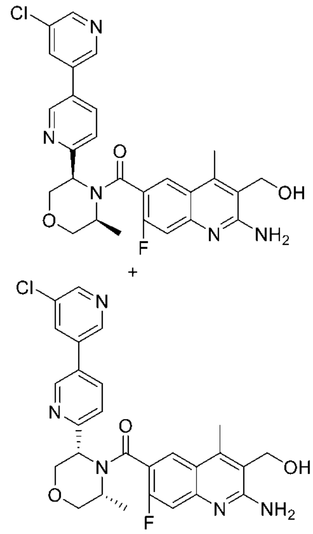 + | ¹H NMR (300 MHz, DMSO-*d*$_6$) δ 9.01 (d, *J* = 19.3 Hz, 2H), 8.69 (d, *J* = 2.3 Hz, 1H), 8.40 (s, 1H), 8.26 (s, 1H), 8.03 (s, 1H), 7.69 (s, 1H), 7.22 (s, 1H), 6.49 (s, 2H), 5.13 (s, 2H), 4.59 (s, 2H), 3.70 (s, 3H), 2.58 (s, 3H), 0.86 (s, 3H). | 522 |
| **A5** | 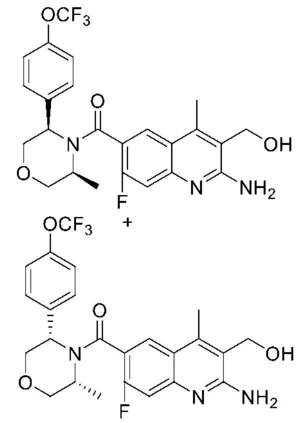 + | ¹H NMR (300 MHz, DMSO-*d*$_6$) δ 7.93 (d, *J* = 7.5 Hz, 1H), 7.70 (s, 2H), 7.39 (d, *J* = 8.3 Hz, 2H), 7.22 (d, *J* = 11.5 Hz, 1H), 6.50 (s, 2H), 5.14 (d, *J* = 5.3 Hz, 1H), 4.71 (d, *J* = 12.2 Hz, 1H), 4.60 (d, *J* = 5.3 Hz, 2H), 3.75 (dd, *J* = 46.5, 16.2 Hz, 4H), 2.56 (s, 3H), 0.76 (s, 3H). | 494 |
| A6 | 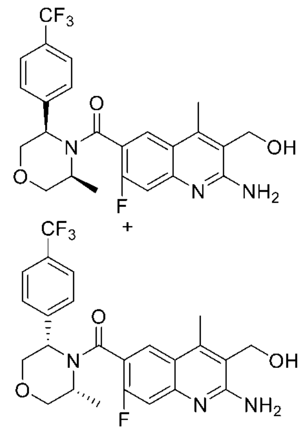 + | ¹H NMR (300 MHz, DMSO-*d*$_6$) δ 7.94 (d, *J* = *7.6* Hz, 1H), 7.76 (d, *J* = 8.1 Hz, 4H), 7.23 (d, *J* = 11.4 Hz, 1H), 6.49 (s, 2H), 5.14 (t, *J* = *5.3* Hz, 1H), 4.75 (d, *J* = 12.7 Hz, 1H), 4.60 (d, *J* = 5.3 Hz, 2H), 3.96-3.51 (m, 4H), 2.56 (s, 3H), 0.75 (s, 3H). | 478 |

(continued)

| Compound | Target molecular structure | Resolution conditions: /$^1$H NMR/ | LC-MS $[M+H]^+$ |
|---|---|---|---|
| **A7** | 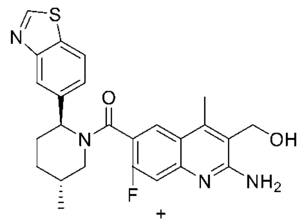 + 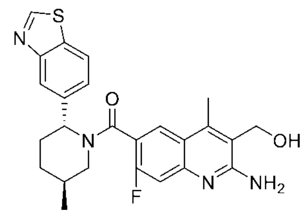 | $^1$H NMR (400 MHz, DMSO-$d_6$) $\delta$ 9.42 (s, 1H), 8.19 (s, 1H), 8.00 (d, $J$ = 56.3 Hz, 2H), 7.51 (s, 1H), 7.22 (s, 1H), 6.48 (s, 2H), 5.12 (s, 1H), 4.58 (s, 2H), 3.18 (s, 1H), 2.54 (s, 3H), 2.27 (d, $J$ = 40.6 Hz, 3H), 1.71 (s, 2H), 1.37 (d, $J$ = 13.3 Hz, 1H), 1.23 (s, 1H), 0.94 (s, 3H). | 465 |
| **A8** | 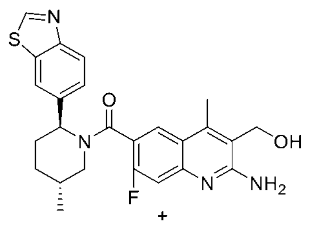 + 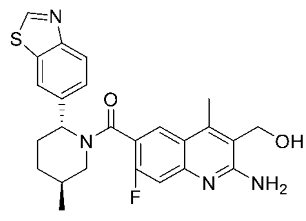 | $^1$H NMR (300 MHz, DMSO-$d_6$) $\delta$ 9.38 (s, 1H), 8.16 (dd, $J$ = 33.2, 12.9 Hz, 3H), 7.88 (s, 1H), 7.53 (s, 1H), 7.21 (d, $J$ = 11.7 Hz, 1H), 6.46 (s, 2H), 6.00 (s, 1H), 4.57 (s, 2H), 3.51 (s, 2H), 2.30 (d, $J$ = 1.7 Hz, 4H), 1.78 (d, $J$ = 37.3 Hz, 3H), 1.36 (d, $J$ = 12.9 Hz, 1H), 0.98 (s, 3H). | 465 |
| **A9** | 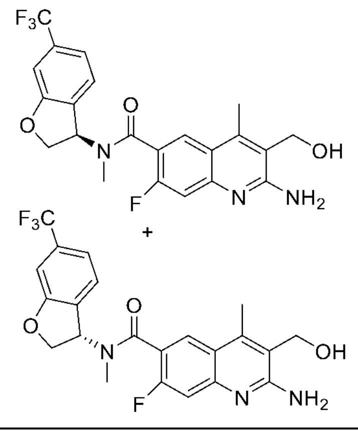 + | $^1$H NMR (400 MHz, DMSO-$d_6$) $\delta$ 7.94 (d, $J$ = 7.6 Hz, 1H), 7.59 (d, $J$ = 7.8 Hz, 1H), 7.39 - 7.10 (m, 3H), 6.51 (s, 3H), 5.68 - 4.80 (m, 1H), 4.77 - 4.56 (m, 4H), 2.71 - 2.52 (m, 6H). | 450 |
| **A9a (S Configuration)** | 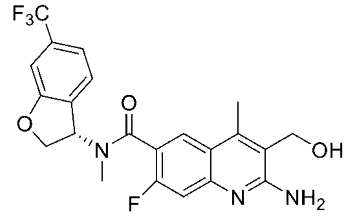 | $^1$H NMR (400 MHz, DMSO-$d_6$) $\delta$ 7.94 (d, $J$ = 7.6 Hz, 1H), 7.59 (d, $J$ = 7.8 Hz, 1H), 7.27 (ddd, $J$ = 41.2, 22.4, 9.9 Hz, 3H), 6.67 - 6.37 (m, 3H), 5.15 (t, $J$ = 5.3 Hz, 1H), 4.91 - 4.53 (m, 4H), 2.71 - 2.53 (m, 6H). | 450 |

(continued)

| Compound | Target molecular structure | Resolution conditions: /$^1$H NMR/ | LC-MS $[M+H]^+$ |
|---|---|---|---|
| **A10** | 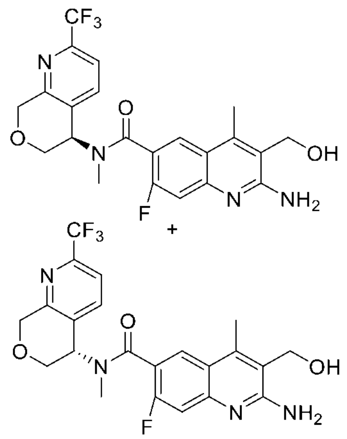 + | $^1$H NMR (300 MHz, DMSO-$d_6$) $\delta$ 8.24 - 7.81 (m, 3H), 7.23 (dd, $J$ = 11.6, 5.7 Hz, 1H), 6.55 (s, 2H), 6.05 - 5.82 (m, 1H), 5.21 - 5.07 (m, 1H), 4.97 - 4.72 (m, 2H), 4.72 - 4.54 (m, 2H), 4.20 (d, $J$ = 5.1 Hz, 1H), 4.16 - 3.99 (m, 1H), 2.84 (d, $J$ = 29.3 Hz, 1H), 2.69 (s, 2H), 2.58 (d, $J$ = 3.3 Hz, 3H). | 465 |
| **A11** | 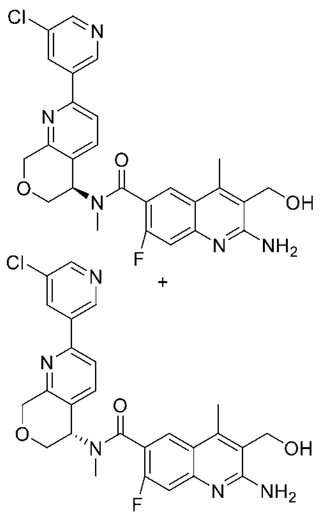 + | $^1$H NMR (300 MHz, DMSO-$d_6$) $\delta$ 9.23 (dd, $J$ = 10.0, 1.9 Hz, 1H), 8.71 (dd, $J$ = 3.9, 2.3 Hz, 1H), 8.54 (dt, $J$ = *9.7,* 2.2 Hz, 1H), 8.14 (t, $J$ = 8.6 Hz, 1H), 8.00 (d, $J$ = 7.6 Hz, 1H), 7.89 (d, $J$ = 8.1 Hz, 1H), 7.23 (dd, $J$ = 11.6, 9.1 Hz, 1H), 6.50 (s, 2H), 5.88 (s, 1H), 5.14 (d, $J$ = 5.7 Hz, 1H), 4.95 - 4.74 (m, 2H), 4.61 (d, $J$ = 4.8 Hz, 2H), 4.19 (d, $J$ = 4.4 Hz, 2H), 2.76 (d, $J$ = 39.6 Hz, 3H), 2.59 (d, $J$ = 1.5 Hz, 3H). | 508 |
| **A12** | 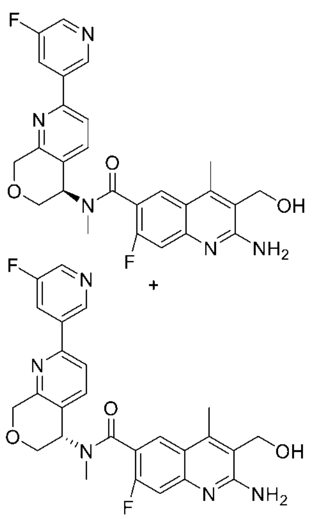 + | $^1$H NMR (300 MHz, DMSO-$d_6$) $\delta$ 9.17 (dt, $J$ = 10.1, 1.8 Hz, 1H), 8.67 (t, $J$ = 3.2 Hz, 1H), 8.40 - 8.26 (m, 1H), 8.13 (t, $J$ = *9.1* Hz, 1H), 8.00 *(d,* $J$ = 7.7 Hz, 1H), 7.89 (d, $J$ = 8.2 Hz, 1H), 7.23 (dd, $J$ = 11.7, 9.2 Hz, 1H), 6.50 (s, 2H), 5.89 (d, $J$ = *4.7* Hz, 1H), 5.15 (q, $J$ = 4.3, 3.2 Hz, 1H), 4.96 - 4.78 (m, 2H), 4.77 - 4.56 (m, 2H), 4.26 - 4.03 (m, 2H), 2.86 - 2.65 (m, 3H), 2.59 (d, $J$ = 1.5 Hz, 3H). | 492 |

(continued)

| Compound | Target molecular structure | Resolution conditions: /$^1$H NMR/ | LC-MS $[M+H]^+$ |
|---|---|---|---|
| **A13** | 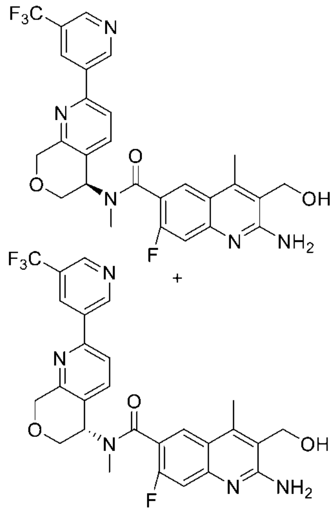 + | $^1$H NMR: (300 MHz, DMSO-$d_6$) $\delta$ 9.58 (d, *J = 12.7* Hz, 1H), 9.08 (s, 1H), 8.78 (d, *J* = 13.3 Hz, 1H), 8.26 (d, *J* = 8.2 Hz, 1H), 8.02 (d, *J* = 7.6 Hz, 1H), 7.93 (d, *J* = 7.7 Hz, 1H), 7.24 (t, *J* = 12.1 Hz, 1H), 6.54 (s, 2H), 5.90 (s, 1H), 5.16 (d, *J* = 5.4 Hz, 1H), 4.95 - 4.80 (m, 2H), 4.61 (d, *J* = 5.3 Hz, 2H), 4.18 (d, *J* = 17.3 Hz, 2H), 2.76 (d, *J* = 52.9 Hz, 3H), 2.60 (d, *J* = 2.0 Hz, 3H). | 542 |

**Example 6:**

MTAP-deficient cells, due to the accumulation of MTA, are sensitive to PRMT5-MTA inhibitors in terms of proliferation; in contrast, MTAP wild-type (MTAP-normal) cells do not exhibit MTA accumulation effects and do not depend on PRMT5. By testing the activity of both of them, the inhibitory effect and selectivity of the molecules of the present invention for PRMT5 at the cellular level were shown.

HCT116 wild-type and MTAP-deficient cells were cultured in MCCOYS 5A medium containing 10% FBS and 1% penicillin-streptomycin and incubated in a 37°C, 5% $CO_2$ incubator. A 40 μL cell suspension was added to each well of a 384-well microplate. Using Echo, 40 nL of the compound at different concentrations were added to each well, followed by incubation in a 37°C, 5% $CO_2$ incubator for 7-10 days. 40 μL of CTG solution (Promega, Cat No. G7573) was added to each well and incubated away from light in a 37°C, 5% $CO_2$ incubator for 30 minutes. Luminescence was measured using an Envision multimode plate reader (Perkin Elmer, Catalog No. Envision 2104). The luminescence signal was proportional to the ATP content in the system, and the ATP content directly reflected the number of viable cells in the system.

ICso value calculation:

$$Y = \text{Lower plateau signal} + (\text{Upper plateau signal} - \text{Lower plateau signal}) / (1 + 10^\wedge((\text{LogIC}_{50} - X) \times \text{Hill slope}))$$

X: Log value of compound concentration
Y: Inhibition rate (%)

Table 1: 2D Anti-proliferation Effects of Compounds on HTC116-MTAP *del* and Wild-type Colorectal Cancer HCT-116 Cell Lines.

| Compound | HCT116-MTAP*del*/$IC_{50}$/nM | HCT116-MTAP*wt*/$IC_{50}$/nM | HCT116 WT/MTAP*del* Selectivity |
|---|---|---|---|
| **P1a** | 6985 | >10000 | >1 |
| **P1b** | 10 | 535 | 54 |
| **P2** | 93 | >10000 | >108 |
| **P3** | 2873 | >10000 | >4 |
| **P4** | 790 | >10000 | >13 |
| **P5** | 612 | >10000 | >16 |
| **P6** | 156 | N.D. | N.D. |

(continued)

| Compound | HCT116-MTAP*del*/$IC_{50}$/nM | HCT116-MTAP*wt*/$IC_{50}$/nM | HCT116 WT/MTAP*del* Selectivity |
|---|---|---|---|
| **P7a** | 230 | 1639 | 7 |
| **P7b** | 2536 | 3803 | 2 |
| **P8** | 96 | >10000 | >104 |
| **P9** | 16 | 936 | 59 |
| **P10** | 34 | 1858 | 55 |
| **H1** | 4250 | >10000 | >2 |
| **H2** | 6292 | >10000 | >1 |
| **H3** | 926 | >10000 | >11 |
| **H4** | 4346 | >10000 | >3 |
| **H5** | 2955 | 8017 | 3 |
| H6 | 33 | 532 | 16 |
| **H7** | 29 | 1109 | 38 |
| **A1a** | 28 | 4073 | 145 |
| **A1b** | 4 | 326 | 82 |
| **A2a** | 3032 | >10000 | >3 |
| **A2b** | 4 | 398 | 100 |
| **A3** | 5 | 1453 | 291 |
| **A4** | 7 | 1145 | 164 |
| **A5** | 18 | 1357 | 75 |
| **A6** | 7 | 1017 | 145 |
| **A7** | 88 | 3879 | 44 |
| **A8** | 150 | 1996 | 13 |
| **A9** | 27 | 4705 | 174 |
| **A9a** | 16 | 2470 | 130 |
| **A10** | 18 | >10000 | >556 |
| **A11** | 26 | 4380 | 168 |
| **A12** | 36 | 3253 | 90 |
| **A13** | 32 | 3753 | 117 |
| **AM9747** | 7 | 237 | 34 |
| **TNG908** | 54 | 1232 | 23 |
| **GSK3326595** | 17 | 72 | 4 |
| N.D. = Not tested | | | |

The above experimental results indicate that the excellent compounds of the present invention, by inhibiting PRMT5, exhibit a significant antiproliferative effect on MTAP-deleted tumor cells, while having weaker inhibition on the wild-type ones. Some molecules exhibit very high selectivity (greater than 50-fold), which is expected to bring higher safety. In contrast, the selectivity of the reference molecule **AM9747** is only around 30-fold, which may lead to dose limitations in clinical use due to insufficient selectivity. **TNG908,** which has just entered Phase 1 clinical trials, has only 23-fold selectivity; additionally, **GSK3326595** was temporarily discontinued in clinical trials due to insufficient selectivity. Therefore, the high selectivity of the present invention is expected to reduce side effects while improving efficacy in clinical use.

The above experimental results prove that, compared with **P2b** and **P1** or **P4,** the substitution of $R_2$ and $R_3$ has a

significant impact on activity.

| AM9747 structure: | GSK3326595 structure: | TNG908 structure: |
|---|---|---|
| 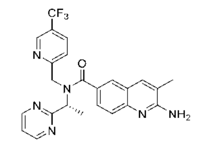 | 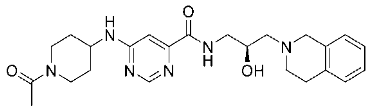 | 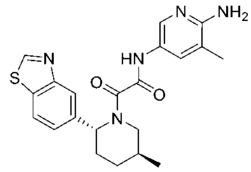 |

**Example 7:**

Microsomal stability experiment of the compound. The details are as follows:
A microsomal stability test was conducted on the compounds of the present invention. The test compounds were co-incubated with liver microsomes from different species, with or without the addition of NADPH. In the test system, the final concentration of the test compound was 1 μM, the final concentration of NADPH was 1 mM, and the final concentration of liver microsomes was 0.5 mg/mL. The compound concentration in the incubation supernatant was measured at different time points within 60 minutes, and pharmacokinetic parameters (such as intrinsic clearance rate $Cl_{int}$) were calculated.

The results indicate that the molecules of the present invention have good metabolic stability (particularly in humans, where they exhibit good metabolic stability). Some molecules exhibit a lower clearance rate in human liver microsomal metabolism compared to **AM9747,** resulting in slower metabolism in the human body.

| Compound | Human $Cl_{int}$/(mL/min/kg) | Mouse $Cl_{int}$/(mL/min/kg) |
|---|---|---|
| **A9** | 3.6 | N.D. |
| **A9a** | 8.9 | N.D. |
| **A10** | 11.1 | N.D. |
| **AM9747** | 27.1 | 169 |
| N.D. = Not tested | | |

**Example 8:**

Membrane permeability evaluation experiment: Caco-2 Assays

Evaluation of membrane permeability of the molecules of the present invention. Samples will be analyzed using LC-MS to estimate the apparent permeability coefficient ($P_{app}$) of the compound in Caco-2 monolayer cells, wherein the pH of the apical chamber is 6.5, and the pH of the basolateral chamber is 7.4. The P-gp efflux transporter, BCRP, and MRP2 inhibitors (50 μM quinidine, 30 μM benzbromarone, and 20 μM sulfasalazine) can block the active efflux transport of the compound. The data will be used for calculating apparent permeability (Papp).

$$P_{app} = (V_A \times [drug]_{acceptor})/(Area \times Time \times [drug]_{initial,donor})$$

wherein, VA is the volume of the receptor well (unit: mL), area is the membrane surface area (for Transwell-96 well permeable support, it is 0.143 $cm^2$), and time is the total transport time (unit: seconds).

$$Efflux\ Ratio = P_{app}(B\text{-}A)/P_{app}(A\text{-}B)$$

The Caco-2 membrane permeability data of the molecules of the present invention are shown as follows:

| Compound | Papp(A-B) ($10^{-6}$ cm/s) | Papp(B-A) ($10^{-6}$ cm/s) | Efflux |
|---|---|---|---|
| **A10** | 8.1 | 31 | 3.8 |
| **A9** | 5.1 | 5.9 | 1.2 |

(continued)

| Compound | Papp(A-B) ($10^{-6}$ cm/s) | Papp(B-A) ($10^{-6}$ cm/s) | Efflux |
|---|---|---|---|
| **A9a** | 6.9 | 10.8 | 1.6 |

The above results indicate that the molecules of the present invention have good membrane permeability, particularly compounds **A9** and **A9a,** which have extremely low efflux rates (efflux < 2), making them promising candidates for achieving better in vivo pharmacokinetics and effective tumor inhibition.

**Example 9:**

Membrane permeability evaluation experiment: MDCK-MDR1 assays

Evaluation of membrane permeability of the molecules of the present invention to predict their permeability for the brain.

Specifically: 1. Pre-incubate MDCK-MDR1 cells at a density of $1.56 \times 10^6$ cells/mL, inoculate them in a 96-well plate to culture at 37°C for 7 days; 2. Remove the MDCKII-MDR1 plate from the incubator, wash twice with pre-warmed HBSS (10 mM HEPES, pH 7.4), and incubate at 37°C for 30 minutes; 3. Dilute the stock solution of the test compound with DMSO to obtain a 0.2 mM solution, then further dilute it with HBSS (10 mM HEPES, pH 7.4) to prepare a 1 μM working solution. For the blank control group, dilute with DMSO to obtain a 0.2 mM solution, then further dilute with HBSS (10 mM HEPES, pH 7.4) to obtain a 1 μM working solution. The final concentration of DMSO in the culture system is 0.5%; 4. Measure the transport rate of the drug from the apical side to the basolateral side. Add 125 μL of the 1 μM test compound solution into the Transwell insert (apical chamber) and immediately transfer 50 μL of the sample (D0 sample) from the apical chamber to a new 96-well plate. Fill the receptor plate wells (basolateral chamber) with 235 μL HBSS (10 mM HEPES, pH 7.4) and incubate at 37°C for 2 hours; 5. At the end of the incubation, transfer 50 μL of the sample from both the donor side and the acceptor side into the wells of a new 96-well plate, then add four volumes of cold acetonitrile containing the appropriate internal standard (IS). Before the LC-MS/MS analysis, take 100 μL of the supernatant and mix it with an appropriate volume of ultrapure water.

$$P_{app} = (V_A \times [drug]_{acceptor}) / (Area \times Time \times [drug]_{initial,donor})$$

wherein VA is the volume of the acceptor well (unit: mL), area is the membrane surface area (for Transwell-96 well permeable support, it is 0.143 $cm^2$), and time is the total transport time (in seconds).

$$\text{Efflux Ratio} = P_{app}(B\text{-}A) / P_{app}(A\text{-}B)$$

The MDCK-MDR1 membrane permeability data of the molecules of the present invention are shown as follows:

| Compound | Papp(A-B) ($10^{-6}$ cm/s) | Papp(B-A) ($10^{-6}$ cm/s) | Efflux |
|---|---|---|---|
| **A9a** | 5.1 | 5.9 | 1.2 |

The above results indicate that the molecules of the present invention have good membrane permeability, particularly compound A9a, which has an extremely low efflux rate (efflux 1.2) and is expected to achieve a favorable brain concentration when administered in vivo.

**Example 10:**

Pharmacokinetic evaluation experiment in mice

CD1 female mice were used as test animals, and the compounds were administered orally or intravenously (oral dose of 10 mg/kg; intravenous dose of 2 mg/kg).

Solvent (intravenous: 5% DMSO + 95% deionized water solution containing 20% HP-β-CD; oral: 0.1% Tween-80 + 0.5% methylcellulose + 99.4% deionized water).

Experiment protocol: Three mice per group for oral administration, and three mice per group for intravenous administration. Oral: Plasma samples were collected before administration (0 h) and at 0.25, 0.5, 1, 2, 4, 8, and 24 hours post-administration; Intravenous: Plasma samples were collected before administration (0 h) and at 0.083, 0.25, 0.5, 1, 2,

4, 8, and 24 hours post-administration. The plasma drug concentrations in mice after oral and intravenous administration were determined using LC/MS/MS, and the collected data were analyzed using AB Sciex QTRAP 6500 software. The experimental results are as follows:

| Compound | | A9a | AM9747 |
|---|---|---|---|
| IV (2 mg/kg) | $T_{1/2}$ (h) | 3.3 | 0.35 |
| | $C_0$ (ng/mL) | 4012 | 1604 |
| | $AUC_{0-24}$ (h*ng/mL) | 19530 | 417 |
| | Cl (mL/min/kg) | 1.7 | 79.2 |
| PO (10 mg/kg) | $T_{1/2}$ (h) | 5.9 | 1.4 |
| | $T_{max}$ (h) | 2.0 | 0.25 |
| | $C_{max}$(ng/mL) | 5337 | 1064 |
| | $AUC_{0-24}$ (h*ng/mL) | 74190 | 1267 |
| | F (%) | 81 | 61 |

The above experimental results indicate that the compounds of the present invention exhibit good absorption when taken orally and have higher in vivo exposure. Due to their superior selectivity and in vivo exposure compared to reported molecules such as AM9747, they are expected to provide higher therapeutic efficacy.

## Claims

1. A compound of formula (I), or a pharmaceutically acceptable salt, isotopic variant, tautomer, or stereoisomer thereof:

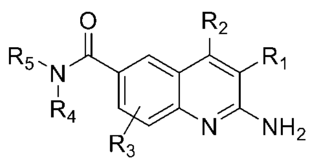
(I)

wherein,

$R_1$ is selected from $C_{1-6}$ alkyl, $C_{3-6}$ cycloalkyl, or $-CH_2OR_a$;
$R_a$ is selected from H or methyl;
$R_2$ is selected from H, $C_{1-6}$ alkyl, $C_{1-6}$ alkoxy, or halogen;
$R_3$ is selected from H, halogen, $OR_b$, CN, $C_{1-6}$ alkyl, or $C_{1-6}$ haloalkyl;
$R_4$ and $R_5$ are independently selected from H, $C_{1-6}$ alkyl, $C_{1-6}$ haloalkyl, $C_{3-6}$ cycloalkyl, 4-12 membered heterocyclyl, or 5-10 membered heteroaryl; the 4-12 membered heterocyclyl or 5-10 membered heteroaryl is optionally substituted with one or two $R_6$, $R_6$ being selected from H, CN, halogen, $C_{1-6}$ alkyl, $C_{1-6}$ haloalkyl, SFs, $OR_b$, $SCF_3$, or 5-6 membered heteroaryl;
alternatively, $R_4$, Rs, and the nitrogen atom to which they are attached together form 4-10 membered heterocyclyl, the 4-10 membered heterocyclyl being optionally substituted with one, two, three, or four $R_x$;
$R_x$ is selected from halogen, $C_{1-6}$ alkyl, $C_{1-6}$ haloalkyl, $C_{1-6}$ haloalkoxy, phenyl or 5-10 membered heteroaryl, the phenyl or 5-10 membered heteroaryl being optionally substituted with one, two, or three $R_y$;
$R_y$ is selected from halogen, $C_{1-6}$ alkyl, $C_{1-6}$ haloalkyl, SFs, CN, $OR_b$, $C_{1-6}$ alkoxy, $C_{3-6}$ cycloalkyl, 4-12 membered heterocyclyl, 5-10 membered heteroaryl or phenyl, the 5-10 membered heteroaryl or phenyl being optionally substituted with one, two, or three $R_z$;
$R_z$ is selected from $C_{1-6}$ alkyl, halogen, CN, $C_{1-6}$ haloalkyl, or $C_{1-6}$ alkoxy;
$R_b$ is selected from H, $C_{1-6}$ alkyl, or $C_{1-6}$ haloalkyl.

2. The compound of claim 1, or a pharmaceutically acceptable salt, isotopic variant, tautomer, or stereoisomer thereof, which is a compound of formula (II):

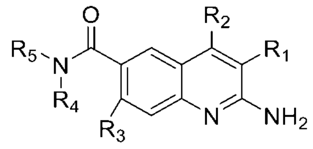 (II)

wherein,

$R_1$ is selected from $C_{1-6}$ alkyl, cyclopropyl, or $-CH_2OH$;
$R_2$ is selected from H, $C_{1-6}$ alkyl, $C_{1-6}$ alkoxy, or halogen;
$R_3$ is selected from H, halogen, CN, or methyl;
$R_4$ and $R_5$ are independently selected from H, $C_{1-6}$ alkyl, $C_{1-6}$ haloalkyl, $C_{3-6}$ cycloalkyl, 4-12 membered heterocyclyl, or 5-10 membered heteroaryl; the 4-12 membered heterocyclyl or 5-10 membered heteroaryl is optionally substituted with one or two $R_6$, $R_6$ being selected from H, CN, halogen, $C_{1-6}$ alkyl, $C_{1-6}$ haloalkyl, SFs, $OR_b$, $SCF_3$, or 5-6 membered heteroaryl;
alternatively, $R_4$, Rs, and the nitrogen atom to which they are attached together form a 4-10 membered heterocyclyl, the 4-10 membered heterocyclyl being optionally substituted with one, two, three, or four $R_x$;
$R_x$ is selected from halogen, $C_{1-6}$ alkyl, $C_{1-6}$ haloalkyl, $C_{1-6}$ haloalkoxy, phenyl or 5-10 membered heteroaryl, the phenyl or 5-10 membered heteroaryl being optionally substituted with one, two, or three $R_y$;
$R_y$ is selected from halogen, $C_{1-6}$ alkyl, $C_{1-6}$ haloalkyl, SFs, CN, $OR_b$, $C_{1-6}$ alkoxy, $C_{3-6}$ cycloalkyl, 4-12 membered heterocyclyl, 5-10 membered heteroaryl or phenyl, the 5-10 membered heteroaryl or phenyl being optionally substituted with one, two, or three $R_z$;
$R_z$ is selected from $C_{1-6}$ alkyl, halogen, CN, $C_{1-6}$ haloalkyl, or $C_{1-6}$ alkoxy;
$R_b$ is selected from H, $C_{1-6}$ alkyl, or $C_{1-6}$ haloalkyl.

**3.** The compound of claim 1 or 2, or a pharmaceutically acceptable salt, isotopic variant, tautomer, or stereoisomer thereof, which has the following structure:

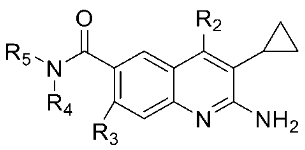 (III) or 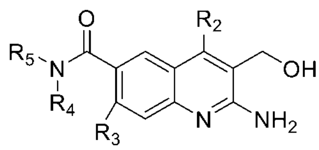 (IV)

wherein, each group is as defined in claim 1 or 2.

**4.** The compound of claim 3, or a pharmaceutically acceptable salt, isotopic variant, tautomer, or stereoisomer thereof, wherein,

$R_2$ is selected from H, methyl, ethyl, methoxy, or F;
$R_3$ is selected from H, F, Cl, CN, or methyl;
$R_4$ and $R_5$ are independently selected from H, $C_{1-6}$ alkyl, $C_{1-6}$ haloalkyl, $C_{3-6}$ cycloalkyl, 4-12 membered heterocyclyl, or 5-10 membered heteroaryl; the 4-12 membered heterocyclyl or 5-10 membered heteroaryl is optionally substituted with one or two $R_6$, $R_6$ being selected from H, CN, halogen, $C_{1-6}$ alkyl, $C_{1-6}$ haloalkyl, SFs, $OR_b$, $SCF_3$, or 5-6 membered heteroaryl;
alternatively, $R_4$, Rs, and the nitrogen atom to which they are attached together form a 4-10 membered heterocyclyl, the 4-10 membered heterocyclyl being optionally substituted with one, two, three, or four $R_x$;
$R_x$ is selected from halogen, $C_{1-6}$ alkyl, $C_{1-6}$ haloalkyl, $C_{1-6}$ haloalkoxy, phenyl or 5-10 membered heteroaryl, the phenyl or 5-10 membered heteroaryl being optionally substituted with one, two, or three $R_y$;
$R_y$ is selected from halogen, $C_{1-6}$ alkyl, $C_{1-6}$ haloalkyl, SFs, CN, $OR_b$, $C_{1-6}$ alkoxy, $C_{3-6}$ cycloalkyl, 4-12 membered heterocyclyl, 5-10 membered heteroaryl or phenyl, the 5-10 membered heteroaryl or phenyl being optionally substituted with one, two, or three $R_z$;
$R_z$ is selected from $C_{1-6}$ alkyl, halogen, CN, $C_{1-6}$ haloalkyl, or $C_{1-6}$ alkoxy;
$R_b$ is selected from H, $C_{1-6}$ alkyl, or $C_{1-6}$ haloalkyl.

**5.** The compound of any one of claims 1-4, or a pharmaceutically acceptable salt, isotopic variant, tautomer, or stereoisomer thereof, which has the following structures:

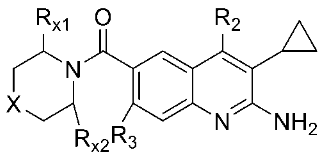 (III-1), 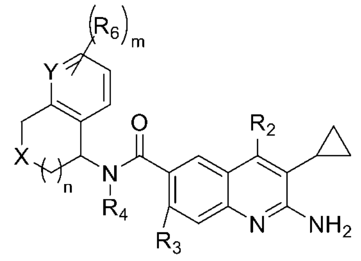 (III-2), 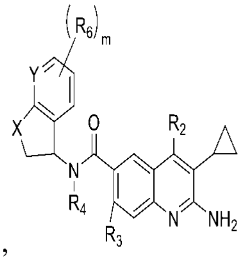 (III-3),

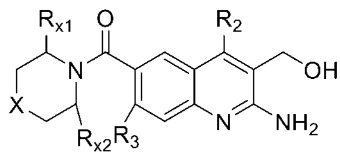 (IV-1), 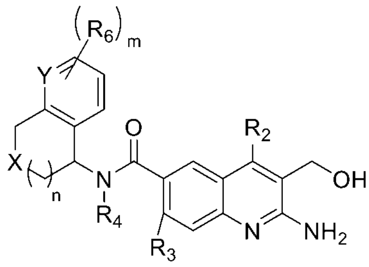 (IV-2),

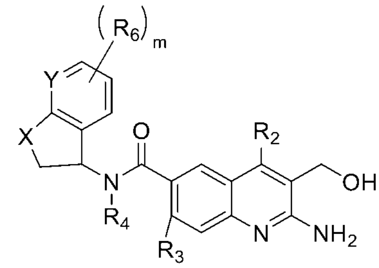 (IV-3),

wherein,

$R_2$ is selected from H, methyl, ethyl, methoxy, or F;
$R_3$ is selected from H, F, Cl, CN, or methyl;
$R_4$ is selected from H, $C_{1-6}$ alkyl, $C_{1-6}$ haloalkyl, $C_{3-6}$ cycloalkyl, or 4-12 membered heterocyclyl;
$R_{x1}$ and $R_{x2}$ are selected from halogen, $C_{1-6}$ alkyl, $C_{1-6}$ haloalkyl, $C_{1-6}$ haloalkoxy, phenyl or 5-10 membered heteroaryl, the phenyl or 5-10 membered heteroaryl being optionally substituted with one, two, or three $R_y$;
$R_y$ is selected from halogen, $C_{1-6}$ alkyl, $C_{1-6}$ haloalkyl, SFs, CN, $OR_b$, $C_{1-6}$ alkoxy, $C_{3-6}$ cycloalkyl, 4-12 membered heterocyclyl, 5-10 membered heteroaryl or phenyl, the 5-10 membered heteroaryl or phenyl being optionally substituted with one, two, or three $R_z$;
$R_z$ is selected from $C_{1-6}$ alkyl, halogen, CN, $C_{1-6}$ haloalkyl, or $C_{1-6}$ alkoxy;
$R_b$ is selected from H, $C_{1-6}$ alkyl, or $C_{1-6}$ haloalkyl;
$R_6$ is selected from H, CN, halogen, $C_{1-6}$ alkyl, $C_{1-6}$ haloalkyl, SFs, $OR_b$, $SCF_3$, or 5-6 membered heteroaryl;
X is selected from $-CH_2-$, O, S, or $-NR_c-$;
Y is selected from CH or N;
$R_c$ is selected from H or $C_{1-6}$ alkyl;
m is 0, 1, or 2;
n is 0, 1, 2, or 3.

**6.** The compound of claim 5, or a pharmaceutically acceptable salt, isotopic variant, tautomer, or stereoisomer thereof, which is a compound of formula (III-1), (III-1a), or (III-1b):

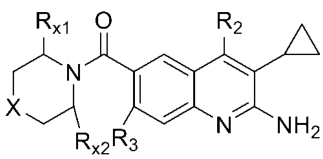 (III-1)

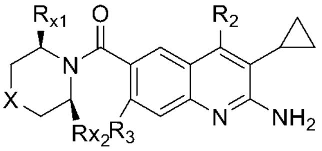 (III-1a) or 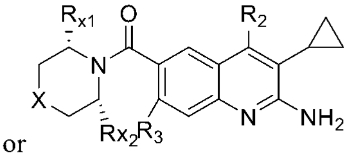 (III-1b)

wherein,

$R_2$ is selected from H, methyl, ethyl, methoxy, or F;
$R_3$ is selected from H, F, Cl, CN, or methyl;
$R_{x1}$ is selected from phenyl or 5-10 membered heteroaryl, the phenyl or 5-10 membered heteroaryl being optionally substituted with one, two, or three $R_y$;
$R_y$ is selected from halogen, $C_{1-6}$ alkyl, $C_{1-6}$ haloalkyl, SFs, CN, $OR_b$, $C_{1-6}$ alkoxy, $C_{3-6}$ cycloalkyl, 4-12 membered heterocyclyl, 5-10 membered heteroaryl or phenyl, the 5-10 membered heteroaryl or phenyl being optionally substituted with one, two, or three $R_z$;
$R_z$ is selected from $C_{1-6}$ alkyl, halogen, CN, $C_{1-6}$ haloalkyl, or $C_{1-6}$ alkoxy;
$R_b$ is selected from H, $C_{1-6}$ alkyl, or $C_{1-6}$ haloalkyl;
$R_{x2}$ is selected from $C_{1-6}$ alkyl, $C_{1-6}$ haloalkyl, or $C_{1-6}$ haloalkoxy;
X is selected from $-CH_2-$, O, S, or $-NR_c-$;
$R_c$ is selected from H or $C_{1-6}$ alkyl.

**7.** The compound of claim 6, or a pharmaceutically acceptable salt, isotopic variant, tautomer, or stereoisomer thereof, wherein,

$R_2$ is selected from H, methyl, ethyl, methoxy, or F;
$R_3$ is selected from H, F, Cl, CN, or methyl;
$R_{x1}$ is selected from phenyl or 6-membered heteroaryl, the phenyl or 6-membered heteroaryl being optionally substituted with one, two, or three $R_y$;
$R_y$ is selected from halogen, $C_{1-6}$ alkyl, $C_{1-6}$ haloalkyl, SFs, CN, $OR_b$, $C_{1-6}$ alkoxy, $C_{3-6}$ cycloalkyl, 4-12 membered heterocyclyl, 5-10 membered heteroaryl or phenyl, the 5-10 membered heteroaryl or phenyl being optionally substituted with one, two, or three $R_z$;
$R_z$ is selected from $C_{1-6}$ alkyl, halogen, CN, $C_{1-6}$ haloalkyl, or $C_{1-6}$ alkoxy;
$R_b$ is selected from H, $C_{1-6}$ alkyl, or $C_{1-6}$ haloalkyl;
$R_{x2}$ is selected from methyl, ethyl, trifluoromethyl, or trifluoromethoxy;
X is selected from $-CH_2-$, O, or S.

**8.** The compound of claim 5, or a pharmaceutically acceptable salt, isotopic variant, tautomer, or stereoisomer thereof, which is a compound of formula (III-2), (III-2a), or (III-2b):

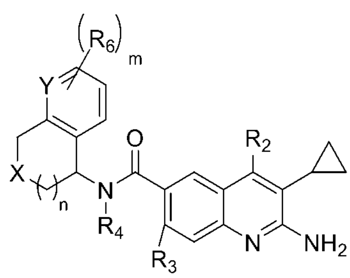 (III-2)

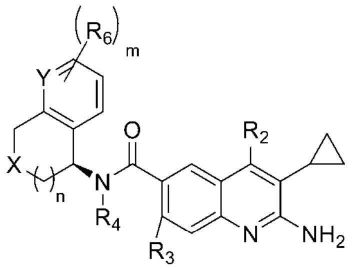 (III-2a) or 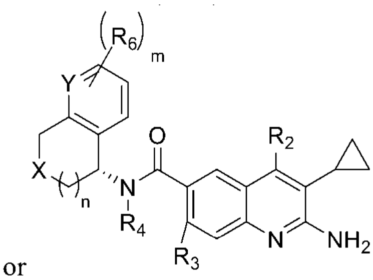 (III-2b)

wherein,

$R_2$ is selected from H, methyl, ethyl, methoxy, or F;
$R_3$ is selected from H, F, Cl, CN, or methyl;
$R_4$ is selected from H, $C_{1-6}$ alkyl, $C_{1-6}$ haloalkyl, $C_{3-6}$ cycloalkyl, or 4-12 membered heterocyclyl;
$R_6$ is selected from H, CN, halogen, $C_{1-6}$ alkyl, $C_{1-6}$ haloalkyl, SFs, $OR_b$, or a 5-6 membered heteroaryl;
$R_b$ is selected from H, $C_{1-6}$ alkyl, or $C_{1-6}$ haloalkyl;
X is selected from $-CH_2-$, O, S, or $-NR_c-$;
Y is selected from CH or N;
$R_c$ is selected from H or $C_{1-6}$ alkyl;
m is 0, 1, or 2;
n is 0, 1, 2, or 3.

**9.** The compound of claim 5, or a pharmaceutically acceptable salt, isotopic variant, tautomer, or stereoisomer thereof, which is a compound of formula (III-3), (III-3a), or (III-3b):

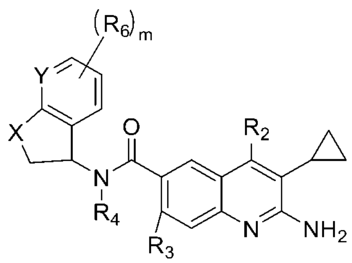 (III-3)

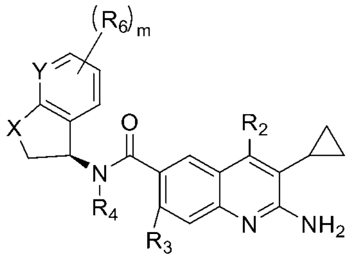 (III-3a) or 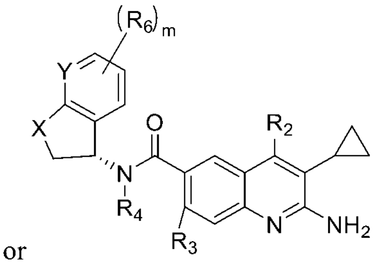 (III-3b)

wherein,

$R_2$ is selected from H, methyl, ethyl, methoxy, or F;
$R_3$ is selected from H, F, Cl, CN, or methyl;
$R_4$ is selected from H, $C_{1-6}$ alkyl, $C_{1-6}$ haloalkyl, $C_{3-6}$ cycloalkyl, or 4-12 membered heterocyclyl;
$R_6$ is selected from H, CN, halogen, $C_{1-6}$ alkyl, $C_{1-6}$ haloalkyl, SFs, $OR_b$, or 5-6 membered heteroaryl;
$R_b$ is selected from H, $C_{1-6}$ alkyl, or $C_{1-6}$ haloalkyl;
X is selected from $-CH_2-$, O, S, or $-NR_c-$;
Y is selected from CH or N;
$R_c$ is selected from H or $C_{1-6}$ alkyl;
m is 0, 1, or 2.

**10.** The compound of claim 5, or a pharmaceutically acceptable salt, isotopic variant, tautomer, or stereoisomer thereof, which is a compound of formula (IV-1), (IV-1a), or (IV-1b):

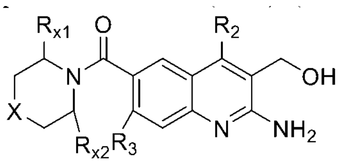

(IV-1)

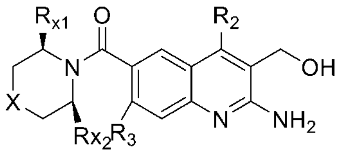

(IV-1a) or

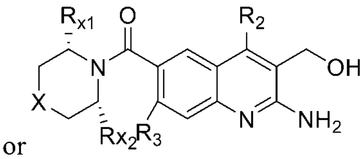

(IV-1b)

wherein,

$R_2$ is selected from H, methyl, ethyl, methoxy, or F;
$R_3$ is selected from H, F, Cl, CN, or methyl;
$R_{x1}$ is selected from phenyl or a 5-10 membered heteroaryl, the phenyl or 5-10 membered heteroaryl being optionally substituted with one, two, or three $R_y$;
$R_y$ is selected from halogen, $C_{1-6}$ alkyl, $C_{1-6}$ haloalkyl, SFs, CN, $OR_b$, $C_{1-6}$ alkoxy, $C_{3-6}$ cycloalkyl, 4-12 membered heterocyclyl, 5-10 membered heteroaryl or phenyl, the 5-10 membered heteroaryl or phenyl being optionally substituted with one, two, or three $R_z$;
$R_z$ is selected from $C_{1-6}$ alkyl, halogen, CN, $C_{1-6}$ haloalkyl, or $C_{1-6}$ alkoxy;
$R_b$ is selected from H, $C_{1-6}$ alkyl, or $C_{1-6}$ haloalkyl;
$R_{x2}$ is selected from $C_{1-6}$ alkyl, $C_{1-6}$ haloalkyl, or $C_{1-6}$ haloalkoxy;
X is selected from $-CH_2-$, O, S, or $-NR_c-$;
$R_c$ is selected from H or $C_{1-6}$ alkyl.

**11.** The compound of claim 10, or a pharmaceutically acceptable salt, isotopic variant, tautomer, or stereoisomer thereof, wherein,

$R_2$ is selected from H, methyl, ethyl, methoxy, or F;
$R_3$ is selected from H, F, Cl, CN, or methyl;
$R_{x1}$ is selected from phenyl or 6-membered heteroaryl, the phenyl or 6-membered heteroaryl being optionally substituted with one, two, or three $R_y$;
$R_y$ is selected from halogen, $C_{1-6}$ alkyl, $C_{1-6}$ haloalkyl, SFs, CN, $OR_b$, $C_{1-6}$ alkoxy, $C_{3-6}$ cycloalkyl, 4-12 membered heterocyclyl, 5-10 membered heteroaryl or phenyl, the 5-10 membered heteroaryl or phenyl being optionally substituted with one, two, or three $R_z$;
$R_z$ is selected from $C_{1-6}$ alkyl, halogen, CN, $C_{1-6}$ haloalkyl, or $C_{1-6}$ alkoxy;
$R_b$ is selected from H, $C_{1-6}$ alkyl, or $C_{1-6}$ haloalkyl;
$R_{x2}$ is selected from methyl, ethyl, trifluoromethyl, or trifluoromethoxy;
X is selected from $-CH_2-$, O, or S.

**12.** The compound of claim 5, or a pharmaceutically acceptable salt, isotopic variant, tautomer, or stereoisomer thereof, which is a compound of formula (IV-2), (IV-2a), or (IV-2b):

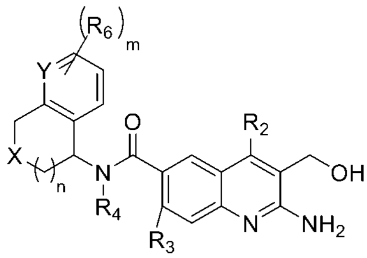

(IV-2)

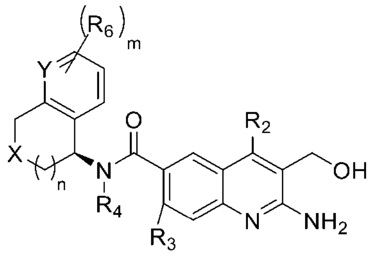
(IV-2a) or
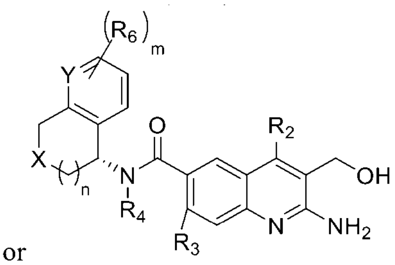
(IV-2b)

wherein,

$R_2$ is selected from H, methyl, ethyl, methoxy, or F;
$R_3$ is selected from H, F, Cl, CN, or methyl;
$R_4$ is selected from H, $C_{1-6}$ alkyl, $C_{1-6}$ haloalkyl, $C_{3-6}$ cycloalkyl, or 4-12 membered heterocyclyl;
$R_6$ is selected from H, CN, halogen, $C_{1-6}$ alkyl, $C_{1-6}$ haloalkyl, SFs, $OR_b$, or a 5-6 membered heteroaryl;
$R_b$ is selected from H, $C_{1-6}$ alkyl, or $C_{1-6}$ haloalkyl;
X is selected from $-CH_2-$, O, S, or $-NR_c-$;
Y is selected from CH or N;
Rc is selected from H or $C_{1-6}$ alkyl;
m is 0, 1, or 2;
n is 0, 1, 2, or 3.

**13.** The compound of claim 12, or a pharmaceutically acceptable salt, isotopic variant, tautomer, or stereoisomer thereof, wherein:

$R_2$ is selected from H, methyl, ethyl, methoxy, or F;
$R_3$ is selected from H, F, Cl, CN, or methyl;
$R_4$ is selected from H, methyl, ethyl, trifluoromethyl, trifluoroethyl, cyclopropyl, cyclobutyl, or oxetanyl;
$R_6$ is selected from H, CN, F, Cl, methyl, trifluoromethyl, SFs, methoxy, or pyridyl;
X is selected from $-CH_2-$, O, or S;
Y is selected from CH or N;
m is 1 or 2;
n is 1 or 2.

**14.** The compound of claim 5, or a pharmaceutically acceptable salt, isotopic variant, tautomer, or stereoisomer thereof, which is a compound of formula (IV-3), (IV-3a), or (IV-3b):

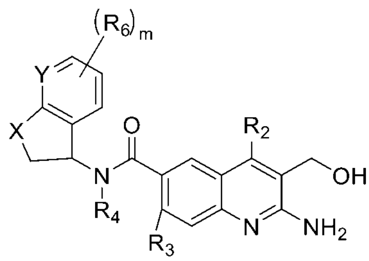
(IV-3)

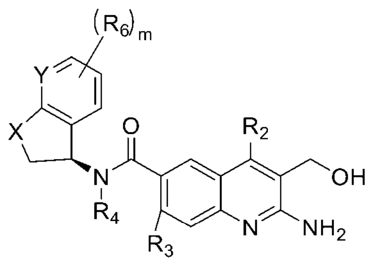
(IV-3a) or
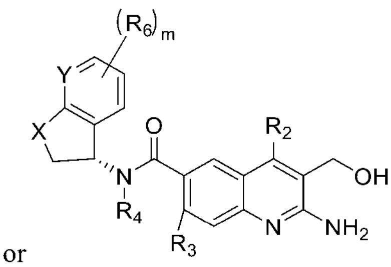
(IV-3b)

wherein,

$R_2$ is selected from H, methyl, ethyl, methoxy, or F;
$R_3$ is selected from H, F, Cl, CN, or methyl;
$R_4$ is selected from H, $C_{1-6}$ alkyl, deuterated $C_{1-6}$ alkyl, $C_{1-6}$ haloalkyl, $C_{3-6}$ cycloalkyl, or a 4-12 membered heterocyclyl;
$R_6$ is selected from H, CN, halogen, $C_{1-6}$ alkyl, $C_{1-6}$ haloalkyl, SFs, $OR_b$, $SCF_3$, or 5-6 membered heteroaryl;
$R_b$ is selected from H, $C_{1-6}$ alkyl, or $C_{1-6}$ haloalkyl;
X is selected from $-CH_2-$, O, S, or $-NR_c-$;
Y is selected from CH or N;
$R_c$ is selected from H or $C_{1-6}$ alkyl;
m is 0, 1, or 2.

**15.** The compound of claim 14, or a pharmaceutically acceptable salt, isotopic variant, tautomer, or stereoisomer thereof, wherein:

$R_2$ is selected from H, methyl, ethyl, methoxy, or F;
$R_3$ is selected from H, F, Cl, CN, or methyl;
$R_4$ is selected from H, methyl, methyl-$d_3$, ethyl, trifluoromethyl, trifluoroethyl, cyclopropyl, cyclobutyl, or oxetanyl;
$R_6$ is selected from H, CN, F, Cl, methyl, trifluoromethyl, $SF_s$, $SCF_3$, methoxy, or pyridyl;
X is selected from $-CH_2-$, O, or S;
Y is selected from CH or N;
m is 1 or 2.

**16.** A compound, or a pharmaceutically acceptable salt, isotopic variant, tautomer, or stereoisomer thereof, wherein the compound is selected from:

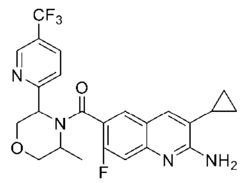,
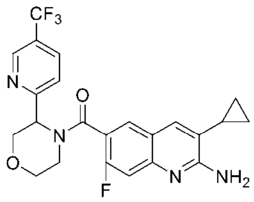,
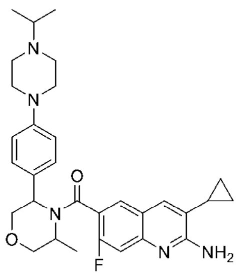,
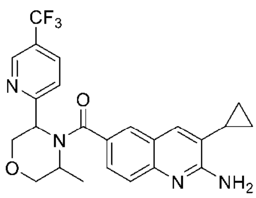,
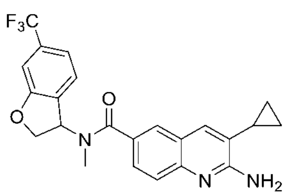,
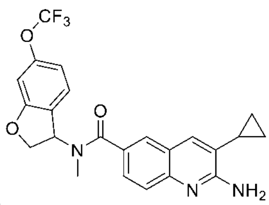,
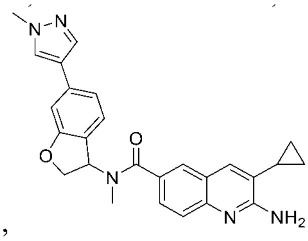,
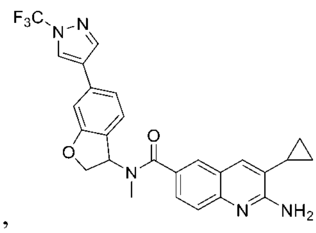,
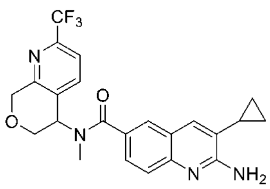,
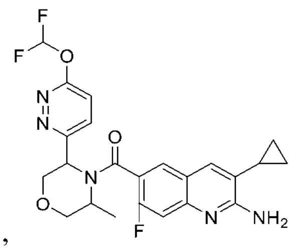,
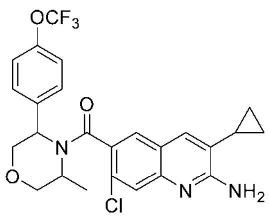,
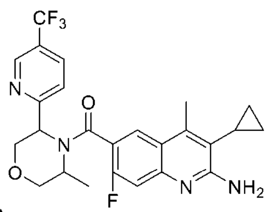,

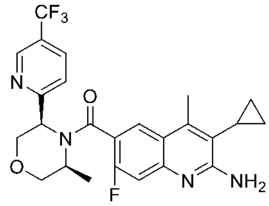,
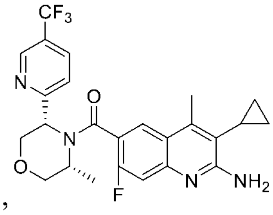,
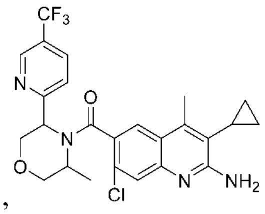,
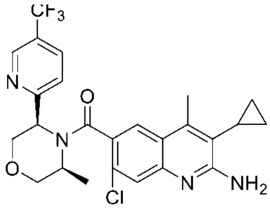,

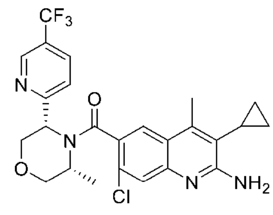,
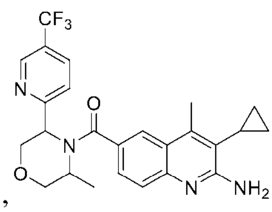,
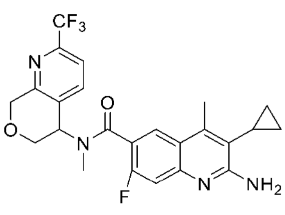,
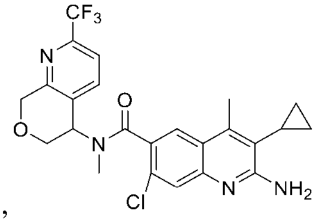,

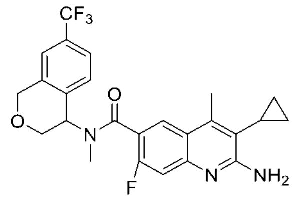,
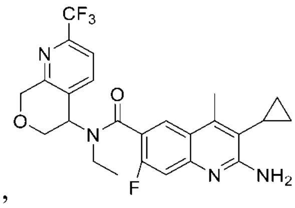,
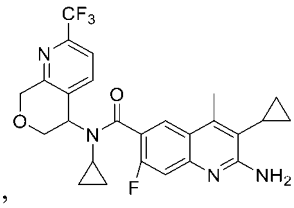,
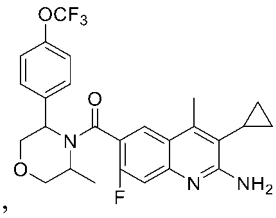,

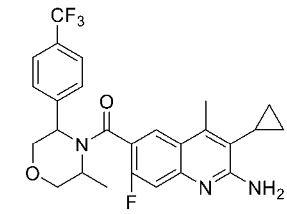,
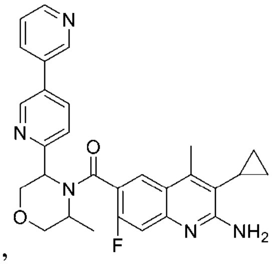,
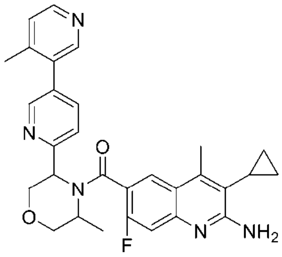,
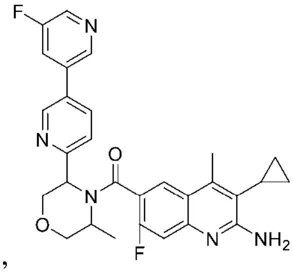,

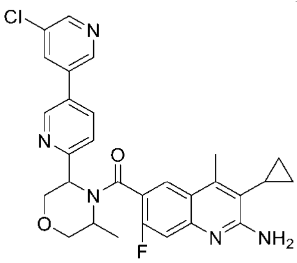,
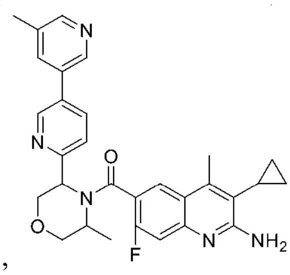,
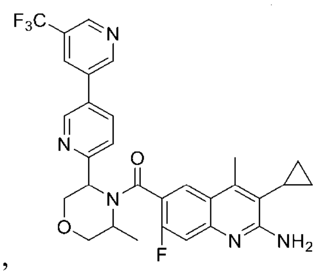,
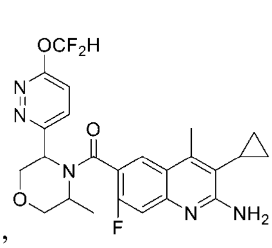,

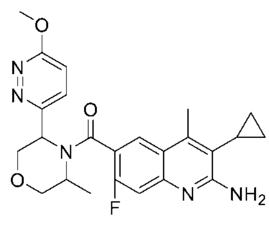,
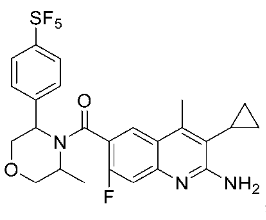,
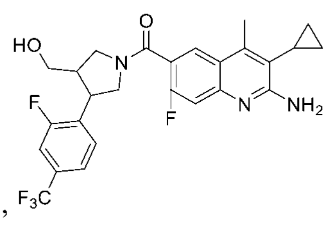,
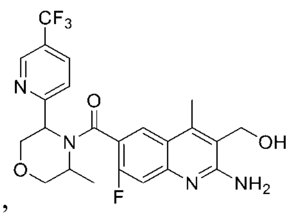,

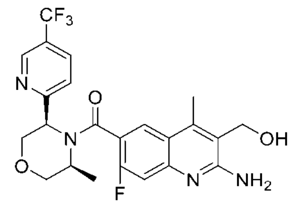, 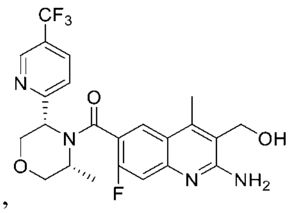, 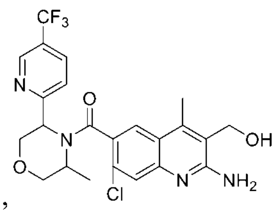, 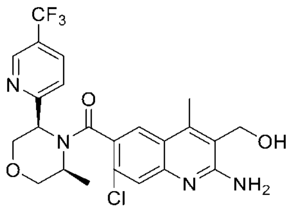,

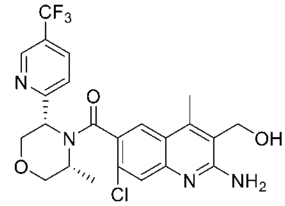, 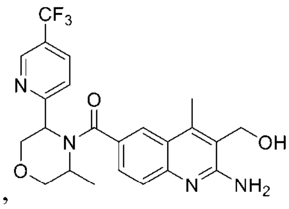, 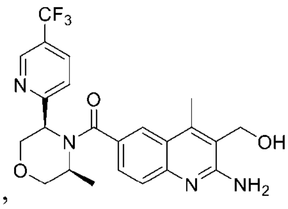, 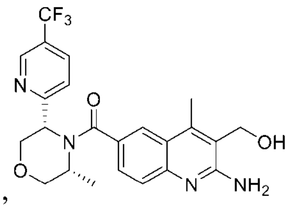,

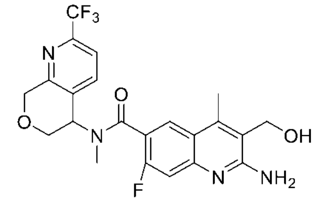, 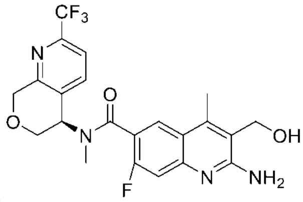, 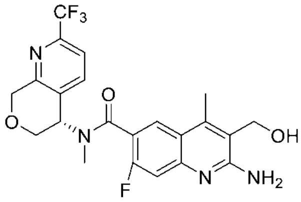,

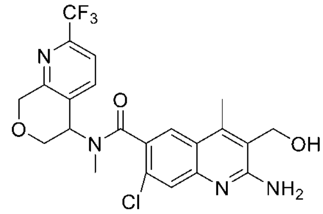, 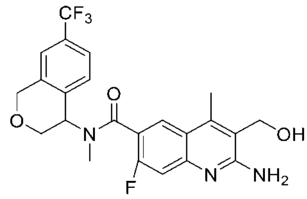, 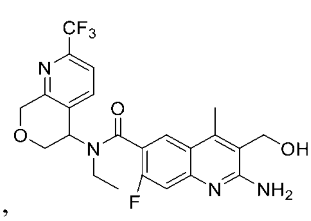,

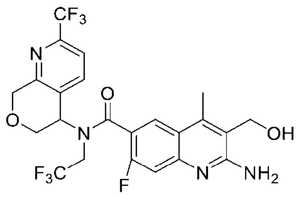, 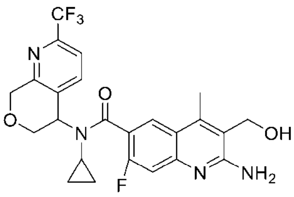, 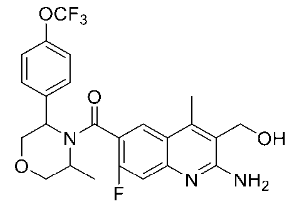, 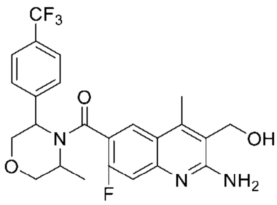,

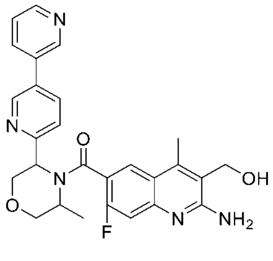, 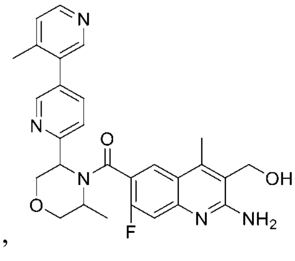, 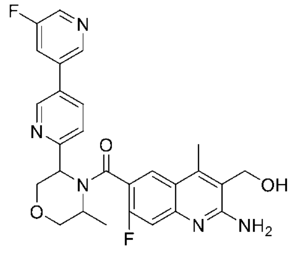, 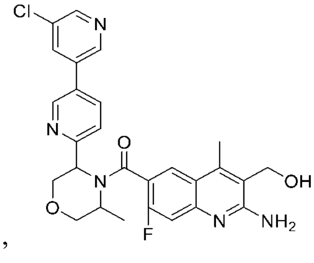,

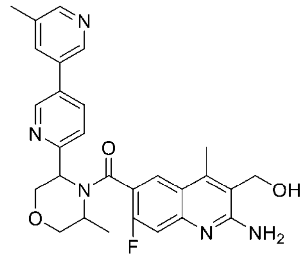, 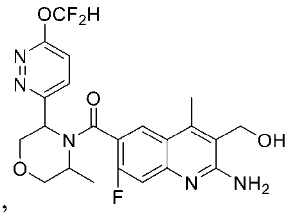, 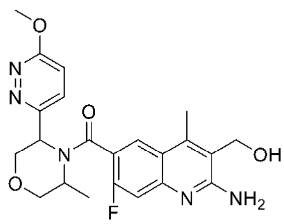, 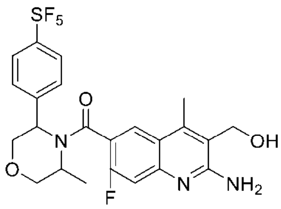,

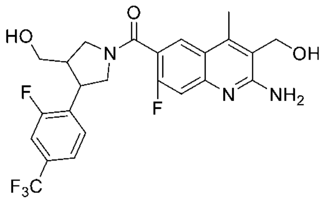, 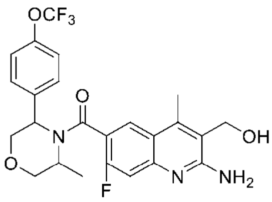, 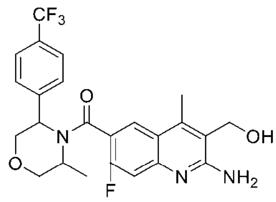, 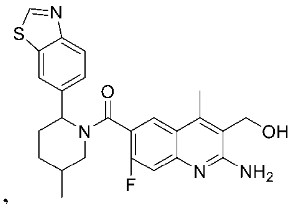,

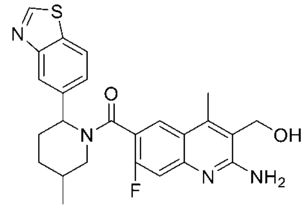, 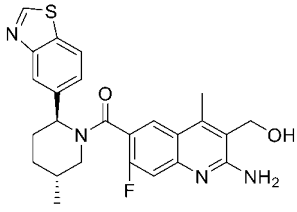, 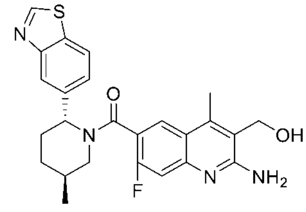,

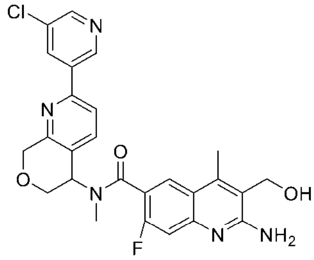, 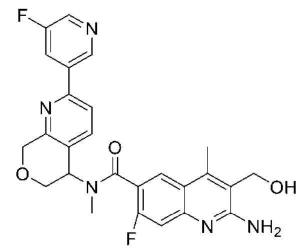, 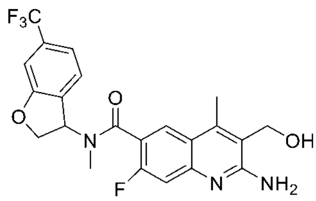,

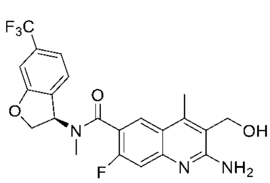, 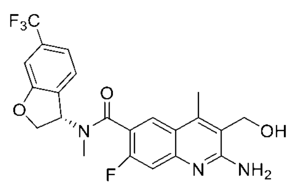, 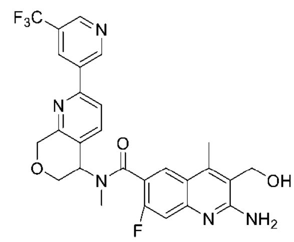, 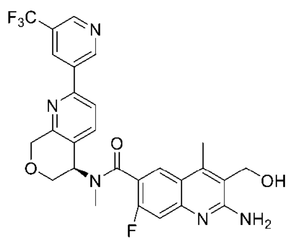,

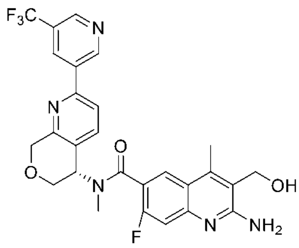, 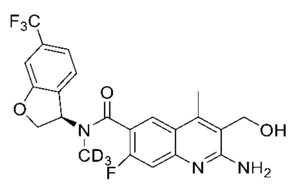, 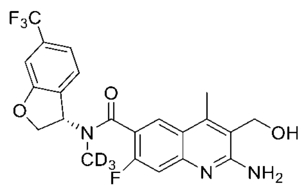,

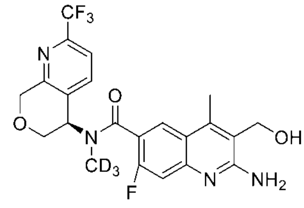,
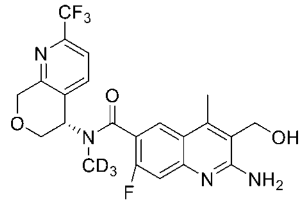,
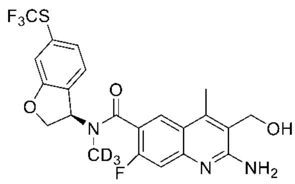,

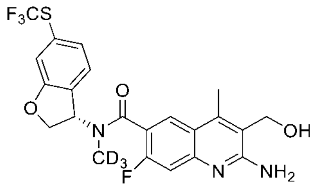,
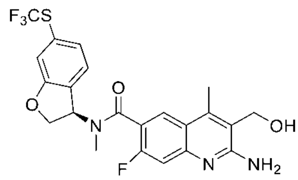 or
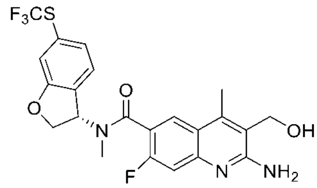.

**17.** A pharmaceutical composition comprising the compound of any one of claims 1-16, or a pharmaceutically acceptable salt, isotopic variant, tautomer, or stereoisomer thereof, and a pharmaceutically acceptable excipient; preferably, it further comprises other therapeutic agents.

**18.** The use of the compound of any one of claims 1-16, or a pharmaceutically acceptable salt, isotopic variant, tautomer, or stereoisomer thereof, in the preparation of a drug for treating and/or preventing PRMT5 methyltransferase-mediated diseases.

**19.** A method for treating and/or preventing PRMT5 methyltransferase-mediated diseases in a subject, comprising administering to the subject the compound of any one of claims 1-16, or a pharmaceutically acceptable salt, isotopic variant, tautomer, or stereoisomer thereof, or the pharmaceutical composition of claim 17.

**20.** The compound of any one of claims 1-16, or a pharmaceutically acceptable salt, isotopic variant, tautomer, or stereoisomer thereof, or the pharmaceutical composition of claim 17, which is used in treating and/or preventing PRMT5 methyltransferase-mediated diseases.

**21.** The use of claim 18, the method of claim 19, or the compound or composition of claim 20, wherein the PRMT5 methyltransferase-mediated disease is cancer, the cancer being selected from: Schwannoma, adenocarcinoma, adrenal cancer, anal cancer, angiosarcoma (e.g., lymphangiosarcoma, lymphangioendothelial sarcoma, hemangioma), appendiceal cancer, benign monoclonal gammopathy, cholangiocarcinoma, bladder cancer, brain cancer (e.g., meningioma, glioma, such as astrocytoma, oligodendroglioma, medulloblastoma), bronchial cancer, carcinoid tumor, cervical cancer (e.g., cervical adenocarcinoma), choriocarcinoma, chordoma, craniopharyngioma, colorectal cancer (e.g., colon cancer, rectal cancer, colorectal adenocarcinoma), epithelial cancer, ependymoma, endothelial sarcoma (e.g., Kaposi's sarcoma, multiple idiopathic hemorrhagic sarcoma), endometrial cancer (e.g., uterine cancer, uterine sarcoma), esophageal cancer (e.g., esophageal adenocarcinoma, Barrett's adenocarcinoma), Ewing's sarcoma, eye cancer (e.g., intraocular melanoma, retinoblastoma), eosinophilia, gallbladder cancer, gastric cancer (e.g., gastric adenocarcinoma), gastrointestinal stromal tumor (GIST), head and neck cancer (e.g., head and neck squamous cell carcinoma, oral cancer (e.g., oral squamous cell carcinoma), laryngeal cancer (e.g., laryngeal cancer, pharyngeal cancer, nasopharyngeal cancer, oropharyngeal cancer)), hematologic cancers (e.g., leukemia, including acute lymphoblastic leukemia (ALL) (e.g., B-cell ALL, T-cell ALL), acute myeloid leukemia (AML) (e.g., B-cell AML, T-cell AML), chronic myeloid leukemia (CML) (e.g., B-cell CML, T-cell CML), chronic lymphocytic leukemia (CLL) (e.g., B-cell CLL, T-cell CLL), follicular lymphoma, chronic lymphocytic leukemia/small lymphocytic lymphoma (CLL/SLL), marginal zone B-cell lymphoma (e.g., mucosa-associated lymphoid tissue (MALT) lymphoma, nodal marginal zone B-cell lymphoma, splenic marginal zone B-cell lymphoma), primary mediastinal B-cell lymphoma, Burkitt's lymphoma, lymphoplasmacytic lymphoma, hairy cell leukemia (HCL), immunoblastic large cell lymphoma, precursor B-lymphoblastic lymphoma, and primary central nervous system (CNS) lymphoma; as well as T-cell non-Hodgkin's lymphoma, such as precursor T-lymphoblastic lymphoma/leukemia, peripheral T-cell lymphoma (e.g., cutaneous T-cell lymphoma (e.g., mycosis fungoides, Sezary syndrome), angioimmunoblastic T-cell lymphoma, extranodal natural killer/T-cell lymphoma, enteropathy-associated T-cell lymphoma, subcutaneous panniculitis-like T-cell lymphoma, anaplastic large cell lymphoma); mixtures of one or more of the aforementioned leukemias/lymphomas; multiple myeloma (MM)), heavy chain diseases (e.g., $\alpha$-chain disease, $\gamma$-chain disease, $\mu$-chain disease), hemangioblastoma, inflammatory myofibroblastic tumor, immunocytic amyloidosis, kidney cancer (e.g., nephroblastoma, renal cell

carcinoma), liver cancer (e.g., hepatocellular carcinoma, malignant hepatocellular carcinoma), lung cancer (e.g., bronchial cancer, small cell lung cancer (SCLC), non-small cell lung cancer (NSCLC), lung adenocarcinoma, leiomyosarcoma (LMS)), mastocytosis (e.g., systemic mastocytosis), myelodysplastic syndrome (MDS), mesothelioma, myeloproliferative diseases (MPD) (e.g., polycythemia vera (PV), primary thrombocythemia (ET), idiopathic myelofibrosis (AMM), chronic idiopathic myelofibrosis, chronic granulocytic leukemia (CML), chronic neutrophilic leukemia (CNL), hypereosinophilic syndrome (HES)), neuroblastoma, neurofibroma (e.g., neurofibromatosis type 1 or type 2, schwannomatosis), neuroendocrine carcinoma (e.g., gastroenteropancreatic neuroendocrine tumors (GEP-NET), carcinoid tumor), osteosarcoma, ovarian cancer (e.g., cystadenocarcinoma, ovarian embryonal carcinoma, ovarian adenocarcinoma), papillary adenocarcinoma, penile cancer.

**INTERNATIONAL SEARCH REPORT**

International application No.
**PCT/CN2023/142809**

**A. CLASSIFICATION OF SUBJECT MATTER**

C07D 413/14(2006.01)i; C07D 401/12(2006.01)i; C07D 401/14(2006.01)i; C07D 403/12(2006.01)i; C07D 405/14(2006.01)i; C07D 417/14(2006.01)i; A61K 31/435(2006.01)i; A61K 31/4353(2006.01)i; A61P 35/00(2006.01)i

According to International Patent Classification (IPC) or to both national classification and IPC

**B. FIELDS SEARCHED**

Minimum documentation searched (classification system followed by classification symbols)

IPC:C07D 413/-; C07D 401/-; C07D 403/-; C07D 405/-; C07D 417/-; A61K 31/-; A61P 35/-

Documentation searched other than minimum documentation to the extent that such documents are included in the fields searched

Electronic data base consulted during the international search (name of data base and, where practicable, search terms used)

CNABS; CNTXT; VEN; WOTXT; USTXT; EPTXT; GBTXT; CATXT; CNKI; 万方, WANFANG; STN; WEB OF SCIENCE; PRMT5抑制剂; 蛋白质精氨酸甲基转移酶抑制剂; 甲硫腺苷磷酸化酶缺失; MTAP缺失; 甲硫腺苷; 癌症; 肿瘤; 喹啉; 吗啉; 哌啶; 吡啶; 苯并噻唑; 苏州浦合医药; 刘彬; 高峰; 郭永起; 景连栋; 吴勇勇; 李治中; 吴卓; PRMT5 inhibit+; protein arginine methyltransferase 5; methylthioadenosine phosphorylase; methylthioadenosine; MTA; cancer+; tumor+; tumour+; quinolin+; morpholin+; piperidin+; pyridin+; benzothiazol+; 式(I), (II), (III), (IV), (III-1), (III-2), (III-3), (IV-1), (IV-2), (IV-3), (III-1a), (III-1b), (III-2a), (III-2b), (III-3a), (III-3b), (IV-1a), (IV-1b), (IV-2a), (IV-2b), (IV-3a), (IV-3b)结构, structures of formulae (I), (II), (III), (IV), (III-1), (III-2), (III-3), (IV-1), (IV-2), (IV-3), (III-1a), (III-1b), (III-2a), (III-2b), (III-3a), (III-3b), (IV-1a), (IV-1b), (IV-2a), (IV-2b), (IV-3a), (IV-3b); 具体化合物结构, structures of specific compounds

**C. DOCUMENTS CONSIDERED TO BE RELEVANT**

| Category* | Citation of document, with indication, where appropriate, of the relevant passages | Relevant to claim No. |
|---|---|---|
| A | WO 2021163344 A1 (AMGEN INC.) 19 August 2021 (2021-08-19)<br>description, page 108, 'Example 300', and paragraph 18 | 1-18, 20-21 |
| A | CN 103492388 A (PFIZER INC.) 01 January 2014 (2014-01-01)<br>claims 1-8 | 1-18, 20-21 |
| A | KR 20090063869 A (KOREA INST SCI. & TECH. et al.) 18 June 2009 (2009-06-18)<br>claims 1-12 | 1-18, 20-21 |
| A | US 2003109547 A1 (AJINOMOTO KK.) 12 June 2003 (2003-06-12)<br>claim 1 | 1-18, 20-21 |
| A | WO 2021127166 A1 (KHAN TANWEER A. et al.) 24 June 2021 (2021-06-24)<br>claim 1 | 1-18, 20-21 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **04.03月2024（04.03.2024）** | **22 March 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

INTERNATIONAL SEARCH REPORT

International application No.
**PCT/CN2023/142809**

**C. DOCUMENTS CONSIDERED TO BE RELEVANT**

| Category* | Citation of document, with indication, where appropriate, of the relevant passages | Relevant to claim No. |
|---|---|---|
| A | WO 2022169948 A1 (AMGEN INC.) 11 August 2022 (2022-08-11) claims 1-27 | 1-18, 20-21 |
| A | WO 2022256806 A1 (IDEAYA BIOSCIENCES INC.) 08 December 2022 (2022-12-08) claims 1-60 | 1-18, 20-21 |

Form PCT/ISA/210 (second sheet) (July 2022)

**INTERNATIONAL SEARCH REPORT**

International application No.
**PCT/CN2023/142809**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **19, 21 (in part)**
because they relate to subject matter not required to be searched by this Authority, namely:

Claim 19 sets forth a method for treating and/or preventing PRMT5-mediated diseases in a subject, which falls within methods for treatment of the human or animal body by therapy, and falls within subject matter for which no search is required by the International Searching Authority as defined in PCT Rule 39.1(iv).

Claim 21 (in part) refers to claim 19. On the basis of the same reasoning mentioned above, the subject matter of claim 21 also falls within subject matter for which no search is required by the International Searching Authority as defined in PCT Rule 39.1(iv). The search and examination for claim 21 are made on the basis of the subject matter thereof which refers to claim 18 or 20.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/142809**

| Patent document cited in search report | Publication date (day/month/year) | Patent family member(s) | Publication date (day/month/year) |
|---|---|---|---|
| WO 2021163344 A1 | 19 August 2021 | None | |
| CN 103492388 A | 01 January 2014 | EA 201391288 A1 | 30 April 2014 |
| | | EA 022375 B1 | 30 December 2015 |
| | | PT 2699576 E | 09 March 2016 |
| | | US 2012270893 A1 | 25 October 2012 |
| | | US 8802688 B2 | 12 August 2014 |
| | | MY 162167 A | 31 May 2017 |
| | | ME 02312 B | 20 June 2016 |
| | | NZ 616156 A | 29 August 2014 |
| | | CL 2013002794 A1 | 27 December 2013 |
| | | TW 201305162 A | 01 February 2013 |
| | | TWI 464171 B | 11 December 2014 |
| | | AP 201307129 D0 | 30 September 2013 |
| | | HUE 028602 T2 | 28 December 2016 |
| | | CU 20130126 A7 | 29 January 2014 |
| | | CU 24164 B1 | 31 March 2016 |
| | | MX 2013012313 A | 31 January 2014 |
| | | MX 348860 B | 30 June 2017 |
| | | US 2015112068 A1 | 23 April 2015 |
| | | DOP 2013000243 A | 31 December 2013 |
| | | AP 2013007129 A0 | 30 September 2013 |
| | | AP 201307129 A0 | 31 December 2015 |
| | | JP 2014515755 A | 03 July 2014 |
| | | JP 5657174 B2 | 21 January 2015 |
| | | ES 2561452 T3 | 26 February 2016 |
| | | IL 228949 A0 | 31 December 2013 |
| | | IL 228949 A | 30 November 2016 |
| | | EP 2699576 A1 | 26 February 2014 |
| | | EP 2699576 B1 | 09 December 2015 |
| | | US 2015336958 A1 | 26 November 2015 |
| | | WO 2012143813 A1 | 26 October 2012 |
| | | CA 2831380 A1 | 26 October 2012 |
| | | CA 2831380 C | 05 April 2016 |
| | | CO 6801757 A2 | 29 November 2013 |
| | | US 2014323730 A1 | 30 October 2014 |
| | | SG 194142 A1 | 29 November 2013 |
| | | HRP 20151397 T1 | 15 January 2016 |
| | | NI 201300111 A | 12 February 2014 |
| | | SI 2699576 T1 | 29 February 2016 |
| | | UY 34027 A | 05 April 2013 |
| | | CR 20130464 A | 16 October 2013 |
| | | DK 2699576 T3 | 15 February 2016 |
| | | PL 2699576 T3 | 31 May 2016 |
| | | PE 20141187 A1 | 18 September 2014 |
| | | ECSP 13013038 A | 31 January 2014 |
| | | CY 1117122 T1 | 05 April 2017 |
| | | KR 20140015511 A | 06 February 2014 |
| | | KR 101567659 B1 | 09 November 2015 |
| | | RS 54526 B1 | 30 June 2016 |
| | | AU 2012245996 A1 | 03 October 2013 |

Form PCT/ISA/210 (patent family annex) (July 2022)

**INTERNATIONAL SEARCH REPORT**
**Information on patent family members**

International application No.
**PCT/CN2023/142809**

| Patent document cited in search report | Publication date (day/month/year) | Patent family member(s) | Publication date (day/month/year) |
|---|---|---|---|
| | | AU 2012245996 B2 | 01 September 2016 |
| | | GEP 20166474 B | 10 May 2016 |
| | | GT 201300258 A | 02 June 2015 |
| | | UA 107753 C2 | 10 February 2015 |
| | | MA 35061 B1 | 03 April 2014 |
| | | AR 086198 A1 | 27 November 2013 |
| | | TN 2013000393 A1 | 20 January 2015 |
| KR 20090063869 A | 18 June 2009 | KR 100937738 B1 | 21 January 2010 |
| US 2003109547 A1 | 12 June 2003 | DE 60039782 D1 | 18 September 2008 |
| | | ATE 403644 T1 | 15 August 2008 |
| | | AU 1651201 A | 18 June 2001 |
| | | WO 0142199 A1 | 14 June 2001 |
| | | EP 1236712 A1 | 04 September 2002 |
| | | EP 1236712 A4 | 20 April 2005 |
| | | EP 1236712 B1 | 06 August 2008 |
| | | US 6710056 B2 | 23 March 2004 |
| WO 2021127166 A1 | 24 June 2021 | MX 2022007442 A | 13 February 2023 |
| | | CR 20220326 A | 10 February 2023 |
| | | EP 4076440 A1 | 26 October 2022 |
| | | EP 4076440 A4 | 08 November 2023 |
| | | BR 112022012273 A2 | 30 August 2022 |
| | | KR 20220129554 A | 23 September 2022 |
| | | US 2023027198 A1 | 26 January 2023 |
| | | JP 2023527487 A | 29 June 2023 |
| | | IL 293839 A | 01 August 2022 |
| | | CA 3162253 A1 | 24 June 2021 |
| | | CO 2022009852 A2 | 11 October 2022 |
| | | AU 2020407589 A1 | 21 July 2022 |
| WO 2022169948 A1 | 11 August 2022 | None | |
| WO 2022256806 A1 | 08 December 2022 | CA 3220160 A1 | 08 December 2022 |
| | | AU 2022287057 A1 | 14 December 2023 |
| | | IL 308853 A | 01 January 2024 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5376645 A


### Non-patent literature cited in the description


- *Nat Rev Mol Cell Biol*, October 2019, vol. 20 (10), 642-657
- *Nat Rev Drug Discov*, July 2021, vol. 20 (7), 509-530
- *Cell Stress*, August 2020, vol. 4 (8), 199-215
- *Cancer Gene Ther*, March 2022, vol. 29 (3-4), 264-276
- *Bioorg Med Chem Lett*, 01 June 2019, vol. 29 (11), 1264-1269
- *Expert Opin Ther Pat*, February 2019, vol. 29 (2), 97-114
- *Annals of Oncology*, 2020, vol. 31 (4), S462-S504
- *Annals of Oncology*, 2019, vol. 30 (5), v159-v193
- *Science*, 11 March 2016, vol. 351 (6278), 1214-8
- *Nat Rev Drug Discov*, January 2020, vol. 19 (1), 23-38
- *Cell Rep*, 19 April 2016, vol. 15 (3), 574-587
- *J Med Chem*, 10 February 2022, vol. 65 (3), 1749-1766
- Remington's Pharmaceutical Sciences. Mack Publishing Company, 1985